(19) Europäisches Patentamt
European Patent Office
Office européen des brevets

(11) EP 3 704 221 B1

(12) # EUROPEAN PATENT SPECIFICATION

(45) Date of publication and mention of the grant of the patent:
**10.04.2024 Bulletin 2024/15**

(21) Application number: **18799475.1**

(22) Date of filing: **31.10.2018**

(51) International Patent Classification (IPC):
*C11D 3/386* (2006.01) *C11D 1/62* (2006.01)
*C11D 1/66* (2006.01) *C11D 1/94* (2006.01)
*C11D 3/12* (2006.01) *C11D 3/20* (2006.01)
*C11D 3/22* (2006.01) *C11D 3/33* (2006.01)
*C11D 3/36* (2006.01) *C11D 3/37* (2006.01)
*C11D 3/38* (2006.01) *C11D 3/39* (2006.01)
*C11D 3/42* (2006.01) *C11D 3/43* (2006.01)
*C11D 3/48* (2006.01) *C11D 3/50* (2006.01)
*C11D 7/20* (2006.01) *C11D 7/36* (2006.01)
*C11D 3/00* (2006.01) *C11D 11/00* (2006.01)
*C11D 17/04* (2006.01)

(52) Cooperative Patent Classification (CPC):
**C11D 3/38636; C11D 1/62; C11D 1/66; C11D 1/94; C11D 3/0036; C11D 3/1246; C11D 3/128; C11D 3/2082; C11D 3/2086; C11D 3/225; C11D 3/227; C11D 3/33; C11D 3/361; C11D 3/3742; C11D 3/3757;** (Cont.)

(86) International application number:
**PCT/EP2018/079837**

(87) International publication number:
**WO 2019/086521 (09.05.2019 Gazette 2019/19)**

(54) **CLEANING COMPOSITIONS CONTAINING DISPERSINS II**

REINIGUNGSZUSAMMENSETZUNG ENTHALTEND DISPERSINS II

COMPOSITIONS DE NETTOYAGE CONTENANT DES DISPERSINS II

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2017 DE 102017125559**

(43) Date of publication of application:
**09.09.2020 Bulletin 2020/37**

(73) Proprietor: **Henkel AG & Co. KGaA**
**40589 Düsseldorf (DE)**

(72) Inventors:
- **WIELAND, Susanne**
  **41541 Zons/Dormagen (DE)**
- **KARSTEN, Stefan**
  **51645 Gummersbach (DE)**
- **WEBER, Thomas**
  **35037 Marburg (DE)**
- **STEHR, Regina**
  **41468 Neuss (DE)**
- **SCHMELING, Marianne**
  **41352 Korschenbroich (DE)**
- **DREJA, Michael**
  **41469 Neuss (DE)**
- **WEIDE, Mirko**
  **40223 Düsseldorf (DE)**
- **OEHLENSCHLAEGER, Christian**
  **2880 Bagsvaerd (DK)**
- **SEGURA, Dorotea Raventos**
  **2880 Bagsvaerd (DK)**
- **VEJBORG, Rebecca**
  **3450 Alleroed (DK)**
- **GEERTZ-HANSEN, Henrik**
  **2880 Bagsvaerd (DK)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BALTSEN, Lilian**
  **2880 Bagsvaerd (DK)**
- **SALOMON, Jesper**
  **2840 Holte (DK)**

(56) References cited:

**EP-A1- 3 088 505** **WO-A1-2017/186937**
**WO-A1-2017/207770** **WO-A1-2018/184873**

- **DATABASE UniProt [Online] 20 January 2016 (2016-01-20), "SubName: Full=Uncharacterized protein {ECO:0000313|EMBL:KQR31125.1};", XP002788152, retrieved from EBI accession no. UNIPROT:A0A0Q5KJC4 Database accession no. A0A0Q5KJC4**
- **CRISTINA VAL-CID ET AL: "Structural-Functional Analysis Reveals a Specific Domain Organization in Family GH20 Hexosaminidases", PLOS ONE, vol. 10, no. 5, 29 May 2015 (2015-05-29), page e0128075, XP055544015, DOI: 10.1371/journal.pone.0128075**

Remarks:

The complete document including Reference Table(s) and the Sequence Listing(s) can be downloaded from the EPO website

(52) Cooperative Patent Classification (CPC): (Cont.)
**C11D 3/3769; C11D 3/3776; C11D 3/378; C11D 3/381; C11D 3/386; C11D 3/38645; C11D 3/3905; C11D 3/42; C11D 3/43; C11D 3/48; C11D 3/50; C11D 3/505; C11D 7/20; C11D 7/36; C11D 17/043; C12N 9/2402; D06L 4/40;**
C11D 2111/12; C11D 2111/14

## Description

## Background of the Invention

### Field of the Invention

The present invention relates to specific cleaning compositions comprising polypeptides having hexosaminidase activity with the polypeptide comprising one or more Glyco_hydro_20 catalytic domains as well as specific conserved motifs. The invention further relates to the use of said compositions as well as a method for cleaning an item in which the item is exposed to said cleaning composition.

### Description of the Related Art

Polypeptides having hexosaminidase activity include dispersins such as Dispersin B (DspB) which are β-N-acetylglucosamininidases belonging to the Glycoside Hydrolase 20 family (GH20). Dispersin B is produced by the periodontal pathogen, *Aggregatibacter actinomycetemcomitans,* a Gram-negative oral bacterium. Dispersin B is a β-hexosaminidase that specifically hydrolyzes β-1,6-glycosidic linkages of acetylglucosamine polymers e.g. found in biofilm. Dispersin B contains three highly conserved acidic residues: an aspartic acid at residue 183 (D183), a glutamic acid at residue 184 (E184), and a glutamic acid at residue 332 (E332). Biofilm have been found attached to various surfaces including medical devices such as implants. WO04061117 A2 (Kane Biotech INC) describe use of compositions comprising DspB for reducing biofilm caused by poly-N-acetylglucosamine-producing bacteria and Kane et al. describes the use of compositions for reduction of biofilm on medical devises and for wound care. Biofilm may also be present on laundry items, such as fabrics, other hard surfaces, such as dish wash utensils, dish washers and washing machines where they may cause malodor, which is difficult to remove and may sustain even after wash. The present invention provides suitable enzymes for use in cleaning compositions e.g. detergents and for deep cleaning of laundry item as well as cleaning processes.

## Summary of the Invention

The present invention and its preferred embodiments are apparent from the appendant set of claims.

## Overview of Sequence Listing

SEQ ID NO 1 is the amino acid sequence of the GH20 catalytic domain of SWISSPROT:Q6GYA5 from *Actinobacillus pleuropneumoniae.*
SEQ ID NO 2 is the amino acid sequence of the GH20 catalytic domain of SWISSPROT:G4AQA6 from *Aggregatibacter actinomycetemcomitans.*
SEQ ID NO 3 is the amino acid sequence of the GH20 catalytic domain of SWISSPROT:A6VMN2 from *Actinobacillus succinogenes.*
SEQ ID NO 4 is the amino acid sequence of the GH20 catalytic domain of SWISSPROT:G4CNH0 from *Neisseria wadsworthii.*
SEQ ID NO 5 is the amino acid sequence of the GH20 catalytic domain of SEQ ID 9 from *Actinobacillus capsulatus.*
SEQ ID NO 6 is the amino acid sequence of the GH20 catalytic domain of SEQ ID 10 from *Terribacillus goriensis.*
SEQ ID NO 7 is the amino acid sequence of the GH20 catalytic domain of SEQ ID 11 from *Terribacillus saccharophilus.*
SEQ ID NO 8 is the amino acid sequence of the catalytic domain of SEQ ID 12 from *Curtobacterium oceanosedimentum.*
SEQ ID NO 9 is the amino acid sequence derived from SEQ ID 13 from *Actinobacillus capsulatus.*
SEQ ID NO 10 is the amino acid sequence derived from SEQ ID 14 from *Terribacillus goriensis.*
SEQ ID NO 11 is the amino acid sequence derived from SEQ ID 15 from *Terribacillus saccharophilus.*
SEQ ID NO 12 is the amino acid sequence derived from SEQ ID 16 from *Curtobacterium oceanosedimentum.*
SEQ ID NO 13 is the DNA encoding the full-length polypeptide from *Actinobacillus capsulatus.*
SEQ ID NO 14 is the DNA encoding the full-length polypeptide from *Terribacillus goriensis.*
SEQ ID NO 15 is the DNA encoding the full-length polypeptide from *Terribacillus saccharophilus.*
SEQ ID NO 16 is the DNA encoding the full-length polypeptide from *Curtobacterium oceanosedimentum*
SEQ ID NO 17 is the mature polypeptide of SEQ ID 13 from *Actinobacillus capsulatus.*
SEQ ID NO 18 is the mature polypeptide of SEQ ID 14 from *Terribacillus goriensis.*

SEQ ID NO 19 is the mature polypeptide of SEQ ID 15 from *Terribacillus saccharophilus.*
SEQ ID NO 20 is the mature polypeptide of SEQ ID 16 from *Curtobacterium oceanosedimentum*
SEQ ID NO 21 is conserved [IV]P[ED][LVI]DXP[AN]H
SEQ ID NO 22 is conserved motif II NYN[AS]Y[SY]LY
SEQ ID NO 23 *Bacillus clausii* secretion signal MKKPLGKIVASTALLISVAFSSSIASA
SEQ ID NO 24 His-tag HHHHHHPR
SEQ ID NO 25 is the amino acid sequence derived from SEQ ID NO 29 from *Curtobacterium flaccumfaciens*
SEQ ID NO 26 is the amino acid sequence derived from SEQ ID NO 30 from *Curtobacterium luteum*
SEQ ID NO 27 is the amino acid sequence derived from SEQ ID NO 31 from *Curtobacterium oceanosedimentum*
SEQ ID NO 28 is the amino acid sequence derived from SEQ ID NO 32 from *Curtobacterium sp. Leaf154*
SEQ ID NO 29 is the DNA encoding the full-length polypeptide from *Curtobacterium flaccumfaciens*
SEQ ID NO 30 is the DNA encoding the full-length polypeptide from *Curtobacterium luteum*
SEQ ID NO 31 is the DNA encoding the full-length polypeptide from *Curtobacterium oceanosedimentum*
SEQ ID NO 32 is the DNA encoding the full-length polypeptide from *Curtobacterium sp. Leaf154*
SEQ ID NO 33 is the mature polypeptide of SEQ ID 25 from *Curtobacterium flaccumfaciens*
SEQ ID NO 34 is the mature polypeptide of SEQ ID 26 from *Curtobacterium luteum*
SEQ ID NO 35 is the mature polypeptide of SEQ ID 27 from *Curtobacterium oceanosedimentum*
SEQ ID NO 36 is the mature polypeptide of SEQ ID 28 from *Curtobacterium Leaf154*
SEQ ID NO 37 is the amino acid sequence of the catalytic domain of SEQ ID 25 from *Curtobacterium flaccumfaciens.*
SEQ ID NO 38 is the amino acid sequence of the catalytic domain of SEQ ID 26 from *Curtobacterium luteum.*
SEQ ID NO 39 is the amino acid sequence of the catalytic domain of SEQ ID 27 from *Curtobacterium oceanosedimentum.*
SEQ ID NO 40 is the amino acid sequence of the catalytic domain of SEQ ID 28 from *Curtobacterium Leaf154*
SEQ ID NO 41 is the polypeptide motif GXDE
SEQ ID NO 42 is the polypeptide motif ARAYYPV
SEQ ID NO 43 is the polypeptide motif AWNDGID
SEQ ID NO 44 is the polypeptide motif DDQNVGI
SEQ ID NO 45 is the polypeptide motif DPRIH
SEQ ID NO 46 is the polypeptide motif [EQ][NRSHA][YVFL][AGSTC][IVLF][EAQYN][SN]

## Definitions

**Dispersin:** The term "dispersin" and the abbreviation "Dsp" means a polypeptide having hexosaminidase activity, EC 3.2.1.- that catalyzes the hydrolysis of β-1,6-glycosidic linkages of N-acetyl-glucosamine polymers (poly-N-acetylglucosamine) found e.g. in biofilm.

**Hexosaminidase:** The term "hexosaminidases" means a polypeptide having hexosaminidase activity (hexosaminidases), and includes EC 3.2.1.e.g. that catalyzes the hydrolysis of of N-acetyl-D-hexosamine or N-acetyl-glucosamine polymers found e.g. in biofilm. The term includes dispersins and includes polypeptides having N-acetylglucosaminidase activity and β-N-acetylglucosamininidase activity. The term "polypeptide having hexosaminidase activity" may be used interchangeably with the term hexosaminidases and similar the term "polypeptide having β-N-acetylglucosaminidase activity" may be used interchangeably with the term β-N-acetylglucosamininidases. For the purposes of the present invention, hexosaminidase activity is determined according to the procedure described in Assay 1 or 2. In one aspect, the polypeptides used in the compositions of the present invention have at least 20%, e.g., at least 40%, at least 50%, at least 60%, at least 70%, at least 80%, at least 90%, at least 95%, or at least 100% of the hexosaminidase activity of the mature polypeptide of SEQ ID NO 20, SEQ ID NO 33, SEQ ID NO 34 or SEQ ID NO 35.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Biofilm:** A biofilm may be produced by any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium. Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer

layer of cells protect the interior of the community. On laundry and hard surfaces biofilm producing bacteria can be found among the following species: *Acinetobacter sp., Aeromicrobium sp., Brevundimonas sp., Microbacterium sp Micrococcus luteus, Pseudomonas sp., Streptococcus sp., Streptococcus dysgalactiae, Staphylococcus epidermidis, Staphylococcus aureus, Staphylococcus pneumoniae, Stenotrophomonas sp., Enterobacter sp., Xanthomonas sp., Yersinia sp., Klebsiella sp., Burkholderia sp.,Stenotrophomonas sp., Variovorax sp., Escherichia sp., Ralstonia sp., Achromobacter sp., Luteibacter sp., Citrobacter sp., Xanthomonadaceae sp., Halomonas sp., Bordetella sp., Lysobacter sp., Serratia sp., Escherichia sp., Aggregatibacter sp., Listeria monocytogenes, Clostridium difficile, Mycobacterium sp., Neisseria gonorrheae, H. influenzae, Haemophilus ducreyi, Helicobacter pylori, Campylobacter jejuni* and *Enterococcus faecalis* as well as the fungi *Candida albicans, Aspergillus flavus, Fusarium solani,* and *Cryptococcus neoformans.* In one aspect, the biofilm component e.g. poly-N-acetylglucosamine comprising strain is *Brevundimonas* sp. In one aspect, the biofilm component e.g. poly-N-acetylglucosamine comprising strain is *Pseudomonas alcaliphila* or *Pseudomonas fluorescens.* In one aspect, the biofilm component e.g. poly-N-acetylglucosamine comprising strain is *Staphylococcus aureus.*

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Deep cleaning:** By the term "deep cleaning" is meant reduction or removal of components of biofilm, such as EPS or parts hereof, polysaccharides, poly-N-acetylglucosamine (PNAG), proteins, DNA, soil or other components present in the biofilm.

**Cleaning/Detergent adjunct ingredient:** The cleaning e.g. detergent adjunct ingredient is different to the hexosaminidases of this invention. The precise nature of these additional adjunct components, and levels of incorporation thereof, will depend on the physical form of the composition and the nature of the operation for which it is to be used. Suitable adjunct materials include, but are not limited to the components described below such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, bittering agents, and/or pigments.

**Cleaning and/or Detergent Composition:** The terms "cleaning composition" and "detergent composition" are used interchangeably herein and refer to compositions that find use in the removal of undesired compounds from items to be cleaned, such as textiles or hard surfaces. The detergent composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners (fabric finishers); and textile and laundry pre-spotters/pretreatment). In addition, the term also includes hand and automatic dishwashing detergents and cleaning detergents for hard surfaces. In addition to containing the enzyme described herein, the detergent formulation may contain one or more additional enzymes (such as proteases, amylases, lipases, cutinases, cellulases, endoglucanases, xyloglucanases, pectinases, pectin lyases, xanthanases, peroxidases, haloperoxygenases, catalases and mannanases, or any mixture thereof), and/or detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, enzyme inhibitors or stabilizers, enzyme activators, transferase(s), hydrolytic enzymes, oxido reductases, bluing agents and fluorescent dyes, antioxidants, and solubilizers.

**Enzyme Detergency benefit:** The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and/or cleaning, prevention or reduction of redeposition of soils released in the washing process (an effect that also is termed anti-redeposition), restoring fully or partly the whiteness of textiles which originally were white but after repeated use and wash have obtained a greyish or yellowish appearance (an effect that also is termed whitening). Textile care benefits, which are not directly related to catalytic stain removal or prevention of redeposition of soils, are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric-softness, color clarification of the fabric and removal of particulate soils which are trapped in the fibers of the fabric or garment.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide having one or more (e.g., several) amino acids absent from the amino and/or carboxyl terminus of a mature polypeptide or domain; wherein the fragment has hexosaminidase activity. In one aspect, a fragment contains at least 350 amino acid residues (e.g., amino acids 105 to 454 of SEQ ID NO 20), at least 355 amino acid residues (e.g., amino acids 100 to 454 of SEQ ID NO 20), at least 360 amino acid residues (e.g., amino acids 100 to 459 of SEQ ID NO 20) or at least 450 amino acid residues (e.g., amino acids 5 to 454 of SEQ ID NO 20) or at least 450 amino acid residues (e.g., amino acids 1 to 450 of SEQ ID NO 20). In one aspect, a fragment contains at least 300 amino acid residues (e.g., amino acids 1 to 300 of SEQ ID NO 33), at least 350 amino acid residues (e.g., amino acids 1 to 350 of SEQ ID NO 33), at least 340 amino acid residues (e.g., amino acids 10 to 349 of SEQ ID NO 33) or at least 400 amino acid residues (e.g., amino acids 5 to 404 of SEQ ID NO 33) or at least 440 amino acid residues (e.g., amino acids 1 to 440 of SEQ ID NO 33). In one aspect, a fragment contains at least 300 amino acid residues (e.g., amino acids 1 to 300 of SEQ ID NO 34), at least 350 amino acid residues (e.g., amino acids 1 to 350 of SEQ ID NO 34), at least 340 amino acid residues (e.g., amino acids 10 to 349 of SEQ ID NO 34) or at least 400 amino acid residues (e.g., amino acids 5 to 404 of SEQ ID NO 34) or at least 440 amino acid residues (e.g., amino acids 1 to 440 of SEQ ID NO 34). In one aspect, a fragment contains at least 350 amino acid residues (e.g., amino acids 105 to 454 of SEQ ID NO 35), at least 355 amino acid residues (e.g., amino acids 100 to 454 of SEQ ID NO 35), at least 360 amino acid residues (e.g., amino acids 100 to 459 of SEQ ID NO 35) or at least 450 amino acid residues (e.g., amino acids 5 to 454 of SEQ ID NO 35) or at least 450 amino acid residues (e.g., amino acids 1 to 450 of SEQ ID NO 35). In one aspect, a fragment contains at least 300 amino acid residues (e.g., amino acids 1 to 300 of SEQ ID NO 34), at least 290 amino acid residues (e.g., amino acids 1 to 290 of SEQ ID NO 34), at least 275 amino acid residues (e.g., amino acids 10 to 284 of SEQ ID NO 34) or at least 270 amino acid residues (e.g., amino acids 5 to 274 of SEQ ID NO 34) or at least 260 amino acid residues (e.g., amino acids 1 to 260 of SEQ ID NO 34).

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance).

**Improved wash performance:** The term "improved wash performance" is defined herein as an enzyme displaying an increased wash performance in a detergent composition relative to the wash performance of same detergent composition without the enzyme e.g. by increased stain removal or less re-deposition. The term "improved wash performance" includes wash performance in laundry.

**Laundering:** The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

**Malodor:** The term "malodor" means an odor which is not desired on clean items. The cleaned item should smell fresh and clean without malodors adhered to the item. One example of malodor is compounds with an unpleasant smell, which may be produced by microorganisms. Another example is unpleasant smells can be sweat or body odor adhered to an item which has been in contact with human or animal. Another example of malodor can be the odor from spices, which sticks to items for example curry or other exotic spices which smells strongly.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide consisting essentially of amino acids 27 to 378 of SEQ ID NO 9 and amino acids 1 to 26 of SEQ ID NO 9 comprise a signal peptide. In one aspect, the mature is the polypeptide is the amino acid sequence shown in SEQ ID NO 17. In another aspect, the mature polypeptide consisting essentially of amino acids 26 to 349 of SEQ ID NO 10 and amino acids 1 to 25 of SEQ ID NO 10 are a signal peptide. In one aspect, the mature is the polypeptide is the amino acid sequence shown in SEQ ID NO 18. In another aspect, the mature polypeptide consisting essentially of amino acids 42 to 365 of SEQ ID NO 11 and amino acids 1 to 41 of SEQ ID NO 11 are a signal peptide. In one aspect, the mature is the polypeptide is the amino acid sequence shown in SEQ ID NO 19. In another aspect, the mature polypeptide consisting essentially of amino acids 25 to 485 of SEQ ID NO 12 and amino acids 1 to 24 of SEQ ID NO 12 are a signal peptide. In one aspect, the mature polypeptide is the amino acid sequence shown in SEQ ID NO 20. In another aspect, the mature polypeptide consisting essentially of amino acids 42 to 486 of SEQ ID NO 25 and amino acids 1 to 41 of SEQ ID NO 25 are a signal peptide. In one aspect, the mature polypeptide is the amino acid sequence shown in SEQ ID NO 33. In another aspect, the mature polypeptide consisting essentially of amino acids 42 to 486 of SEQ ID NO 26 and amino acids 1 to 41 of SEQ ID NO 26 are a signal peptide. In one aspect, the mature polypeptide is the amino acid sequence shown in SEQ ID NO 34. In another aspect, the mature polypeptide consisting essentially of amino acids 24 to 481 of SEQ ID NO 27 and amino acids 1 to 23 of SEQ ID NO 27 are a signal peptide. In one aspect, the mature polypeptide is the amino acid sequence shown in SEQ ID NO 35. In another aspect, the mature polypeptide consisting essentially of amino acids 26 to 475 of SEQ ID NO 28 and amino acids 1 to 25 of SEQ ID NO 28 are a signal peptide.

It is known in the art that a host cell may produce a mixture of two of more different mature polypeptides (i.e., with a different C-terminal and/or N-terminal amino acid) expressed by the same polynucleotide. It is also known in the art that different host cells process polypeptides differently, and thus, one host cell expressing a polynucleotide may produce a different mature polypeptide (e.g., having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having hexosaminidase activity. In one aspect, the mature polypeptide coding sequence consisting essentially of nucleotides 73 to 1455 of SEQ ID NO 16 or the cDNA sequence thereof and nucleotides 1 to 72 of SEQ ID NO 16 are the signal peptide. In one aspect, the mature polypeptide coding sequence consisting essentially of nucleotides 124 to 1458 of SEQ ID NO 29 or the cDNA sequence thereof and nucleotides 1 to 123 of SEQ ID NO 29 are the signal peptide. In one aspect, the mature polypeptide coding sequence consisting essentially of nucleotides 124 to 1458 of SEQ ID NO 30 or the cDNA sequence thereof and nucleotides 1 to 123 of SEQ ID NO 30 are the signal peptide. In one aspect, the mature polypeptide coding sequence consisting essentially of nucleotides 70 to 1443 of SEQ ID NO 31 or the cDNA sequence thereof and nucleotides 1 to 69 of SEQ ID NO 31 are the signal peptide. In one aspect, the mature polypeptide coding sequence consisting essentially of nucleotides 76 to 1425 of SEQ ID NO 32 or the cDNA sequence thereof and nucleotides 1 to 75 of SEQ ID NO 32 are the signal peptide.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic and/or which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) is used as the percent identity and is calculated as follows:

(Identical Residues x 100)/ (Length of Alignment – Total Number of Gaps in Alignment)

**Stringency conditions:** The different stringency conditions are defined as follows.

The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.8X SSC, 0.2% SDS at 55°C.

The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.8X SSC, 0.2% SDS at 60°C.

The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.8X SSC, 0.2% SDS at 65°C.

The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.8X SSC, 0.2% SDS at 70°C.

The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.4X SSC, 0.2% SDS at 70°C.

The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.4X SSC, 0.2% SDS at 75°C.

**Subsequence:** The term "subsequence" means a polynucleotide having one or more (*e.g.*, several) nucleotides absent from the 5' and/or 3' end of a mature polypeptide coding sequence; wherein the subsequence encodes a fragment having hexosaminidase activity.

**Substantially pure polypeptide:** The term "substantially pure polypeptide" means a preparation that contains at most 10%, at most 8%, at most 6%, at most 5%, at most 4%, at most 3%, at most 2%, at most 1%, and at most 0.5% by weight of other polypeptide material with which it is natively or recombinantly associated. Preferably, the polypeptide is at least 92% pure, e.g., at least 94% pure, at least 95% pure, at least 96% pure, at least 97% pure, at least 98% pure, at least 99%, at least 99.5% pure, and 100% pure by weight of the total polypeptide material present in the preparation. The polypeptides of the present invention are preferably in a substantially pure form. This can be accomplished, for example, by preparing the polypeptide by well-known recombinant methods or by classical purification methods.

**Variant:** The term "variant" means a polypeptide having hexosaminidase activity comprising an alteration, *i.e.*, a substitution, insertion, and/or deletion, of one or more (several) amino acid residues at one or more (*e.g.*, several) positions. A substitution means replacement of the amino acid occupying a position with a different amino acid; a deletion means removal of the amino acid occupying a position; and an insertion means adding 1, 2, 3, 4, 5 etc. amino acids adjacent to and immediately following the amino acid occupying the position.

In one aspect, a hexosaminidase variant may comprise from 1 to 5; from 1 to 10; from 1 to 15; from 1 to 20; from 1 to 25; from 1 to 30; from 1 to 35; from 1 to 40; from 1 to 45; or from 1-50, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 alterations and have at least 20%, e.g., at least 40%, at least 50%, at least 60%, at least 70%, at least 80%, at least 90%, at least 95%, or at least 100% of the hexosaminidase activity of the parent hexosaminidase, such as SEQ ID NO 20.

In one aspect, a hexosaminidase variant may comprise from 1 to 5; from 1 to 10; from 1 to 15; from 1 to 20; from 1 to 25; from 1 to 30; from 1 to 35; from 1 to 40; from 1 to 45; or from 1-50, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 alterations and have at least 20%, e.g., at least 40%, at least 50%, at least 60%, at least 70%, at least 80%, at least 90%, at least 95%, or at least 100% of the hexosaminidase activity of the parent hexosaminidase, such as SEQ ID NO 33.

In one aspect, a hexosaminidase variant may comprise from 1 to 5; from 1 to 10; from 1 to 15; from 1 to 20; from 1 to 25; from 1 to 30; from 1 to 35; from 1 to 40; from 1 to 45; or from 1-50, i.e. 1, 2, 3, 4, 5, 6, 7, 8, 9, 10, 11, 12,

13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 alterations and have at least 20%, e.g., at least 40%, at least 50%, at least 60%, at least 70%, at least 80%, at least 90%, at least 95%, or at least 100% of the hexosaminidase activity of the parent hexosaminidase, such as SEQ ID NO 34.

In one aspect, a hexosaminidase variant may comprise from 1 to 5; from 1 to 10; from 1 to 15; from 1 to 20; from 1 to 25; from 1 to 30; from 1 to 35; from 1 to 40; from 1 to 45; or from 1-50, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 alterations and have at least 20%, e.g., at least 40%, at least 50%, at least 60%, at least 70%, at least 80%, at least 90%, at least 95%, or at least 100% of the hexosaminidase activity of the parent hexosaminidase, such as SEQ ID NO 35.

**Nomenclature**

For purposes of the present invention, the nomenclature [IV] or [I/V] means that the amino acid at this position may be isoleucine (Ile, I) or valine (Val, V). Likewise, the nomenclature [LVI] and [L/V/I] means that the amino acid at this position may be a leucine (Leu, L), valine (Val, V) or isoleucine (Ile, I), and so forth for other combinations as described herein. Unless otherwise limited further, the amino acid X is defined such that it may be any of the 20 natural amino acids.

**Detailed Description of the Invention**

The present invention relates to compositions as defined herein comprising polypeptides having hexosaminidase activity preferably PNAG (poly-N-acetylglucosamine) activity. Organic matter such as biofilm produces EPS (extra polymeric substances), which often comprises polysaccharides such as PNAG. The polypeptides described herein are therefore effective in preventing, reducing and removing organic components such as PNAG. Organic matter such as biofilm associated with cleaning processes e.g. in textiles such as laundry is an important challenge since it may be associated with consumer relevant problems such as e.g. malodor and re-deposition. Several attempts have been made to solve various aspects of biofilm related problems in laundry many of which relate to removal of malodor. WO2014/087011 describes the use of a deoxyribonuclease (DNase) and other enzymes for reducing malodor from laundry and/or textile, WO99/09143 describes the use of one or more oxidoreductases in combination with a mediator for the reduction of malodor and WO2012/112718 describes a method for inhibiting production of laundry malodor caused by bacteria by using various strains of *Bacillus.* The present invention relates to cleaning e.g. detergent compositions comprising polypeptides preferably having a Glyco_hydro_20 catalytic domains as defined PFAM (PF00728, Pfam version 31.0 Finn (2016). Nucleic Acids Research, Database Issue 44: D279-D285) optionally wherein the GH20 catalytic domain gives a domT score of 150 or more when queried using a Profile Hidden Markov Model prepared using SEQ ID NOs: 1 to 7 using the software program hmmbuild, the query being carried out using the hmmscan software program with default settings and wherein the polypeptide has hexosaminidase activity. According to the present invention, the polypeptide comprises one or more Glyco_hydro_20 catalytic domains, wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41). The motif GXDE (SEQ ID NO 41) is situated in positions corresponding to positions 275 to 278 in *Curtobacterium luteum* (SEQ ID NO 34). Residues D and E are the key catalytic residues of hexosaminidases (position 277 to 278 in SEQ ID NO 34).

The invention relates to compositions, as defined herein, comprising polypeptides comprising one or more of the motif(s) [IV]P[ED][LVI]DXP[AN]H (SEQ ID NO 21), NYN[AS]Y[SY]LY (SEQ ID NO 22) or GXDE (SEQ ID NO 41), wherein the polypeptides have hexosaminidase activity, preferably β-N-acetylglucosamininidase activity, such as PNAG activity. In one particular aspect, the polypeptide is obtained from the genus *Curtobacterium.* Preferably the polypeptides belong to the ARAY clade which comprises polypeptides of bacterial origin, having PNAG activity. The polypeptides of the clade comprise the motif example ARAYYPV (SEQ ID NO 42), corresponding to pos 124 to 130 of SEQ ID NO 34, where R at position 125 in SEQ ID NO 34 is part of the active site. Another motif which may be comprised by the polypeptides of the ARAY clade is AWNDGID (SEQ ID NO 43), corresponding to 306 to 312 in SEQ ID NO 34. A further motif which may be comprised by the polypeptides of the ARAY clade is DDQNVGI (SEQ ID NO 44), corresponding to amino acids 154 to 160 in SEQ ID NO 34. An additional motif which may be comprised by the polypeptides of the ARAY clade is DPRIH (SEQ ID NO 45), corresponding to amino acids 320 to 324 in SEQ ID NO 34

One aspect of the invention relates to compositions as defined herein comprising polypeptides comprising the motif(s) [IV]P[ED][LVI]DXP[AN]H (SEQ ID NO 21), NYN[AS]Y[SY]LY (SEQ ID NO 22), and/or GXDE (SEQ ID NO 41). The polypeptide may further comprise the motif(s) ARAYYPV (SEQ ID NO 42), AWNDGID (SEQ ID NO 43), DDQNVGI (SEQ ID NO 44) or DPRIH (SEQ ID NO 45), wherein the polypeptides have hexosaminidase activity, preferably β-N-acetylglucosamininidase activity, such as PNAG activity.

An alignment of the polypeptides described herein and comprised in the clade is shown in Figure 1. A phylogenetic tree of the ARAY clade is shown in Figure 2.

Also disclosed are cleaning/laundering methods and method for deep cleaning of an item such as a hard surface or a textile and the use of a composition as defined in the claims. In particular, polypeptides comprising one or more Glyco_hydro_20 catalytic domains, such as the conserved motif I [IV]P[ED][LVI]DXP[AN]H (SEQ ID NO 21), conserved motif II NYN[AS]Y[SY]LY (SEQ ID NO 22) or conserved motif III GXDE (SEQ ID NO 41) and which have hexosaminidase activity are useful in laundering methods for deep cleaning of items being washed. The inventors have surprisingly found that polypeptides having hexosaminidase activity and which comprises one or more GH20 catalytic domain, wherein the GH20 catalytic domain gives a domT score of 150 or more when queried as described above e.g. polypeptides comprising one or more of the motifs [IV]P[ED][LVI]DXP[AN]H, NYN[AS]Y[SY]LY or GXDE are particularly useful for deep cleaning of laundry and hard surfaces. Dispersin B (DspB) which belongs to the GH20 family is known for its PNAG removal and reduction properties e.g. WO200406117 describe compositions comprising DspB. The composition may include a detergent, which may be anionic, cationic, or non-ionic. However, there is no indication in the art of the use of DspB in cleaning processes such as laundry or in detergent compositions comprising e.g. builders and/or bleaches. To be useful in cleaning processes the enzymes need to perform its action in detergents under the conditions of cleaning processes such as laundry, which includes stability in the presence of detergent components such as surfactants, builders and bleach components. The components of a detergent may significantly effect on the performance of the enzymes such as DspB. The present application relates to another group of dispersins, which are distinguishable from dispersin B. The polypeptides having hexosaminidase activity comprises one or all the motifs [IV]P[ED][LVI]DXP[AN]H (SEQ ID NO 21), NYN[AS]Y[SY]LY (SEQ ID NO 22) or GXDE (SEQ ID NO 41), preferably is obtained from *Curtobacterium* and are preferably comprised in the ARAY clade. The ARAY clade comprises polypeptides comprising one or more of the motifs ARAYYPV (SEQ ID NO 42), AWNDGID (SEQ ID NO 43), DDQNVGI (SEQ ID NO 44) or DPRIH (SEQ ID NO 45). The inventors surprisingly show that polypeptides having hexosaminidase activity and which comprises one or more catalytic domain, and optionally one or more of the motifs [IV]P[ED][LVI]DXP[AN]H (SEQ ID NO 21), NYN[AS]Y[SY]LY (SEQ ID NO 22), GXDE (SEQ ID NO 41) and/or one or more of the motifs ARAYYPV (SEQ ID NO 42), AWNDGID (SEQ ID NO 43), DDQNVGI (SEQ ID NO 44) or DPRIH (SEQ ID NO 45), wherein the GH20 catalytic domain preferably gives a domT score of 150 or more when queried as described above, are useful for reduction and/or removing of organic matter e.g. EPS and/or PNAG associated with cleaning e.g. on textiles or washing machines.

For example, the polypeptide of SEQ ID NO 8 comprise a Glyco_hydro_20 catalytic domain and gives a domT score of 282 when queried using a Profile Hidden Markov Model prepared as described below, displays hexosaminidase activity (see Assay 1 and e.g. table 2 of example 4) and deep cleaning capability in the presence of detergent (see table 3 of example 5).

**Cleaning Compositions Comprising Polypeptides Having Hexosaminidase Activity**

One aspect, the invention relates to a cleaning composition, as defined herein, comprising one or more polypeptides having hexosaminidase activity, wherein polypeptide comprises one or more GH20 catalytic domains, wherein the polypeptide comprises one or more GH20 catalytic domains, wherein the GH20 catalytic domain gives a domT score of 150 or more when queried using a Profile Hidden Markov Model prepared using SEQ ID NOs: 1 to 7 using the software program hmmbuild, the query being carried out using the hmmscan software program with default settings.

The theory behind Profile HMMs as described in Durbin et al. (Biological sequence analysis: probabilistic models of proteins and nucleic acids, Cambridge University Press, 1998) and Krogh et al. (1994 J. Mol. Biol. 235:1501- 1531), both incorporated herein by reference, is characterization of a set of proteins based on the probability of each amino acid occurring at each position in the alignment of the proteins of the set.

A GH20 domain is defined in the following manner. SEQ ID NOs: 1 to 7, which are partial sequences of the Uniprot entries or of the polypeptide sequences disclosed herein, as explained in the 'overview of sequence listing' section, are aligned using the software program MUSCLE v3.8.31 with the default settings. Using this alignment, a hidden Markov model (HMM) is built for the GH20 domain as an extension of the state-of-the-art GH20 domain definition. The HMM is constructed using the software program 'hmmbuild' from the package HMMER 3.1b2 (February 2015) (http://hmmer.org/) and the software is invoked using the default settings.

A GH20 domain is defined to match the above mentioned HMM using the software program 'hmmscan' from the package HMMER 3.1b2 (February 2015) (http://hmmer.org/) using the default settings if the domT score is 150 or more. In a preferred embodiment, the domT score is 160 or more, preferably 180 or more, more preferably 190 or more, even more preferably 200 or more, or most preferably 250 or more.

The HMM profile of the GH20 domain as generated using SEQ ID NOs: 1 to 7 according to the procedure above is given in example 1. The HMM profile can be copied into a text file which is subsequently loaded into the software program 'hmmscan' so that other polypeptides can be tested to see whether the polypeptide comprises one or more GH20 catalytic domains.

Likewise, the HMM profile of the Glyco_hydro_20 domain has been generated by Pfam (PF00728, http://pfam.xfam.org/family/PF00728) using hmmbuild and parameters specified (http://pfam.xfam.org/family/PF00728#tabview=tab6). The HMM profile can be obtained from Pfam (http://pfam.xfam.org/family/PF00728) and copied into a text file which is subsequently loaded into the software program 'hmmscan' so that other polypeptides can be tested to see whether the polypeptide comprises one or more Glyco_hydro_20 catalytic domains. In some aspects, the invention relates to a cleaning composition comprising one or more polypeptides having hexosaminidase activity, wherein polypeptide comprises one or more Glyco_hydro_20 catalytic domains, wherein the Glyco_hydro_20 catalytic domain comprises one or more motifs selected from motif I: [IV]P[ED][LVI]DXP[AN]H (SEQ ID NO 21) or one or more motif II NYN[AS]Y[SY]LY (SEQ ID NO 22).

Some aspects of the invention relate to the use in a cleaning process of a cleaning composition, as defined herein, comprising a polypeptide comprising a GH20 catalytic domain having a domT score of 150 or more when queried using a Profile Hidden Markov Model prepared using SEQ ID NOs: 1 to 7 using the software program hmmbuild as describe above, preferably comprising one or more motifs selected from motif I: [IV]P[ED][LVI]DXP[AN]H (SEQ ID NO 21) and/or one or more motif II NYN[AS]Y[SY]LY (SEQ ID NO 22), wherein the polypeptide has hexosaminidase activity. Some aspects of the invention relates to detergent compositions as defined herein comprising a polypeptide comprising a GH20 catalytic domain having a domT score of 150 or more when queried using a Profile Hidden Markov Model prepared using SEQ ID NOs: 1 to 7 using the software program hmmbuild as describe above, preferably comprising one or more motifs selected from motif I: [IV]P[ED][LVI]DXP[AN]H (SEQ ID NO 21) and/or one or more motif II NYN[AS]Y[SY]LY (SEQ ID NO 22), wherein the polypeptide has hexosaminidase activity.

One aspect of the invention relates to a cleaning composition, as defined herein, comprising one or more polypeptides having hexosaminidase activity, whereinthe polypeptide comprises one or more GH20 catalytic domains, wherein the GH20 catalytic domain gives a domT score of 150 or more when queried using a Profile Hidden Markov Model prepared using SEQ ID NOs: 1 to 7 inclusive using the software program hmmbuild, the query being carried out using the hmmscan software program with default settings

In a preferred aspect, the polypeptide has N-acetylglucosaminidase activity and/or β-N-acetylglucosamininidase activity. The polypeptide has hexosaminidase activity, preferably N-acetylglucosaminidase activity and/or β-N-acetylglucosamininidase activity and has at least 93%, at least 94%, at least 95%, at least 96%, at least 97%, at least 98%, at least 99% or 100% sequence identity to the mature polypeptide of SEQ ID NO 20, SEQ ID NO 33, SEQ ID NO 34 or SEQ ID NO 35. One aspect relates to a cleaning composition, as defined herein, comprising one or more polypeptides having hexosaminidase activity, preferably N-acetylglucosaminidase activity and/or β-N-acetylglucosamininidase activity wherein the polypeptide comprises one or more GH20 catalytic domains, wherein the GH20 catalytic domain gives a domT score of 150 or more when queried using a Profile Hidden Markov Model prepared using SEQ ID NOs: 1 to 7 inclusive using the software program hmmbuild, the query being carried out using the hmmscan software program with default settings, wherein the polypeptide has at least 93%, at least 94%, at least 95%, at least 96%, at least 97%, at least 98%, at least 99% or 100% sequence identity to the mature polypeptide of SEQ ID NO 20, SEQ ID NO 33, SEQ ID NO 34 or SEQ ID NO 35.

The polypeptides comprise the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41).

One aspect of the invention relates to a cleaning composition, as defined herein, comprising one or more polypeptides having hexosaminidase activity, wherein the polypeptide comprises one or more Glyco_hydro_20 catalytic domains, wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41).

The polypeptides having hexosaminidase activity, preferably N-acetylglucosaminidase activity and/or β-N-acetylglucosamininidase activity.

One aspect of the invention relates to a cleaning composition, as defined herein, comprising one or more polypeptides having hexosaminidase activity, preferably N-acetylglucosaminidase activity and/or β-N-acetylglucosamininidase activity wherein the polypeptide comprises one or more Glyco_hydro_20 catalytic domains, wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41).

The polypeptide has hexosaminidase activity, preferably N-acetylglucosaminidase activity and/or β-N-acetylglucosamininidase activity and has at least 93%, at least 94%, at least 95%, at least 96%, at least 97%, at least 98%, at least 99% or 100% sequence identity to the mature polypeptide of SEQ ID NO 20, SEQ ID NO 33, SEQ ID NO 34 or SEQ ID NO 35.

The invention relates to a cleaning composition, as defined herein, comprising one or more polypeptides having hexosaminidase activity, preferably N-acetylglucosaminidase activity and/or β-N-acetylglucosamininidase activity whereinthe polypeptide comprises one or more Glyco_hydro_20 catalytic domains, wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41),

wherein the polypeptide has at least 93%, at least 94%, at least 95%, at least 96%, at least 97%, at least 98%, at least

99% or 100% sequence identity to the mature polypeptide of SEQ ID NO 20, SEQ ID NO 33, SEQ ID NO 34 or SEQ ID NO 35.

The polypeptide preferably further comprises one or more of the motifs ARAYYPV (SEQ ID NO 42), AWNDGID (SEQ ID NO 43), DDQNVGI (SEQ ID NO 44) or DPRIH (SEQ ID NO 45).

One aspect of the invention relates to a cleaning composition, as defined herein, wherein the polypeptide further comprises one or more of the motifs ARAYYPV (SEQ ID NO 42), AWNDGID (SEQ ID NO 43), DDQNVGI (SEQ ID NO 44) or DPRIH (SEQ ID NO 45).

One aspect of the invention relates to a cleaning composition, as defined herein, comprising one or more polypeptides having hexosaminidase activity, preferably N-acetylglucosaminidase activity and/or β-N-acetylglucosaminidase activity wherein the polypeptide further comprises one or more of the motifs ARAYYPV (SEQ ID NO 42), AWNDGID (SEQ ID NO 43), DDQNVGI (SEQ ID NO 44) or DPRIH (SEQ ID NO 45),
wherein the polypeptide has at least 93%, at least 94%, at least 95%, at least 96%, at least 97%, at least 98%, at least 99% or 100% sequence identity to the mature polypeptide of SEQ ID NO 20, SEQ ID NO 33, SEQ ID NO 34 or SEQ ID NO 35.

The amount of polypeptide may be in the range of 0.00004-100 ppm, such as in the range of 0.00008-50 ppm, in the range of 0.00001-20, in the range of 0.0002-20 ppm, in the range of 0.0001-50 ppm, in the range of 0.0002-50, in the range of 0.0004-50, in the range of 0.0008-50, in the range of 0.001-50 ppm, 0.01-50 ppm, preferably 0.0001-50 ppm, more preferably 0.0002-20 ppm, more preferably 0.0002-10 ppm, more preferably 0.001-10 ppm, and most preferably 0.002-10 ppm. The hexosaminidase may be in an amount corresponding to at least 0.00001 ppm, such as at least 0.00002 ppm, at least 0.0001 ppm, at least 0.0002 ppm, at least 0.0005 ppm, at least 0.001 ppm, at least 0.002 mg ppm, at least 0.005 ppm, at least 0.01 ppm or at least 0.02 ppm. The composition may comprise at least 0.00008%, preferably at least 0.0000.1 %, 0.00002%, 0.000.1%, 0.0002%, 0.001%, 0.002%, 0.003%, 0.004%, 0.005%, 0.006%, 0.008%, 0.01%, 0.02%, 0.03%, 0.05%, 0.1%, 0.2%, 0.3%, 0.4%, 0.6%, 0.7%, 0.8%, 0.9% or 1.0% hexosaminidase.

The cleaning compositions, as defined herein, comprising one or more polypeptides having hexosaminidase activity, wherein the polypeptide comprises one or more Glyco_hydro_20 catalytic domains, wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41).

Some aspect of the invention relate to detergent compositions, as defined herein, comprising a) a polypeptide comprising a Glyco_hydro_20 catalytic domain, preferably comprising one or both motif(s) [IV]P[ED][LVI]DXP[AN]H (SEQ ID NO 21) or NYN[AS]Y[SY]LY (SEQ ID NO 22), wherein the polypeptide is selected from polypeptides comprising an amino acid sequence shown in SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 37, SEQ ID NO 38, SEQ ID NO 39, SEQ ID NO 40 and polypeptides having at least 60% e.g. 80 %, 85%, 90%, 95%, 98% or 100% sequence identity hereto, wherein the polypeptide has hexosaminidase activity and b) at least one cleaning ingredient such as a surfactant, preferably at least one surfactant selected from the group consisting of anionic, nonionic and/or cationic surfactants.

Some aspects of the invention relate the use in a cleaning process of a cleaning composition, as defined herein, comprising a polypeptide comprising SEQ ID NO 20 or a polypeptide having at least 93%, 95%, 98% or 100% sequence identity hereto, wherein the polypeptide has hexosaminidase activity. Some aspects of the invention relate to detergent compositions, as defined herein, comprising a polypeptide comprising the amino acid sequence shown in SEQ ID NO 20 or a polypeptide having at least 93%, 95%, 98% or 100% sequence identity hereto, wherein the polypeptide has hexosaminidase activity.

Some aspects of the invention relate the use in a cleaning process of a cleaning composition, as defined herein, comprising a polypeptide comprising SEQ ID NO 33 or a polypeptide having at least 93%, 95%, 98% or 100% sequence identity hereto, wherein the polypeptide has hexosaminidase activity. Some aspects of the invention relate to detergent compositions as defined herein comprising a polypeptide comprising the amino acid sequence shown in SEQ ID NO 33 or a polypeptide having at least 93%, 95%, 98% or 100% sequence identity hereto, wherein the polypeptide has hexosaminidase activity.

Some aspects of the invention relate the use in a cleaning process of a cleaning composition, as defined herein, comprising a polypeptide comprising SEQ ID NO 34 or a polypeptide having at least 93%, 95%, 98% or 100% sequence identity hereto, wherein the polypeptide has hexosaminidase activity. Some aspects of the invention relate to detergent compositions, as defined herein, comprising a polypeptide comprising the amino acid sequence shown in SEQ ID NO 34 or a polypeptide having at least 93%, 95%, 98% or 100% sequence identity hereto, wherein the polypeptide has hexosaminidase activity.

Some aspects of the invention relate the use in a cleaning process of a cleaning composition, as defined herein, comprising a polypeptide comprising SEQ ID NO 35 or a polypeptide having at least 93%, 95%, 98% or 100% sequence identity hereto, wherein the polypeptide has hexosaminidase activity. Some aspects of the invention relate to detergent compositions, as defined herein, comprising a polypeptide comprising the amino acid sequence shown in SEQ ID NO 35 or a polypeptide having at least 93%, 95%, 98% or 100% sequence identity hereto, wherein the polypeptide has

hexosaminidase activity.

Some aspect of the invention relates to a detergent composition, as defined herein, comprisingat least 0.01 ppm of active enzyme polypeptide, wherein the polypeptide comprises one or more GH20 catalytic domains, wherein the GH20 catalytic domain gives a domT score of 150 or more when queried using a Profile Hidden Markov Model prepared using SEQ ID NOs: 1 to 7 inclusive using the software program hmmbuild, the query being carried out using the hmmscan software program with default settings, optionally comprising one or more motifs selected from motif I: [IV]P[ED][LVI]DXP[AN]H (SEQ ID NO 21) and/or one or more motif II NYN[AS]Y[SY]LY (SEQ ID NO 22).

Some aspect of the invention relates to a detergent composition, as defined herein, comprisingat least 0.0001 ppm of active enzyme polypeptide, wherein the polypeptide comprises one or more GH20 catalytic domains, wherein the GH20 catalytic domain gives a domT score of 150 or more when queried using a Profile Hidden Markov Model prepared using SEQ ID NOs: 1 to 7 inclusive using the software program hmmbuild, the query being carried out using the hmmscan software program with default settings, wherein the polypeptide optionally comprising one or both motif(s) [IV]P[ED][LVI]DXP[AN]H (SEQ ID NO 21) or NYN[AS]Y[SY]LY (SEQ ID NO 22), wherein the polypeptide is selected from polypeptides comprising an amino acid sequence shown in SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 37, SEQ ID NO 38, SEQ ID NO 39, SEQ ID NO 40 and polypeptides having at least 60%, e.g. 80 %, 85%, 90%, 95%, 98% or 100% sequence identity hereto, wherein the polypeptide has hexosaminidase activity.

Some aspect of the invention relates to a detergent composition, as defined herein, comprising at least 0.01 ppm of active enzyme polypeptide comprising SEQ ID NO 20 or a polypeptide having at least 93% sequence identity hereto, wherein the polypeptide has hexosaminidase activity.

Some aspect of the invention relates to a detergent composition, as defined herein, comprising at least 0.0001 ppm of active enzyme polypeptide comprising SEQ ID NO 20 or a polypeptide having at least 93%, 95%, 98% or 100% sequence identity hereto, wherein the polypeptide has hexosaminidase activity.

Some aspect of the invention relates to a detergent composition, as defined herein, comprising at least 0.01 ppm of active enzyme polypeptide comprising SEQ ID NO 33 or a polypeptide having at least 93%, 95%, 98% or 100% sequence identity hereto, wherein the polypeptide has hexosaminidase activity.

Some aspect of the invention relates to a detergent composition, as defined herein, comprising at least 0.01 ppm of active enzyme polypeptide comprising SEQ ID NO 34 or a polypeptide having at least 93%, 95%, 98% or 100% sequence identity hereto, wherein the polypeptide has hexosaminidase activity. Some aspect of the invention relates to a detergent composition, as defined herein, comprising at least 0.01 ppm of active enzyme polypeptide comprising SEQ ID NO 35 or a polypeptide having at least 93%, 95%, 98% or 100% sequence identity hereto, wherein the polypeptide has hexosaminidase activity.

All compositions described herein may comprise surfactants, preferably, if not indicated otherwise, from about 5 wt % to about 50 wt %, from about 5 wt % to about 40 wt %, from about 5 wt % to about 30 wt %, from about 5 wt % to about 20 wt %, from about 5 wt % to about 10 wt % surfactants. "About", as used herein in relation to a numerical value means said value ±10%, preferably ±5%. "About 5 wt %" thus means from 4.5 to 5.5 wt %, preferably from 4.75 to 5.25 wt %. If not indicated otherwise, the surfactant may be generally selected among nonionic, anionic and/or amphoteric surfactants. In general, bleach-stable surfactants are preferred. Preferred anionic surfactants are sulphate surfactants and in particular alkyl ether sulphates, especially C9-C15 alcohol ether sulfates, preferably ethoxylates or mixed ethoxylates/propoxylates, such as those with 1 to 30 EO, C12-C15 primary alcohol ethoxylate, , such as those with 1 to 30 EO, C8-C16 ester sulphates and C10-C14 ester sulphates, such as mono dodecyl ester sulphates. Non-limiting examples of anionic surfactants include sulfates and sulfonates, in particular, linear alkylbenzenesulfonates (LAS), in particular C12-C13 alkyl benzene sulfonates, isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof. The anionic surfactants are preferably added to the detergent in the form of salts. Suitable cations in these salts are alkali metal ions, such as sodium, potassium and lithium and ammonium salts, for example (2-hydroxyethyl) ammonium, bis(2-hydroxyethyl) ammonium and tris(2-hydroxyethyl) ammonium salts. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or

fatty acid glucamides, FAGA), as well as products available under the trade names SPAN and TWEEN, and combinations thereof. Commercially available nonionic surfactants include Plurafac ™, Lutensol™ and Pluronic™ range from BASF, Dehypon™ series from Cognis and Genapol™ series from Clariant.

In various embodiments, said surfactant preferably comprises at least one alkyl ether sulfate.

Preferred alkyl ether sulfates are those of formula (I)

$R^1\text{-O-(AO)}_n\text{-SO}_3^-\ X^+$ (I).

In formula (I) $R^1$ represents a linear or branched, substituted or unsubstituted alkyl group, preferably a linear, unsubstituted alkyl group, more preferably a fatty alcohol moiety. Preferred $R^1$ moieties are selected from the group consisting of decyl, undecyl, dodecyl, tridecyl, tetradecyl, pentadecyl, hexadecyl, heptadecyl, octadecyl, nonadecyl, eicosyl moieties and mixtures thereof, wherein those groups with an even number of carbon atoms are preferred. Particularly preferred $R^1$ moieties are derived from $C_{10}$-$C_{18}$ fatty alcohols, such as those derived from coconut oil alcohols, tallow fatty alcohols, lauryl, myristyl, cetyl or stearyl alcohol or from $C_{10}$-$C_{20}$ oxoalcohols.

AO represents an ethyleneoxide (EO) or propyleneoxide (PO) group, preferably an ethyleneoxide group. The index n represents an integer from 1 to 50, preferably from 1 to 20 and more preferably from 1 to 10. Particularly preferably, n is 1, 2, 3, 4, 5, 6, 7 or 8. X represents a monovalent cation or the n-th part of an n-valent cation, preferred are alkali metal cations, specifically $Na^+$ and $K^+$, most preferably $Na^+$. Further cations $X^+$ may be selected from $NH_4^+$, ½ $Zn^{2+}$,½ $Mg^{2+}$,½ $Ca^{2+}$,½ $Mn^{2+}$, and combinations thereof.

In various preferred embodiments, the detergent compositions comprise an alkyl ether sulfate selected from fatty alcohol ether sulfates of formula (II)

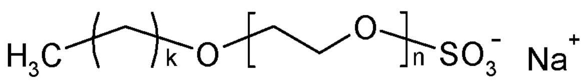

(II)

wherein k = 9 to 19, and n = 1, 2, 3, 4, 5, 6, 7 or 8. Preferred are $C_{10-16}$ fatty alcohol ether sulfates with 1-7 EO (k = 9-15, n = 1-7), such as the $C_{12-14}$ fatty alcohol ether sulfates with 1-3, particularly 2 EO (k = 11-13, n = 1-3 or 2), more particularly the sodium salts thereof. One specific embodiment thereof is lauryl ether sulfate sodium salt with 2 EO. The level of ethoxylation is an average value and can, for a specific compound, be an integer or fractional number.

In various embodiments, the surfactant comprises at least one alkyl benzene sulfonate. Said alkyl benzene sulfonate may be present alternatively to the above alkyl ether sulfate or, preferably, in addition to it.

Exemplary alkyl benzene sulfonates include, but are not limited to linear and branched alkyl benzene sulfonates, preferably linear alkyl benzene sulfonates. Exemplary compounds are those of formula (III)

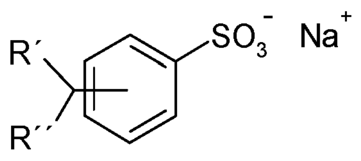

(III),

wherein R' and R" are independently H or alkyl and combined comprise 9 to 19, preferably 9 to 15 and more preferably 9 to 13 carbon atoms. Particularly preferred are dodecyl and tridecyl benzene sulfonates, in particular the sodium salts thereof.

In addition or alternatively, the compositions of the invention may further comprise one or more nonionic surfactants. Preferred nonionic surfactants are those of formula (IV)

$R^2\text{-O-(AO)}_m\text{-H}$ (IV),

wherein $R^2$ represents a linear or branched substituted or unsubstituted alkyl moiety, AO represents an ethylene oxide (EO) or propylene oxide (PO) group and m is an integer from 1 to 50.

In formula (IV) $R^2$ preferably represents a linear or branched, substituted or unsubstited alkyl group, preferably a linear, unsubstituted alkyl group, particularly preferred a fatty alcohol group. Preferred groups are $R^2$ are selected from decyl, undecyl, dodecyl, tridecyl, tetradecyl, pentadecyl, hexadecyl, heptadecyl, octadecyl, nonadecyl, eicosyl groups and combinations thereof, wherein those groups with an even number of carbon atoms are preferred. Particularly preferred are $R^2$ groups derived from $C_{12}$-$C_{18}$ fatty alcohols, such as coconut oil alcohol, tallow oil alcohol, lauryl, myristyl, cetyl

or stearyl alcohol or from $C_{10}$-$C_{20}$ oxoalcohols.

AO represents an ethyleneoxide (EO) or propyleneoxide (PO) group, preferably an ethyleneoxide group. The index m represents an integer from 1 to 50, preferably from 1 to 20 and more preferably from 1 to 6. Particularly preferably, m is 1, 2, 3, 4 or 5, most preferably 3-5, as higher degrees of ethoxylation may negatively influence viscosity and stability.

In various preferred embodiments, the detergent compositions comprise an alkyl ether selected from fatty alcohol ethers of formula (V)

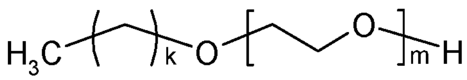

(V)

wherein k = 11 to 19, m = 1, 2, 3, 4, 5, 6, 7 or 8. Preferred are $C_{12-18}$ fatty alcohols with 1-6 EO (k = 11-17, m = 1-5 in formula (V)). More preferred are $C_{12-14}$ alcohols having 1-5 EO, most preferred are $C_{12-14}$ alkyl ethers with 3-5 EO, in particular lauryl ether with 5 EO.

The detergent compositions may further include other nonionic surfactants, such as alkyl glucosides of the general formula $RO(G)_x$, where R is a primary linear or 2-methyl-branched aliphatic radical containing 8 to 22 and preferably 12 to 18 carbon atoms and G stands for a glucose unit. The degree of oligomerization x, which indicates the distribution of monoglucosides and oligoglucosides, is a number of 1 to 10 and preferably a number of 1.2 to 1.4.

In various embodiments, the composition comprises at least two anionic surfactants, e.g. at least one alkyl ether sulfate and preferably at least one alkyl benzene sulfonate, and optionally an alkyl ether.

Suitable amphoteric surfactants comprise betains. Preferred betaines are the alkylbetaines, the alkylamidobetaines, the imidazolinium betaines, the sulfobetaines (INCI Sultaines) and the phosphobetaines. Examples of suitable betaines and sulfobetaines are the following compounds designated as INCI: almondamidopropyl betaines, apricotam idopropyl betaines, avocadamidopropyl betaines, babassuamidopropyl betaines, behenamide idopropyl betaines, behenyl betaines, betaines, canola idopropyl betaines, caprylic / capram idopropyl betaines, carnitines, cetyl betaines, Cocamidoethyl betaines, cocamidopropyl betaines, cocam idopropyl hydroxysultaines, cocobetaines, coco-hydroxysultaines, coco / oleam idopropyl betaines, coco-sultaines, decyl betaines, dihydroxyethyl oleyl glycinates, dihydroxyethyl soy glycinates, dihydroxyethyl stearyl glycinates, dihydroxyethyl tallow glycinates, dimethicones propyl PG Betaines, erucam idopropyl hydroxysultaines, hydrogenated tallow betaines, isostearam idopropyl betaines, lauram idopropyl betaines, lauryl betaines, lauryl hydroxysultaine, lauryl sultaines, milkamidopropyl betaines, minkam idopropyl betaines, myristamine idopropyl betaines, myristyl betaines, oleam idopropyl betaines, oleam idropy Hydroxysultain, Oleyl Betaine, Olivamidopropyl Betaine, Palmam Idopropyl Betaine, Palm Itam Idopropyl Betaine, Palmitoyl Carnitine, Palm Kernelamidopropyl Betaine, Polytetrafluoroethylene Acetoxypropyl Betaine, Ricinoleam Idopropyl Betaine, Sesamidopropyl Betaine, Soyamidopropyl Betaine, Stearam Idopropyl Betaine, Stearyl Betaine, Tallowam Idopropyl Betaine, Tallowamidopropyl Hydroxysultaine, Tallow Betaine, Tallow Dihydroxyethyl Betaine, Undecylenamidopropyl Betaine and Wheat Germamidopropyl Betaine. A preferred betaine is, for example, cocamidopropyl betaine (cocoamidopropylbetaine). The betaines are particularly preferred for dishwashing compositions, most preferably hand dishwashing detergent compositions.

Further suitable surfactants include the amine oxides. The amine oxides suitable in accordance with the invention include alkylamine oxides, in particular alkyldimethylamine oxides, alkylamidoamine oxides and alkoxyalkylamine oxides. Examples of suitable amine oxides are the following compounds designated as INCI: Almond amidopropylamine oxides, Babassu amidopropylamine oxides, Behenamine oxides, Cocamidopropyl Amine oxides, Cocamidopropylamine oxides, Cocamine oxides, Coco-Morpholine oxides, Decylamine oxides, Decyltetradecylamine oxides, Diaminopyrimidine oxides, Dihydroxyethyl C8-10 alkoxypropylamines oxides , Dihydroxyethyl C9-11 alkoxypropylamines oxides, dihydroxyethyl C12-15 alkoxypropylamines oxides, dihydroxyethyl cocamine oxides, dihydroxyethyl lauramine oxides, dihydroxyethyl stearamines oxides, dihydroxyethyl tallowamine oxides, hydrogenated palm kernel amine oxides, hydrogenated tallowamine oxides, hydroxyethyl hydroxypropyl C12-15 alkoxypropylamines oxides, isostearamidopropylamines Oxides, isostearamidopropyl morpholine oxides, lauram idopropylamine oxides, lauramine oxides, methyl morpholine oxides, milkamidopropyl amine oxides, mincamidopropylamine oxides, myristamine idopropylamine oxides, myristamine oxides, myristyl / cetyl amines Oxides, Oleam idopropylamine oxides, Oleamine oxides, Ol ivam idopropylam ine oxides, Palmitamidopropylamine oxides, Palmitamine oxides, PEG-3 Lauramine oxides, Potassium dihydroxyethyl Cocamine oxides phosphates, Potassium Trisphosphonomethylamine oxides, Sesamidopropylamine oxides, Soyamidopropylamine oxides, Stearam idopropylam ine oxides, stearamines Oxides, Tallowam idopropylam ine oxides, Tallowamine oxides, Undecylenamidopropylamine oxides and Wheat Germam idopropylam ine oxides. A preferred amine oxide is, for example, cocamidopropylamine oxides (cocoamidopropylamine oxide).

For automatic dishwashing applications, low-foaming nonionic surfactants are preferably used, in particular alkoxylated, especially ethoxylated, low-foaming nonionic surfactants. With particular preference, the automatic dishwashing detergents contain nonionic surfactants from the group of the alkoxylated alcohols. Particular preference is given to nonionic surfactants which have a melting point above room temperature. Nonionic surfactants having a melting point above 20 ° C, preferably above 25 ° C, more preferably between 25 and 60 ° C and especially between 26.6 and 43.3 ° C, are particularly preferred. Preferably used surfactants are those from the groups of alkoxylated nonionic surfactants, in particular the ethoxylated primary alcohols and mixtures of these surfactants with structurally more complex surfactants such as polyoxypropylene / polyoxyethylene / polyoxypropylene ((PO / EO / PO) surfactants). Such (PO / EO / PO) nonionic surfactants are also characterized by good foam control. Particularly preferred nonionic surfactants are those containing alternating ethylene oxide and different alkylene oxide units. Among these, in turn, surfactants with EO-AO-EO-AO blocks are preferred, with one to ten EO or AO groups before one block from the other group follows. Exemplary nonionic surfactants are those having a C9-alkyl group with 1 to 4 ethylene oxide units followed by 1 to 4 propylene oxide units, followed by 1 to 4 ethylene oxide units followed by 1 to 4 propylene oxide units. Preference is given in particular to end-capped, poly (oxyalkylated) nonionic surfactants with the end-cap being a linear or branched, saturated or unsaturated, aliphatic or aromatic hydrocarbon radical R having 1 to 30 carbon atoms. The alkyl groups may also comprise hydroxyl groups. The group of these nonionic surfactants include, for example, the C4-22 fatty alcohol $(EO)_{10-50}$-2- hydroxyalkyl ethers, in particular also the C8-12 fatty alcohol $(EO)_{22}$-2-hydroxydecyl ethers and the C4-22 fatty alcohol $(EO)_{40-80}$-2-hydroxyalkyl ethers.

The composition, as defined herein, may comprise, if not indicated otherwise, from 0-65 wt %, for example about 1 wt% to about 65 wt%, from about 5 wt% to about 50 wt%, preferably from about 40 wt% to 65 wt%, such as 50 - 65 wt %, particularly about 20 wt% to about 65 wt%, particularly from 10 wt% to 50 wt% of at least one builder.

Generally and if not indicated otherwise, the builder may be preferably selected from citrate, carbonate, silicate, aluminosilicate (zeolite) and combinations thereof. Suitable builders also include phosphonates, polyphosphonates, bicarbonates, borates, and further polycarboxylates. Citrate builders, e.g., citric acid and soluble salts thereof (particularly sodium salt), are particularly suitable water-soluble organic builders. Citrates can be used in combination with zeolite, silicates like the BRITESIL types, and/or layered silicate builders. The builder and/or co-builder may be any chelating agent that forms water-soluble complexes with Ca and Mg. Any builder and/or co-builder known in the art for use in cleaning detergents may be utilized. Non-limiting examples of builders include zeolites, in particular zeolite A or P or X, carbonates such as sodium carbonate, soluble silicates such as sodium metasilicate, layered silicates (e.g., SKS-6 from Hoechst), and (carboxymethyl)inulin (CMI), and combinations thereof. Further non-limiting examples of builders include aminocarboxylates, aminopolycarboxylates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycine-N,N- diacetic acid (MGDA), glutamic acid-N,N-diacetic acid (GLDA), 1-hydroxyethane-1,1-diphosphonic acid, N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(sulfomethyl)aspartic acid (SMAS), N-(2-sulfoethyl)-aspartic acid (SEAS), N-(sulfomethylglutamic acid (SMGL), N-(2-sulfoethyl)-glutamic acid (SEGL), N-methyliminodiacetic acid (MIDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA) , taurine-N,N-diacetic acid (TUDA) and N'-(2-hydroxyethyl)ethylenediamine-N,N,N'-triacetic acid (HEDTA), diethanolglycine (DEG), and combinations and salts thereof. Phosphonates suitable for use herein include 1-hydroxyethane-1,1-diphosphonic acid (HEDP), ethylenediaminetetrakis (methylenephosphonic acid) (EDTMPA), diethylenetriaminepentakis (methylenephosphonic acid) (DTMPA or DTPMPA or DTPMP), nitrilotris (methylenephosphonic acid) (ATMP or NTMP), 2-phosphonobutane-1,2,4-tricarboxylic acid (PBTC), hexamethylenediaminetetrakis (methylenephosphonic acid) (HDTMP). Particularly preferred are HEDP and DTPMP.

Suitable silicates are crystalline, layered sodium silicates of the general formula $NaMSi_xO_{2+1}*yH_2O$, wherein M is sodium or H, x a number of from 1.9 to 4 and y a number of from 0 to 20 and x is preferably 2, 3 or 4. Such silicates are for example disclosed in EP-A-0 164 514. Preferred are silicates in which M is sodium and is 2 or 3. Particularly preferred are β- and δ-sodium disilicate $Na_2Si_2O_5*yH_2O$.

Although not preferred, the compositions may also comprise phosphates, diphosphates (pyrophosphates) and/or triphosphates such as sodium triphosphate (STP or STPP).

If not indicated otherwise, the composition may also contain 0-50% by weight, such as about 5% to about 30%, of a detergent co-builder. The composition may include a co-builder alone, or in combination with a builder, for example a zeolite builder. Non-limiting examples of co-builders include homopolymers of polyacrylates or copolymers thereof, such as poly (acrylic acid) (PAA) or copoly (acrylic acid/maleic acid) (PAA/PMA) or polyaspartic acid. Further exemplary builders and/or co-builders are described in, e.g., WO 09/102854, US 5977053.

Preferred as co-builders are acrylate-containing water-soluble polymers, such as alkali metal salts of polyacrylic acid or polymethacrylic acid, for example those having a molecular weight $M_w$ in the range of 600 to 750,000 g / mol, as determined by gel permeation chromatography (GPC) according to DIN 55672-1:2007-08 with THF as an eluent.

Preferred polymers are polyacrylates with a molecular weight $M_w$ of 1,000 to 15,000 g / mol, more preferred,

due to their solubility, are short-chain polyacrylates with a molecular weight $M_w$ of 1,000 to 10,000 g / mol, most preferred from 1,000 to 5,000 g / mol.

Preferred acrylates for use in the present invention are alkali metal salts of polymers of acrylic acid, preferably the sodium salts, in particular those with molecular weights in the range of 1,000 to 10,000 g / mol or 1,000 to 5,000 g / mol. Suitable acrylates are commercially available, for example under the tradename Acusol® from Dow Chemical. Suitable are also copolymers of acrylates, in particular those of acrylic acid and methacrylic acid, and acrylic acid or methacrylic acid and maleic acid.

In preferred embodiments, the compositions of the invention comprise a sulfopolymer, preferably a copolymer comprising an ethylenically unsaturated sulfonate/sulfonic acid as a comonomer. Particularly suitable are monomers of allyl sulfonic acids, such as allyloxybenzene sulfonic acid and methallyl sulfonic acid. Particularly preferred sulfonic acid group-containing monomers are 1-acrylamido propane sulfonic acid-1, 2-acrylamido-2-propanesulfonic acid, 2-acrylamido-2-methyl-1-propanesulfonic acid, 2-methacrylamido-2-methyl-1-propanesulfonic acid, 3- methacrylamido-2-hydroxy-propanesulfonic acid, allylsulfonic acid, methallylsulfonic acid, allyloxybenzenesulfonic acid, methallyloxybenzolsulfonsäure, 2-hydroxy-3- (2-propenyloxy) propanesulfonic acid, 2-methyl-2-propenl-sulfonic acid, styrenesulfonic acid, vinylsulfonic acid, 3-sulfopropyl, 3-sulfo - propyl, sulfomethacrylamide, sulfomethylmethacrylamide and mixtures of said acids or their water-soluble salts.

The sulfopolymers are preferably copolymers of the afore-described monomers with unsaturated carboxylic acids, Especially preferred unsaturated carboxylic acids are acrylic acid, methacrylic acid, ethacrylic acid, chloroacrylic acid, alpha-cyanoacrylic acid, crotonic acid, alpha-phenylacrylic acid, maleic acid, maleic anhydride, fumaric acid, itaconic acid, citraconic acid, methylenemalonic acid, sorbic acid, cinnamic acid or mixtures thereof. Usable are of course also the unsaturated dicarboxylic acids. Preferred are copolymers with acrylates, in particular with acrylic acid and methacrylic acid, and acrylic acid or methacrylic acid and maleic acid.

Such polymers are, for example, commercially available under the trade names Acusol ®590 or Acusol® 588 from Dow Chemical.

In one aspect of the invention, the cleaning compositions of the invention comprise a polypeptide as defined herein and at least one sulfopolymer, as defined above. Such compositions are preferably dishwashing compositions.

In one preferred embodiment, the builder is a non-phosphorus based builder such as citric acid and/or methylglycine-N,N-diacetic acid (MGDA) and/or glutamic-N,N-diacetic acid (GLDA) and / or salts thereof. The compositions disclosed herein may, if not indicated otherwise, contain from about 1 wt% to about 40 wt%, preferably from about 0.5 wt% to about 30 wt%, preferably from about 0.1 wt% to about 10 wt% 0-30% by weight, such as about 1% to about 20%, such as about 1% to about 10%, such as about 1% to about 5%, such as about 10% to about 30%, such as about 5% to about 10% or such as about 10% to about 20% by weight (wt%) of a bleaching system. Any bleaching system comprising components known in the art for use in cleaning detergents may be utilized. Suitable bleaching system components include sources of hydrogen peroxide; sources of peracids; and bleach catalysts or boosters.

Suitable sources of hydrogen peroxide are inorganic persalts, including alkali metal salts such as sodium percarbonate and sodium perborates (usually mono- or tetrahydrate), and hydrogen peroxide-urea (1/1). Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and - terephthalic acids; perimidic acids; peroxymonosulfuric acid; peroxydisulfuric acid; peroxyphosphoric acid; peroxysilicic acid; and mixtures of compounds. It is understood that the peracids mentioned may in some cases be best added as suitable salts, such as alkali metal salts (e.g., Oxone®) or alkaline earth-metal salts.

b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl) oxy] benzene-1-sulfonate (ISONOBS), sodium 4-(dodecanoyloxy) benzene-1-sulfonate (LOBS), sodium 4-(decanoyloxy) benzene-1-sulfonate, 4-(decanoyloxy) benzoic acid (DOBA), sodium 4-(nonanoyloxy) benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

The bleaching system may also include a bleach catalyst or booster.

Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal compounds, such as iron or cobalt complexes.

In some embodiments, where a source of a peracid is included, an organic bleach catalyst or bleach booster may be used having one of the following formulae:

(i)

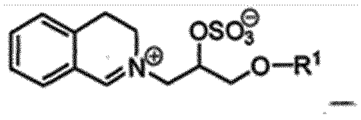

(ii)

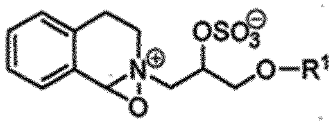

(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

In a preferred aspect, the polypeptide used in the compositions of the invention comprises the amino acids sequence with SEQ ID NO 20 or an amino acid sequence having at least 93%, such as at least 95% sequence identity to SEQ ID NO 20.

In a preferred aspect the polypeptide used in the compositions of the invention comprises the amino acids sequence with SEQ ID NO 20 or an amino acid sequence having as at least 93%, such as at least 94%, such as at least 95%, such as at least 96%, such as at least 97%, such as at least 98%, such as at least 99% or 100% sequence identity to SEQ ID NO 20.

In a preferred aspect the polypeptide used in the compositions of the invention comprises the amino acids sequence with SEQ ID NO 33 or an amino acid sequence having as at least 93%, such as at least 94%, such as at least 95%, such as at least 96%, such as at least 97%, such as at least 98%, such as at least 99% or 100% sequence identity to SEQ ID NO 33.

In a preferred aspect the polypeptide used in the compositions of the invention comprises the amino acids sequence with SEQ ID NO 34 or an amino acid sequence having at least 93%, such as at least 94%, such as at least 95%, such as at least 96%, such as at least 97%, such as at least 98%, such as at least 99% or 100% sequence identity to SEQ ID NO 34.

In a preferred aspect the polypeptide used in the compositions of the invention comprises the amino acids sequence with SEQ ID NO 35 or an amino acid sequence having at least 93%, such as at least 94%, such as at least 95%, such as at least 96%, such as at least 97%, such as at least 98%, such as at least 99% or 100% sequence identity to SEQ ID NO 35.

The composition of the invention comprising the polypeptides having hexosaminidase activity, preferably N-acetylglucosaminidase activity and/or β-N-acetylglucosamininidase activity may be used for reducing and/or removing components of biofilm e.g. EPS and/or PNAG on items such as hard surfaces, textiles and/or fabric. Organic matter such as biofilm can develop on surfaces such as textiles when microorganisms are present on the item. The organic matter maybe sticky and soils may stick to the organic matter, which may be difficult to remove. One aspect of the

invention relates to the use of a composition as described above for deep-cleaning of an item, wherein the item is a textile.

One aspect of the invention relates to the use of a composition, as described herein, comprising a polypeptide, having hexosaminidase activity, preferably N-acetylglucosaminidase activity and/or β-N-acetylglucosamininidase activity, wherein the polypeptide comprises one or more Glyco_hydro_20 catalytic domains, wherein the polypeptide comprises one or more Glyco_hydro_20 catalytic domains and wherein the polypeptide comprises the conserved motif I [IV]P[ED][LVI]DXP[AN]H (SEQ ID NO 21), the conserved motif II NYN[AS]Y[SY]LY (SEQ ID NO 22) and/or the conserved motif III GXDE (SEQ ID NO 41).

One aspect of the invention relates to the use of a composition, as described herein, comprising a polypeptide, having hexosaminidase activity, preferably N-acetylglucosaminidase activity and/or β-N-acetylglucosamininidase activity, wherein the polypeptide comprises one or more Glyco_hydro_20 catalytic domains, wherein the polypeptide further comprises one or more of the motif(s) ARAYYPV (SEQ ID NO 42), AWNDGID (SEQ ID NO 43) or DDQNVGI (SEQ ID NO 44) or DPRIH (SEQ ID NO 45).

One aspect relates to the use of a composition, as described herein, comprising a polypeptide, having hexosaminidase activity, preferably N-acetylglucosaminidase activity and/or β-N-acetylglucosamininidase activity, wherein the polypeptide comprises one or more Glyco_hydro_20 catalytic domains, wherein the polypeptide comprises one or more of the motif(s) [IV]P[ED][LVI]DXP[AN]H (SEQ ID NO 21), NYN[AS]Y[SY]LY (SEQ ID NO 22), GXDE (SEQ ID NO 41), and further comprises one or more of the motifs ARAYYPV (SEQ ID NO 42), AWNDGID (SEQ ID NO 43), DDQNVGI (SEQ ID NO 44) or DPRIH (SEQ ID NO 45),

(i) for preventing, reducing or removing stickiness of the item;
(ii) for pretreating stains on the item;
(iii) for preventing, reducing or removing redeposition of soil during a wash cycle;
(iv) for preventing, reducing or removing adherence of soil to the item;
(v) for maintaining or improving whiteness of the item;
(vi) for preventing, reducing or removal malodor from the item, wherein the item is a textile.

The polypeptide used in the compositions of the invention preferably has at least 93%, at least 94%, at least 95%, at least 96%, at least 97%, at least 98%, at least 99% or 100% sequence identity to the polypeptide shown in SEQ ID NO 20, SEQ ID NO 33, SEQ ID NO 34 or SEQ ID NO 35.

The present invention concerns the use of a composition, as described herein, comprising one or more polypeptides comprising the Glyco_hydro_20 catalytic domain and having hexosaminidase activity for deep cleaning of an item, wherein the polypeptide is a polypeptide having at least 93%, 95%, 98% or 100% sequence identity to the polypeptide comprising SEQ ID NO 20, SEQ ID NO 33, SEQ ID NO 34, or SEQ ID NO 35 and wherein the item is a textile. In one aspect of the invention the composition comprising a polypeptide comprising the Glyco_hydro_20 catalytic domain having hexosaminidase activity is used for preventing, reducing or removing the stickiness of an item, wherein the polypeptide is a polypeptide having at least 93%, 95%, 98% or 100% sequence identity to the polypeptide comprising SEQ ID NO 20, SEQ ID NO 33, SEQ ID NO 34 or SEQ ID NO 35. The polypeptide comprising the Glyco_hydro_20 catalytic domain having hexosaminidase activity can further be used for pre-treating stains on textile such as textile.

Additionally, the invention relates to the use of a composition, as defined herein, comprising a polypeptide comprising the Glyco_hydro_20 catalytic domain having hexosaminidase activity e.g. a polypeptide having at least 93%, 95%, 98% or 100% sequence identity to the polypeptide comprising SEQ ID NO 20, SEQ ID NO 33, SEQ ID NO 34 or SEQ ID NO 35 for preventing, reducing or removing re-deposition of soil during a wash cycle. When the polypeptide is used for example in the laundering of textile, the polypeptide hinders deposition of soil present in the wash liquor to deposit on the textile.

Further, the invention concerns the use of a composition, as defined herein, comprising polypeptide Glyco_hydro_20 catalytic domain having hexosaminidase activity e.g. a polypeptide having at least 93%, 95%, 98% or 100% sequence identity to the polypeptide comprising SEQ ID NO 20, SEQ ID NO 33, SEQ ID NO 34 or SEQ ID NO 35 for preventing, reducing or removing the adherence of soil to an item. In one embodiment, the item is textile. When the soil does not adhere to the item, the item appears cleaner. Thus, the invention further concerns the use of a composition, as defined herein, comprising a polypeptide comprising a Glyco_hydro_20 catalytic domain e.g. a polypeptide having at least 93%, 95%, 98% or 100% sequence identity to the polypeptide comprising SEQ ID NO 20, SEQ ID NO 33, SEQ ID NO 34 or SEQ ID NO 35 having hexosaminidase activity for maintaining or improving the whiteness of the item.

When items like T-shirts or sportswear are used, they are exposed to bacteria from the body of the user and from the rest of the environment in which they are used, such bacteria may form organic matter such as EPS (extracellular polymeric substances), which comprises PNAG. The organic matter may cause malodor on the item even after the item is washed. The polypeptides of the invention have hexosaminidase activity e.g. PNAG activity and are thus effective in preventing, reducing or removing organic components such as PNAG and the EPS comprising PNAG. The present invention therefore also concerns removal or reduction of malodor on textile. The malodor can be present on newly

washed textile which is still wet. Or the malodor can be present on newly washed textile, which has subsequently been dried. The malodor may also be present on textile, which has been stored for some time after wash. The present invention relates to reduction or removal of malodor such as E-2-nonenal from wet or dry textile.

The cleaning composition according to the invention may further comprise one or more additional components, such as a cleaning ingredient or a detergent adjunct; the detergent adjunct ingredient may be surfactants and builders and/or chelators such as those described above. The adjunct ingredients may also be any of the following flocculating aid, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, bittering agents, and/or pigments.

In various embodiments, the invention relates to cleaning compositions which comprise the polypeptides having hexosaminidase activity, as described herein, and any one or more of an adjunct ingredient selected from bittering agents and organic solvents, such as glycerol and 1 ,2-propane diol.

In one embodiment, the detergent adjunct ingredient is a builder or a clay soil removal/anti-redeposition agent.

In one embodiment, detergent adjunct ingredient is an enzyme. The one or more enzymes may be selected from the group consisting of proteases, lipases, cutinases, amylases, carbohydrases, cellulases, pectinases, mannanases, arabinases, galactanases, xylanases and oxidases.

In addition to the polypeptides having hexosaminidase activity comprising SEQ ID NO 20, SEQ ID NO 33, SEQ ID NO 34, SEQ ID NO 35, or polypeptide having at least 93% sequence identity hereto the cleaning composition of the invention may further comprise cellulases. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase polypeptides such as those described in WO 94/07998, EP 0 531 315, US 5,457,046, US 5,686,593, US 5,763,254, WO 95/24471, WO 98/12307 and PCT/DK98/00299.

Example of cellulases exhibiting endo-beta-1,4-glucanase activity (EC 3.2.1.4) are those having described in WO02/099091.

Other examples of cellulases include the family 45 cellulases described in WO96/29397, and especially polypeptides thereof having substitution, insertion and/or deletion at one or more of the positions corresponding to the following positions in SEQ ID NO 8 of WO 02/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146 R.

Commercially available cellulases include Celluzyme™, Celluclean and Carezyme™ (Novozymes A/S), Clazinase™, and Puradax HA™ (Genencor International Inc.), and KAC-500(B)™ (Kao Corporation).

In addition to the polypeptides having hexosaminidase activity comprising SEQ ID NO 20, SEQ ID NO 33, SEQ ID NO 34, SEQ ID NO 35 or polypeptide having at least 93% sequence identity hereto the cleaning composition of the invention may further comprise proteases. Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147* and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases

are trypsin (e.g. of porcine or bovine origin) and the *Fusarium* protease described in WO89/06270, WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO1 1/036263, WO11/036264, especially the variants with substitutions in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269, compared to SEQ ID NO 1 of WO 2016/001449, wherein the positions correspond to the positions of the Bacillus Lentus protease shown in SEQ ID NO 1 of WO 2016/001449. More preferred the subtilase variants may comprise one or more of the mutations: S3T, V4I, S9R, S9E, A15T, S24G, S24R, K27R, N42R, S55P, G59E, G59D, N60D, N60E, V66A, N74D, N85S, N85R, , G96S, G96A, S97G, S97D, S97A, S97SD, S99E, S99D, S99G, S99M, S99N, S99R, S99H, S101A, V102I, V102Y, V102N, S104A, G116V, G116R, H118D, H118N, N120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A or R269H. The protease variants are preferably variants of the Bacillus Lentus protease (Savinase®) shown in SEQ ID NO 1 of WO 2016/001449, the Bacillus amylolichenifaciens protease (BPN') shown in SEQ ID NO 2 of WO2016/001449. The protease variants preferably have at least 80 % sequence identity to SEQ ID NO 1 or SEQ ID NO 2 of WO 2016/001449.

A protease variant comprising a substitution at one or more positions corresponding to positions 171, 173, 175, 179, or 180 of SEQ ID NO 1 of WO2004/067737, wherein the protease variant has a sequence identity of at least 75% but less than 100% to SEQ ID NO 1 of WO2004/067737.

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase® and Esperase® (Novozymes A/S), those sold under the tradename Maxatase®, Maxacal®, Maxapem®, Purafect Ox®, Purafect OxP®, Puramax®, FN2®, FN3®, FN4®, Excellase®, Excellenz P1000™, Excellenz P1250™, Eraser®, Preferenz P100™, Purafect Prime®, Preferenz P110™, Effectenz P1000™, Purafect®™, Effectenz P1050™, Purafect Ox®™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

In addition to the polypeptides having hexosaminidase activity comprising SEQ ID NO 20, SEQ ID NO 33, SEQ ID NO 34, SEQ ID NO 35 or polypeptide having at least 93% sequence identity hereto the cleaning composition of the invention may further comprise lipases and cutinases which include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147). Other examples are lipase polypeptides such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500. Preferred commercial lipase products include include Lipolase™, Lipex™; Lipolex™ and Lipoclean™ (Novozymes A/S), Lumafast (originally from Genencor) and Lipomax (originally from Gist-Brocades). Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and polypeptides of the *M. smegmatis* perhydrolase in particular the S54V variant used in the commercial product Gentle Power Bleach from Huntsman Textile Effects Pte Ltd (WO10/100028).

In addition to the polypeptides having hexosaminidase activity comprising SEQ ID NO 20, SEQ ID NO 33, SEQ ID NO 34, SEQ ID NO 35 or polypeptide having at least 93% sequence identity hereto the cleaning composition of the invention may further comprise amylases which can be used together with a polypeptide of the invention. The amylase

may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839. Suitable amylases include amylases having SEQ ID NO 3 in WO 95/10603 or polypeptides having 90% sequence identity to SEQ ID NO 3 thereof. Preferred polypeptides are described in WO 94/02597, WO 94/18314, WO 97/43424 and SEQ ID NO 4 of WO 99/019467, such as polypeptides with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444. Different suitable amylases include amylases having SEQ ID NO 6 in WO 02/010355 or polypeptides thereof having 90% sequence identity to SEQ ID NO 6. Preferred polypeptides of SEQ ID NO 6 are those having a deletion in positions 181 and 182 and a substitution in position 193. Other amylases which are suitable are hybrid alpha-amylase comprising residues 1-33 of the alpha-amylase derived from *B. amyloliquefaciens* shown in SEQ ID NO 6 of WO 2006/066594 and residues 36-483 of the *B. licheniformis* alpha-amylase shown in SEQ ID NO 4 of WO 2006/066594 or polypeptides having 90% sequence identity thereof. Preferred polypeptides of this hybrid alpha-amylase are those having a substitution, a deletion or an insertion in one of more of the following positions: G48, T49, G107, H156, A181, N190, M197, I201, A209 and Q264. Most preferred polypeptides of the hybrid alpha-amylase comprising residues 1-33 of the alpha-amylase derived from *B. amyloliquefaciens* shown in SEQ ID NO 6 of WO 2006/066594 and residues 36-483 of SEQ ID NO 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO 6 in WO 99/019467 or polypeptides thereof having 90% sequence identity to SEQ ID NO 6. Preferred polypeptides of SEQ ID NO 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO 1, SEQ ID NO 3, SEQ ID NO 2 or SEQ ID NO 7 of WO 96/023873 or polypeptides thereof having 90% sequence identity to SEQ ID NO 1, SEQ ID NO 2, SEQ ID NO 3 or SEQ ID NO 7. Preferred polypeptides of SEQ ID NO 1, SEQ ID NO 2, SEQ ID NO 3 or SEQ ID NO 7 are those having a substitution, a deletion or an insertion in one or more of the following positions: 140, 181, 182, 183, 184, 195, 206, 212, 243, 260, 269, 304 and 476. More preferred polypeptides are those having a deletion in positions 181 and 182 or positions 183 and 184. Most preferred amylase polypeptides of SEQ ID NO 1, SEQ ID NO 2 or SEQ ID NO 7 are those having a deletion in positions 183 and 184 and a substitution in one or more of positions 140, 195, 206, 243, 260, 304 and 476. Other amylases which can be used are amylases having SEQ ID NO 2 of WO 08/153815, SEQ ID NO 10 in WO 01/66712 or polypeptides thereof having 90% sequence identity to SEQ ID NO 2 of WO 08/153815 or 90% sequence identity to SEQ ID NO 10 in WO 01/66712. Preferred polypeptides of SEQ ID NO 10 in WO 01/66712 are those having a substitution, a deletion or an insertion in one of more of the following positions: 176, 177, 178, 179, 190, 201, 207, 211 and 264. Further suitable amylases are amylases having SEQ ID NO 2 of WO 09/061380 or polypeptides having 90% sequence identity to SEQ ID NO 2 thereof. Preferred polypeptides of SEQ ID NO 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred polypeptides of SEQ ID NO 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase polypeptides of SEQ ID NO 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the polypeptides are C-terminally truncated and optionally further comprises a substitution at position 243 and/or a deletion at position 180 and/or position 181.

Other suitable amylases are the alpha-amylase having SEQ ID NO 12 in WO01/66712 or a variant having at least 90% sequence identity to SEQ ID NO 12. Preferred amylase polypeptides are those having a substitution, a deletion or an insertion in one of more of the following positions of SEQ ID NO 12 in WO01/66712: R28, R118, N174; R181, G182, D183, G184, G186, W189, N195, M202, Y298, N299, K302, S303, N306, R310, N314; R320, H324, E345, Y396,

R400, W439, R444, N445, K446, Q449, R458, N471, N484. Particular preferred amylases include polypeptides having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase polypeptides such as those described in WO2011/098531, WO2013/001078 and WO2013/001087. Commercially available amylases are Duramyl™, Termamyl™, Fungamyl™, Stainzyme™, Stainzyme Plus™, Natalase™, Liquozyme X and BAN™ (from Novozymes A/S), and Rapidase™, Purastar™/Effectenz™, Powerase and Preferenz S100 (from Genencor International Inc./DuPont).

In addition to the polypeptides having hexosaminidase activity comprising SEQ ID NO 20, SEQ ID NO 33, SEQ ID NO 34, SEQ ID NO 35 or a polypeptide having at least 93% sequence identity hereto the cleaning composition of the invention may further comprise peroxidases/oxidases including those of plant, bacterial or fungal origin. Chemically modified or protein engineered mutants are included. Examples of useful peroxidases include peroxidases from *Coprinus, e.g.,* from *C. cinereus,* and polypeptides thereof as those described in WO 93/24618, WO 95/10602, and WO 98/15257. Commercially available peroxidases include Guardzyme™ (Novozymes A/S).

The cleaning compositions of the invention may, if not indicated otherwise, also contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide anti-redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), (carboxymethyl)inulin (CMI), and polycarboxylates such as PAA, PAA/PMA, polyaspartic acid, and lauryl methacrylate/acrylic acid copolymers , hydrophobically modified CMC (HM-CMC) and silicones, copolymers of terephthalic acid and oligomeric glycols, copolymers of polyethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

The cleaning compositions of the present invention may also include fabric hueing agents such as dyes or pigments, which when formulated in detergent compositions can deposit onto a fabric when the fabric is contacted with a wash liquor comprising the cleaning e.g. detergent compositions and thus altering the tint of the fabric through absorption/reflection of visible light. Fluorescent whitening agents emit at least some visible light if subjected to ultraviolet light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

The cleaning composition may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

The cleaning compositions of the present invention can also contain dispersants. In particular, powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

The cleaning compositions of the present invention may also include one or more dye transfer inhibiting agents. Suitable polymeric dye transfer inhibiting agents include, but are not limited to, polyvinylpyrrolidone polymers, polyamine-N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

The cleaning compositions of the present invention may also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the laundry composition of the present invention. The most commonly used fluorescent whitening agents are those belonging to the classes of diaminostilbene-sulfonic acid derivatives, diarylpyrazoline derivatives and biphenyl-distyryl derivatives. Examples of the diaminostilbene-sulfonic acid derivative type of fluorescent whitening agents include the sodium salts of: 4,4'-bis[(4-anilino-6-diethanolamino-s-triazin-2-yl)amino]stilbene-2,2'-disulfonate, 4,4'-bis[(4,6-dianilino-s-triazin-2-yl)amino]stilbene-2,2'-disulfonate, 4,4'-bis{4-anilino-6-[methyl(2-hydroxyethyl)amino]-s-triazin-2-ylamino}stilbene-2,2'-disulfonate, 4,4'-bis(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from BASF. Tinopal DMS is the disodium salt of 4,4'-bis[(4-anilino-6-morpholino-s-triazin-2-yl) amino] stilbene-2,2'-disulfonate. Tinopal CBS is the disodium salt of 2,2'-[biphenyls-4,4'-di(2,1-ethenediyl)] dibenzene-1-sulfonate. Also preferred is the commercially available Parawhite KX, supplied by Paramount Minerals and Chemicals, Mumbai, India. Other fluorescers suitable for use in the invention include the 1-3-diarylpyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

The cleaning compositions of the present invention may also include one or more soil-release polymers which aid the removal of soils from fabrics such as cotton and polyester-based fabrics, in particular the removal of hydrophobic soils from polyester-based fabrics. The soil release polymers may for example be nonionic or anionic terephthalate-based polymers, polyvinylcaprolactam and related copolymers, vinyl graft copolymers or polyester polyamides; see for example Chapter 7 in Powdered Detergents, Surfactant science series volume 71, Marcel Dekker, Inc. Another type of soil release polymers is amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore, random graft co-polymers are suitable soil-release polymers. Suitable graft co-polymers are described in more detail in WO 2007/138054, WO 2006/108856 and WO 2006/113314 (hereby incorporated by reference). Other soil-release polymers are substituted polysaccharide structures especially substituted cellulosic structures such as modified cellulose derivatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof.

The cleaning compositions of the present invention may also include one or more anti-redeposition agents such as (carboxymethyl) cellulose (CMC), poly (vinyl alcohol) (PVA), homopolymers of acrylic acid, copolymers of acrylic acid and maleic acid, and ethoxylated polyethyleneimines. The cellulose based polymers described under soil-release polymers above may also function as anti-redeposition agents.

The cleaning composition of the invention may also contain one or more alternative or additional adjunct materials. Suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

The cleaning composition of the invention may be in any convenient form, e.g., a bar, a homogenous tablet, a tablet having two or more layers, a pouch having one or more compartments, such as 2 or more, preferably 2, 3, 4 or 5 compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably, the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water-soluble film. The compartment for liquid components can be different in composition than compartments containing solids: US2009/0011970 A1.

Cleaning and detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

The present invention is also directed to methods for using the compositions tdisclosed herein in laundering of textile and fabrics, such as house hold laundry washing and industrial laundry washing.

The invention is also directed to methods for using the compositions disclosed herein in cleaning hard surfaces such as floors, tables, walls, roofs etc. as well as surfaces of hard objects such as cars (car wash) and dishes (dish wash).

The cleaning process or the textile care process may for example be a laundry process, a dishwashing process or cleaning of hard surfaces such as bathroom tiles, floors, table tops, drains, sinks and washbasins. Laundry processes can for example be household laundering, but it may also be industrial laundering. Furthermore, the invention relates to a process for laundering of fabrics and/or garments where the process comprises treating fabrics with a washing solution containing a detergent composition of the invention. The cleaning process or a textile care process can for example be carried out in a machine washing process or in a manual washing process. The washing solution can for example be an aqueous washing solution containing a detergent composition.

The last few years there has been an increasing interest in replacing components in detergents, which is derived from petrochemicals with renewable biological components such as enzymes and polypeptides without compromising the wash performance. When the components of detergent compositions change new enzyme activities or new enzymes having alternative and/or improved properties compared to the common used detergent enzymes such as proteases, lipases and amylases are needed to achieve a similar or improved wash performance when compared to the traditional detergent compositions.

The polypeptides comprising the Glyco_hydro_20 catalytic domain are thus particularly useful in composition, such as detergent compositions, comprising cleaning ingredients and the polypeptides may preferably be used in cleaning processes such as laundry and dish wash.

**Novel hexosaminidases**

Disclosed herein is a polypeptide having hexosaminidase activity, wherein the polypeptide comprises one or more Glyco_hydro_20 catalytic domains and one or more domain(s) [IV]P[ED][LVI]DXP[AN]H (SEQ ID NO 21), NYN[AS]Y[SY]LY (SEQ ID NO 22), GXDE (SEQ ID NO 41).. One aspect relates to a polypeptide having hexosaminidase activity, selected from the group consisting of:

(a) a polypeptide having at least 93% sequence identity to the polypeptide of SEQ ID NO 20;
(b) a polypeptide having at least 93% sequence identity to the polypeptide of SEQ ID NO 33;
(c) a polypeptide having at least 93% sequence identity to the polypeptide of SEQ ID NO 34;
(d) a polypeptide having at least 93% sequence identity to the polypeptide of SEQ ID NO 35;
(e) a variant of the polypeptide selected from the group consisting of SEQ ID NO 20, SEQ ID NO 33, SEQ ID NO 34 or SEQ ID NO 35, wherein the variant has hexosaminidase activity and comprises one or more amino acid substitutions, and/or one or more amino acid deletions, and/or one or more amino acid insertions or any combination thereof in 1, 2, 3, 4, 5, 6, 7, 8, 9, 10, 11, 12, 13, 14, 15, 16, 17, 18, 19 or 20 positions;
(f) a polypeptide comprising the polypeptide of (a) to (e) and a N-terminal and/or C-terminal His-tag and/or HQ-tag;
(g) a polypeptide comprising the polypeptide of (a) to (e) and a N-terminal and/or C-terminal extension of between 1 and 10 amino acids;
(h) a fragment of the polypeptide of (a) to (e) having hexosaminidase activity and having at least 90% of the length of the mature polypeptide;
(i) a polypeptide comprising one or more of the motifs [IV]P[ED][LVI]DXP[AN]H (SEQ ID NO 21), NYN[AS]Y[SY]LY (SEQ ID NO 22), and/or GXDE (SEQ ID NO 41), and optionally further comprising one or more of the motifs ARAYYPV (SEQ ID NO 42), AWNDGID (SEQ ID NO 43), DDQNVGI (SEQ ID NO 44) or DPRIH (SEQ ID NO 45); and
(j) polypeptide having N-acetylglucosaminidase activity and/or β-N-acetylglucosamininidase activity.

The polypeptide preferably has at least 93%, at least 94%, at least 95%, at least 96%, at least 97%, at least 98%, at least 99% or 100% sequence identity to the polypeptide shown in SEQ ID NO 20, SEQ ID NO 33, SEQ ID NO 34 or SEQ ID NO 35.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 5 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 5.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 6 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 6.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 7 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 7.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 8 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 8.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 37 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 37.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 38 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 38.

One aspect relates to a polypeptide having hexosaminidase activity, wherein the polypeptide comprises one or more domain(s) [IV]P[ED][LVI]DXP[AN]H (SEQ ID NO 21), NYN[AS]Y[SY]LY (SEQ ID NO 22), GXDE (SEQ ID NO 41) and wherein the polypeptide comprise the amino acid sequence shown in SEQ ID NO 39 or comprise an amino acids sequence having at least 60%, such as at least 70%, such as at least 75%, such as at least 80%, such as at least 85%, such as at least 86%, such as at least 87%, such as at least 88%, such as at least 89%, such as at least 90%, such as at least 91%,, such as at least 92%, such as at least 93%, such as at least 94%, such as at least 95%, such as at least 96%, such as at least 97%, such as at least 98%, such as at least 99% or 100% sequence identity to the amino acid sequence shown in SEQ ID NO 39.

In some aspects of the invention the polypeptides used in the compositions of the invention e.g. the polypeptides having at least at least 60%, such as at least 70%, such as at least 80% or such as at least 90% sequence identity the

mature polypeptides of SEQ ID NO 12 or to the mature polypeptide with SEQ ID NO 20 have β-N-acetylglucosamininidase activity and in some aspect, the hexosaminidase activity is β-N-acetylglucosamininidase activity and the polypeptide of the invention are β-N-acetylglucosamininidases.

In some aspects of the invention the polypeptides e.g. the polypeptides having at least at least 60%, such as at least 70%, such as at least 80% or such as at least 90% sequence identity the mature polypeptides of SEQ ID NO 25 or to the mature polypeptide with SEQ ID NO 33 have β-N-acetylglucosamininidase activity and in some aspect, the hexosaminidase activity is β-N-acetylglucosamininidase activity and the polypeptide of the invention are β-N-acetylglucosamininidases.

In some aspects of the invention the polypeptides e.g. the polypeptides having at least at least 60%, such as at least 70%, such as at least 80% or such as at least 90% sequence identity the mature polypeptides of SEQ ID NO 26 or to the mature polypeptide with SEQ ID NO 34 have β-N-acetylglucosamininidase activity and in some aspect, the hexosaminidase activity is β-N-acetylglucosamininidase activity and the polypeptide of the invention are β-N-acetylglucosamininidases.

In some aspects of the invention the polypeptides e.g. the polypeptides having at least at least 60%, such as at least 70%, such as at least 80% or such as at least 90% sequence identity the mature polypeptides of SEQ ID NO 27 or to the mature polypeptide with SEQ ID NO 35 have β-N-acetylglucosamininidase activity and in some aspect, the hexosaminidase activity is β-N-acetylglucosamininidase activity and the polypeptide of the invention are β-N-acetylglucosamininidases.

The disclosure further relates to a polypeptide, comprising one or more domain(s) selected from the group consisting of [IV]P[ED][LVI]DXP[AN]H (SEQ ID NO 21), NYN[AS]Y[SY]LY (SEQ ID NO 22), and GXDE (SEQ ID NO 41), and optionally further comprises one or more domain(s) selected from the group consisting of ARAYYPV (SEQ ID NO 42), AWNDGID (SEQ ID NO 43), DDQNVGI (SEQ ID NO 44) and DPRIH (SEQ ID NO 45), wherein the polypeptide has a sequence identity to the mature polypeptide shown in SEQ ID NO 20 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% and which has hexosaminidase activity. In one aspect, the polypeptides differ by up to 49 amino acids, e.g., between 1 and 49 amino acids, such as 1-45, 1-40, 1-35, 1-30, 1-25, 1-20, 1-15, 1-10 or 1-5 amino acids, or 1, 2, 3, 4, 5, 6, 7, 8, 9, 10, 11, 12, 13 ,14, 15, 16, 17, 18, 19, 20, 21, 22, 23, 24, 25, 26, 27, 28, 29, 30, 31, 32, 33, 34, 35, 36, 37 ,38, 39, 40, 41, 42, 43, 44, 45, 46, 47, 48 or 49 amino acids from the mature polypeptide of SEQ ID NO 20. In an embodiment, the polypeptide has been isolated. In an embodiment, the polypeptide has at least at least 70%, e.g. at least 80%, at least 90%, at least 95% or at least 100% of the hexosaminidase activity of the mature polypeptide of SEQ ID NO 20.

The disclosure further relates to a polypeptide, comprising one or more domain(s) selected from the group consisting of [IV]P[ED][LVI]DXP[AN]H (SEQ ID NO 21), NYN[AS]Y[SY]LY (SEQ ID NO 22), and GXDE (SEQ ID NO 41), and optionally further comprises one or more domain(s) selected from the group consisting of ARAYYPV (SEQ ID NO 42), AWNDGID (SEQ ID NO 43), DDQNVGI (SEQ ID NO 44) and DPRIH (SEQ ID NO 45), wherein the polypeptide has a sequence identity to the mature polypeptide shown in SEQ ID NO 33 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% and which has hexosaminidase activity. In one aspect, the polypeptides differ by up to 49 amino acids, e.g., between 1 and 49 amino acids, such as 1-45, 1-40, 1-35, 1-30, 1-25, 1-20, 1-15, 1-10 or 1-5 amino acids, or 1, 2, 3, 4, 5, 6, 7, 8, 9, 10, 11, 12, 13 ,14, 15, 16, 17, 18, 19, 20, 21, 22, 23, 24, 25, 26, 27, 28, 29, 30, 31, 32, 33, 34, 35, 36, 37 ,38, 39, 40, 41, 42, 43, 44, 45, 46, 47, 48 or 49 amino acids from the mature polypeptide of SEQ ID NO 33. In an embodiment, the polypeptide has been isolated. In an embodiment, the polypeptide has at least at least 70%, e.g. at least 80%, at least 90%, at least 95% or at least 100% of the hexosaminidase activity of the mature polypeptide of SEQ ID NO 33.

The disclosure further relates to a polypeptide, comprising one or more domain(s) selected from the group consisting of [IV]P[ED][LVI]DXP[AN]H (SEQ ID NO 21), NYN[AS]Y[SY]LY (SEQ ID NO 22), and GXDE (SEQ ID NO 41), and optionally further comprises one or more domain(s) selected from the group consisting of ARAYYPV (SEQ ID NO 42), AWNDGID (SEQ ID NO 43), DDQNVGI (SEQ ID NO 44) and DPRIH (SEQ ID NO 45), wherein the polypeptide has a sequence identity to the mature polypeptide shown in SEQ ID NO 34 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% and which has hexosaminidase activity. In one aspect, the polypeptides differ by up to 49 amino acids, e.g., between 1 and 49 amino acids, such as 1-45, 1-40, 1-35, 1-30, 1-25, 1-20, 1-15, 1-10 or 1-5 amino acids, or 1, 2, 3, 4, 5, 6, 7, 8, 9, 10, 11, 12, 13 ,14, 15, 16, 17, 18, 19, 20, 21, 22, 23, 24, 25, 26, 27, 28, 29, 30, 31, 32, 33, 34, 35, 36, 37 ,38, 39, 40, 41, 42, 43, 44, 45, 46, 47, 48 or 49 amino acids from the mature polypeptide of SEQ ID NO 34. In an embodiment, the polypeptide has been isolated. In an embodiment, the polypeptide has at least at least 70%, e.g. at least 80%, at least 90%, at least 95% or at least 100% of the hexosaminidase activity of the mature polypeptide of SEQ ID NO 34.

The disclosure further relates to a polypeptide, comprising one or more domain(s) selected from the group

consisting of [IV]P[ED][LVI]DXP[AN]H (SEQ ID NO 21), NYN[AS]Y[SY]LY (SEQ ID NO 22), and GXDE (SEQ ID NO 41), and optionally further comprises one or more domain(s) selected from the group consisting of ARAYYPV (SEQ ID NO 42), AWNDGID (SEQ ID NO 43), DDQNVGI (SEQ ID NO 44) and DPRIH (SEQ ID NO 45), wherein the polypeptide has a sequence identity to the mature polypeptide shown in SEQ ID NO 35 of at least 80%, e.g., at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% and which has hexosaminidase activity. In one aspect, the polypeptides differ by up to 49 amino acids, e.g., between 1 and 49 amino acids, such as 1-45, 1-40, 1-35, 1-30, 1-25, 1-20, 1-15, 1-10 or 1-5 amino acids, or 1, 2, 3, 4, 5, 6, 7, 8, 9, 10, 11, 12, 13 ,14, 15, 16, 17, 18, 19, 20, 21, 22, 23, 24, 25, 26, 27, 28, 29, 30, 31, 32, 33, 34, 35, 36, 37 ,38, 39, 40, 41, 42, 43, 44, 45, 46, 47, 48 or 49 amino acids from the mature polypeptide of SEQ ID NO 35. In an embodiment, the polypeptide has been isolated. In an embodiment, the polypeptide has at least at least 70%, e.g. at least 80%, at least 90%, at least 95% or at least 100% of the hexosaminidase activity of the mature polypeptide of SEQ ID NO 35.

A polypeptide as disclosed herein preferably comprises or consists of the amino acid sequence of SEQ ID NO 20 or an allelic variant thereof; comprises or consists of the amino acid sequence of SEQ ID NO 20 and a N-terminal and/or C-terminal His-tag and/or HQ-tag; or is a fragment thereof having hexosaminidase activity wherein the fragment comprises at least 300 amino acids, such as at least 320 amino acids, at least 350 amino acids, at least 400 amino acids, at least 410 amino acids, at least 420 amino acids, at least 430 amino acids, at least 440 amino acids, at least 450 amino acids or at least 460 amino acids.

A polypeptide as disclosed herein preferably comprises or consists of the amino acid sequence of SEQ ID NO 33 or an allelic variant thereof; comprises or consists of the amino acid sequence of SEQ ID NO 33 and a N-terminal and/or C-terminal His-tag and/or HQ-tag; or is a fragment thereof having hexosaminidase activity wherein the fragment comprises at least 300 amino acids, such as at least 320 amino acids, at least 350 amino acids, at least 400 amino acids, at least 410 amino acids, at least 420 amino acids, at least 430 amino acids, at least 440 amino acids, at least 450 amino acids or at least 460 amino acids.

A polypeptide as disclosed herein preferably comprises or consists of the amino acid sequence of SEQ ID NO 34 or an allelic variant thereof; comprises or consists of the amino acid sequence of SEQ ID NO 34 and a N-terminal and/or C-terminal His-tag and/or HQ-tag; or is a fragment thereof having hexosaminidase activity wherein the fragment comprises at least 300 amino acids, such as at least 320 amino acids, at least 350 amino acids, at least 400 amino acids, at least 410 amino acids, at least 420 amino acids, at least 430 amino acids, at least 440 amino acids, at least 450 amino acids or at least 460 amino acids.

A polypeptide as disclosed herein preferably comprises or consists of the amino acid sequence of SEQ ID NO 35 or an allelic variant thereof; comprises or consists of the amino acid sequence of SEQ ID NO 35 and a N-terminal and/or C-terminal His-tag and/or HQ-tag; or is a fragment thereof having hexosaminidase activity wherein the fragment comprises at least 300 amino acids, such as at least 320 amino acids, at least 350 amino acids, at least 400 amino acids, at least 410 amino acids, at least 420 amino acids, at least 430 amino acids, at least 440 amino acids, at least 450 amino acids or at least 460 amino acids.

In another aspect, the present disclosure relates to a polypeptide having hexosaminidase activity encoded by a polynucleotide that hybridizes under medium-high stringency conditions, high stringency conditions, or very high stringency conditions with (i) the mature polypeptide coding sequence of SEQ ID NO 16, (ii) the cDNA sequence thereof; or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present disclosure relates to a polypeptide having hexosaminidase activity encoded by a polynucleotide having a sequence identity to the mature polypeptide coding sequence of SEQ ID NO 16 or the cDNA sequence thereof of at least 80%, *e.g.*, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another aspect, the present disclosure relates to a polypeptide having hexosaminidase activity encoded by a polynucleotide that hybridizes under medium-high stringency conditions, high stringency conditions, or very high stringency conditions with (i) the mature polypeptide coding sequence of SEQ ID NO 29, (ii) the cDNA sequence thereof; or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present disclosure relates to a polypeptide having hexosaminidase activity encoded by a polynucleotide having a sequence identity to the mature polypeptide coding sequence of SEQ ID NO 29 or the cDNA sequence thereof of at least 80%, *e.g.*, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another aspect, the present disclosure relates to a polypeptide having hexosaminidase activity encoded by a polynucleotide that hybridizes under medium-high stringency conditions, high stringency conditions, or very high

stringency conditions with (i) the mature polypeptide coding sequence of SEQ ID NO 30, (ii) the cDNA sequence thereof; or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present disclosure relates to a polypeptide having hexosaminidase activity encoded by a polynucleotide having a sequence identity to the mature polypeptide coding sequence of SEQ ID NO 30 or the cDNA sequence thereof of at least 80%, *e.g.*, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another aspect, the present disclosure relates to a polypeptide having hexosaminidase activity encoded by a polynucleotide that hybridizes under medium-high stringency conditions, high stringency conditions, or very high stringency conditions with (i) the mature polypeptide coding sequence of SEQ ID NO 31, (ii) the cDNA sequence thereof; or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

In another embodiment, the present disclosure relates to a polypeptide having hexosaminidase activity encoded by a polynucleotide having a sequence identity to the mature polypeptide coding sequence of SEQ ID NO 31 or the cDNA sequence thereof of at least 80%, *e.g.*, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another aspect, the present disclosure relates to a polypeptide having hexosaminidase activity encoded by a polynucleotide that hybridizes under medium-high stringency conditions, high stringency conditions, or very high stringency conditions with (i) the mature polypeptide coding sequence of SEQ ID NO 32, (ii) the cDNA sequence thereof; or (iii) the full-length complement of (i) or (ii) (Sambrook et al., 1989, Molecular Cloning, A Laboratory Manual, 2d edition, Cold Spring Harbor, New York). In an embodiment, the polypeptide has been isolated.

The polynucleotide of SEQ ID NO 16, SEQ ID NO 29, SEQ ID NO 30, SEQ ID NO 31, SEQ ID NO 32 or a subsequence thereof, as well as the mature polypeptide of SEQ ID NO 20, SEQ ID NO 33, SEQ ID NO 34, SEQ ID NO 35 or a fragment thereof may be used to design nucleic acid probes to identify and clone DNA encoding polypeptides having hexosaminidase activity from strains of different genera or species according to methods well known in the art. Such probes can be used for hybridization with the genomic DNA or cDNA of a cell of interest, following standard Southern blotting procedures, to identify and isolate the corresponding gene therein. Such probes can be considerably shorter than the entire sequence, but should be at least 15, *e.g.*, at least 25, at least 35, or at least 70 nucleotides in length. Preferably, the nucleic acid probe is at least 100 nucleotides in length, *e.g.*, at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having hexosaminidase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. To identify a clone or DNA that hybridizes with SEQ ID NO 16, 29, 30, 31 or 32 or a subsequence thereof, the carrier material is used in a Southern blot.

For purposes of the present invention, hybridization indicates that the polynucleotide hybridizes to a labeled nucleic acid probe corresponding to (i) SEQ ID NO 16, SEQ ID NO 29, SEQ ID NO 30, SEQ ID NO 31, SEQ ID NO 32; (ii) the mature polypeptide coding sequence of SEQ ID NO 20, SEQ ID NO 33, SEQ ID NO 34, SEQ ID NO 35; (iii) the full-length complement thereof; or (iv) a subsequence thereof; under very low to very high stringency conditions. Molecules to which the nucleic acid probe hybridizes under these conditions can be detected using, for example, X-ray film or any other detection means known in the art.

The present disclosure relates to polynucleotides encoding a polypeptide or a catalytic domain, as described herein. In an embodiment, the polynucleotide encoding the polypeptide or catalytic domain domain has been isolated.

The techniques used to isolate or clone a polynucleotide are known in the art and include isolation from genomic DNA or cDNA, or a combination thereof. The cloning of the polynucleotides from genomic DNA can be effected, *e.g.*, by using the well-known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Curtobacterium,* or a related organism and thus, for example, may be an allelic or species variant of the polypeptide encoding region of the polynucleotide. Modification of a polynucleotide encoding a polypeptide of the present invention may be necessary for synthesizing polypeptides substantially similar to the polypeptide. The

term "substantially similar" to the polypeptide refers to non-naturally occurring forms of the polypeptide.

In another embodiment, the present invention relates to compositions as described herein comprising a polypeptide having hexosaminidase activity encoded by a polynucleotide having a sequence identity to the mature polypeptide coding sequence of SEQ ID NO 16 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 29 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 30 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 31 of at least 60%, *e.g.*, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 32 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 33 of at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 34 of at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 35 of at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising a polypeptide having hexosaminidase activity encoded by a polynucleotide having a sequence identity to the mature polypeptide coding sequence of SEQ ID NO 32 or the cDNA sequence thereof of at least 80%, *e.g.*, at least 85%, at least 90%, at least 91%, at least 92%, at least 93%, at least 94%, at least 95%, at least 96%, at least 97%, at least 98%, at least 99%, or 100%. In a further embodiment, the polypeptide has been isolated.

In another embodiment, the present invention relates to compositions as described herein comprising variants of the mature polypeptide of SEQ ID NO 20, SEQ ID NO 33, SEQ ID NO 34 or SEQ ID NO 35 comprising a substitution, and/or deletion, and/or insertion at one or more (*e.g.*, several) positions. In an embodiment, the number of positions comprising one or more amino acid substitutions, and/or one or more amino acid deletions, and/or one or more amino acid insertions or any combination thereof in SEQ ID NO 20 is not more than 49, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49. In another embodiment, the number of positions comprising one or more amino acid substitutions, and/or one or more amino acid deletions, and/or one or more amino acid insertions or any combination thereof in SEQ ID NO 20, SEQ ID NO 33, SEQ ID NO 34 or SEQ ID NO 35 is between 1 and 45, such as 1-40, 1-35, 1-30, 1-25, 1-20, 1-15, 1-10 or 1-5 positions. In an embodiment, the number of positions comprising one or more amino acid substitutions, and/or one or more amino acid deletions, and/or one or more amino acid insertions or any combination thereof in SEQ ID NO 20, SEQ ID NO 33, SEQ ID NO 34 or SEQ ID NO 35 is not more than 10, *e.g.,* 1, 2, 3, 4, 5, 6, 7, 8, 9 or 10. In another embodiment, the number of substitutions and/or deletions and/or insertions in SEQ ID NO 20, SEQ ID NO 33, SEQ ID NO 34 or SEQ ID NO 35 is not more than 10, *e.g.,* 1, 2, 3, 4, 5, 6, 7, 8, 9 or 10. In a further embodiment, the number of substitutions in SEQ ID NO 20, SEQ ID NO 33, SEQ ID NO 34 or SEQ ID NO 35 is not more than 10, *e.g.,*

1, 2, 3, 4, 5, 6, 7, 8, 9 or 10. In a further embodiment, the number of conservative substitutions in SEQ ID NO 20, SEQ ID NO 33, SEQ ID NO 34 or SEQ ID NO 35 is not more than 10, *e.g.,* 1, 2, 3, 4, 5, 6, 7, 8, 9 or 10. In an embodiment, the variant has at least at least 70%, e.g. at least 80%, at least 90%, at least 95% or at least 100% of the hexosaminidase activity compared to the parent e.g. compared to SEQ ID NO 20, SEQ ID NO 33, SEQ ID NO 34 or SEQ ID NO 35.

The amino acid changes in the mature polypeptide of SEQ ID NO 20, SEQ ID NO 33, SEQ ID NO 34 or SEQ ID NO 35 described above may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for hexosaminidase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labelling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

## Sources of Polypeptides Having Hexosaminidase Activity

A polypeptide having hexosaminidase activity may be obtained from microorganisms of any genus. For purposes

of the present invention, the term "obtained from" as used herein about a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a bacterial polypeptide. In one aspect, the polypeptide is a polypeptide having hexosaminidase activity from a bacterium of the class *Actinobacteria,* such as from the order *Micrococcales,* or from the family *Microbacteriaceae,* or from the genus *Curtobacterium* or from the species *Curtobacterium oceanosedimentum, Curtobacterium flaccumfaciens, Curtobacterium luteum* or

*Curtobacterium sp. Leaf154*

It will be understood that for the aforementioned species, the invention encompasses both the perfect and imperfect states, and other taxonomic equivalents, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.*, Sambrook *et al.,* 1989, *supra*).

**Polynucleotides**

The present disclosure also relates to polynucleotides encoding a polypeptide as described herein. In an embodiment, the polynucleotide encoding the polypeptide of the present invention has been isolated.

The techniques used to isolate or clone a polynucleotide are known in the art and include isolation from genomic DNA or cDNA, or a combination thereof. The cloning of the polynucleotides from genomic DNA can be effected, *e.g.*, by using the well-known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Trichophaea* or a strain of *Trichoderma,* or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, *e.g.*, variants that differ in specific activity, thermostability, pH optimum, or the like. The variants may be constructed on the basis of the polynucleotide presented as the mature polypeptide coding sequence of SEQ ID NO 20 or the cDNA sequence thereof, *e.g.*, a subsequence thereof, and/or by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.*, Ford et al., 1991, Protein Expression and Purification 2: 95-107.

**Nucleic Acid Constructs**

The present disclosure also relates to nucleic acid constructs comprising a polynucleotide of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of

a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis crylIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene *(dagA),* and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis crylIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA

amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

## Expression Vectors

The present disclosure also relates to recombinant expression vectors comprising a polynucleotide of the present invention, a promoter, and transcriptional and translational stop signals. The various nucleotide and control

sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid ortwo or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, adeA (phosphoribosylaminoimidazole-succinocarboxamide synthase), *adeB* (phosphoribosyl-aminoimidazole synthase), *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at

least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra).*

**Host Cells**

The present disclosure also relates to recombinant host cells, comprising a polynucleotide as described herein operably linked to one or more control sequences that direct the production of a polypeptide. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, *e.g.,* a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter*, *Neisseria, Pseudomonas, Salmonella,* and *Ureaplasma.*

The bacterial host cell may be any Bacillus cell including, but not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, and Bacillus thuringiensis cells.

The bacterial host cell may also be any Streptococcus cell including, but not limited to, Streptococcus equisimilis, Streptococcus pyogenes, Streptococcus uberis, and Streptococcus equi subsp. Zooepidemicus cells.

The bacterial host cell may also be any Streptomyces cell including, but not limited to, Streptomyces achromogenes, Streptomyces avermitilis, Streptomyces coelicolor, Streptomyces griseus, and Streptomyces lividans cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an E. *coli* cell may be effected by protoplast transformation (see, e.g., Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities* of *Yeast* (Skinner, Passmore, and Davenport, editors, Soc. App. Bacteriol. Symposium Series No. 9, 1980).

The yeast host cell may be a Candida, Hansenula, Kluyveromyces, Pichia, Saccharomyces, Schizosaccharomyces, or Yarrowia cell, such as a Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, or Yarrowia lipolytica cell.

The fungal host cell may be a filamentous fungal cell. "Filamentous fungi" include all filamentous forms of the

subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell.

For example, the filamentous fungal host cell may be an Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei, or Trichoderma viride cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen etal., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

**Methods of Production**

The present disclosure also relates to methods of producing a polypeptide as disclosed herein, comprising (a) cultivating a cell, which in its wild-type form produces the polypeptide, under conditions conducive for production of the polypeptide; and optionally, (b) recovering the polypeptide. In one aspect, the cell is a *Curtobacterium* cell. In another aspect, the cell is a *Curtobacterium oceanosedimentum,* a *Curtobacterium flaccumfaciens* a *Curtobacterium luteum* or a *Curtobacterium Leaf154* cell.

The present disclosure also relates to methods of producing a polypeptide as disclosed herein, comprising (a) cultivating a recombinant host cell of the present invention under conditions conducive for production of the polypeptide; and optionally, (b) recovering the polypeptide.

One embodiment relates to a method of producing the polypeptide selected from a polypeptide comprising the amino acid sequence shown in SEQ ID NO 20, SEQ ID NO 33, SEQ ID NO 34, SEQ ID NO 35 or a polypeptide having at least 93% sequence identity hereto, comprising cultivating a cell, which in its wild-type form produces the polypeptide, under conditions conducive for production of the polypeptide e.g. further comprising recovering the polypeptide.

One embodiment relates to a method of producing a polypeptide having hexosaminidase activity, comprising cultivating the host cell under conditions conducive for production of the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermenters in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions *(e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a fermentation broth comprising the polypeptide is recovered.

The polypeptide may be purified by a variety of procedures known in the art including, but not limited to, chromatography *(e.g.,* ion exchange, affinity, hydrophobic, chromatofocusing, and size exclusion), electrophoretic procedures *(e.g.,* preparative isoelectric focusing), differential solubility *(e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Fermentation Broth Formulations or Cell Compositions**

The present disclosure also relates to a fermentation broth formulation or a cell composition comprising a polypeptide as disclosed herein. The fermentation broth product further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis *(e.g.,* expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells *(e.g.,* filamentous fungal cells) are removed, *e.g.,* by centrifugation. In some embodiments, the fermentation broth contains spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compositions may further comprise a preservative and/or anti-microbial (e.g., bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (e.g., filamentous fungal cells) are grown to saturation, incubated under carbon-limiting conditions to allow protein synthesis. In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges

were all expressly written herein.

## Assays

### Wash assays

#### Mini Launder-O-Meter (MiniLOM) Model Wash System

MiniLOM is a mini wash system in which washes are performed in 50 ml test tubes placed in a Stuart rotator. Each tube simulates one small washing machine and during an experiment, each will contain a solution of a specific detergent/enzyme system to be tested along with the soiled and unsoiled fabrics it is tested on. Mechanical stress is achieved via rotation (typically 20rpm), and the temperature is controlled by placement of the rotator in a heating cabinet/room.

#### Terg-O-timeter (TOM) wash assay

The Tergo-To-Meter (TOM) is a medium scale model wash system that can be applied to test 12 different wash conditions simultaneously. A TOM is basically a large temperature controlled water bath with up to 12 open metal beakers submerged into it. Each beaker constitutes one small top loader style washing machine and during an experiment, each of them will contain a solution of a specific detergent/enzyme system and the soiled and unsoiled fabrics its performance is tested on. Mechanical stress is achieved by a rotating stirring arm, which stirs the liquid within each beaker. Because the TOM beakers have no lid, it is possible to withdraw samples during a TOM experiment and assay for information on-line during wash.

The TOM model wash system is mainly used in medium scale testing of detergents and enzymes at US or LA/AP wash conditions. In a TOM experiment, factors such as the ballast to soil ratio and the fabric to wash liquor ratio can be varied. Therefore, the TOM provides the link between small scale experiments, such as AMSA and mini-wash, and the more time consuming full scale experiments in top loader washing machines. Equipment: The water bath with 12 steel beakers and 1 rotating arm per beaker with capacity of 500 or 1200mL of detergent solution. Temperature ranges from 5 to 80°C. The water bath must be filled up with deionised water. Rotational speed can be set up to 70 to 120rpm/min. Set temperature in the Terg-O-Tometer and start the rotation in the water bath. Wait for the temperature to adjust (tolerance is +/- 0,5°C). All beakers shall be clean and without traces of prior test material. The wash solution with desired amount of detergent, temperature and water hardness is prepared in a bucket. The detergent is allowed to dissolve during magnet stirring for 10 min. Wash solution shall be used within 30 to 60 min after preparation. 800ml wash solution is added into a TOM beaker. The wash solution is agitated at 120rpm and optionally one or more enzymes are added to the beaker. The swatches are sprinkled into the beaker and then the ballast load. Time measurement starts when the swatches and ballast are added to the beaker. The swatches are washed for 20 minutes after which agitation is terminated. The wash load is subsequently transferred from the TOM beaker to a sieve and rinse with cold tap water. The soiled swatches are separated from the ballast load. The soil swatches are transferred to a 5L beaker with cold tap water under running water for 5 minutes. The ballast load is kept separately for the coming inactivation. The water is gently pressed out of the swatches by hand and placed on a tray covered with a paper. Another paper is placed on top of the swatches. The swatches are allowed to dry overnight before subjecting the swatches to analysis, such as measuring the color intensity using a Color Eye as described herein.

The present invention is further described by the following examples that should not be construed as limiting the scope of the invention.

## Assay

### Assay 1: Testing of hexosaminidase activity

The hexosaminidase activity of the mature polypeptide with SEQ ID NO 20 was determined using 4-nitrophenyl N-acetyl-β-D-glucosaminide (Sigma-Aldrich) as substrate. The enzymatic reaction was performed in triplicates in a 96 well flat bottom polystyrene microtiter plate (Thermo Scientific) with the following conditions: 50 mM 2-(N-morpholino) ethanesulfonic acid pH 6 buffer, 1.5 mg/ml 4-nitrophenyl N-acetyl-β-D-glucosaminide and 20 μg/ml purified enzyme sample in a total reaction volume of 100 μl. Blank samples without enzyme were run in parallel. The reactions were carried out at 37°C in a Thermomixer comfort (Eppendorf). After 10 minutes of incubation, 5 μl 1 M NaOH was added to each reaction mixture to stop the enzymatic reaction. The absorbance was read at 405 nm using a POLARstar Omega plate reader (BMG LABTECH) to estimate the formation of 4-nitrophenolate ion released because of enzymatic hydrolysis of the 4-nitrophenyl N-acetyl-β-D-glucosaminide substrate.

The results are summarized in the table below. Table 1 shows the average absorbance measured at 405 nm for each reaction performed in triplicates. It is seen that the absorbance is higher for the reaction carried out with SEQ ID NO 20 compared to blank without enzyme which demonstrates that SEQ ID NO 20 exhibit hexosaminidase activity.

Table 1. Hexosaminidase activity of SEQ ID NO 20.

| Enzyme | Enzyme concentration | A405nm | ΔA405nm ($A405nm_{sample}$ - $A405nm_{blank}$) |
|---|---|---|---|
| Blank | 0 μg/ml | 0.179 | - |
| SEQ ID NO 20 | 20 μg/ml | 0.376 | 0.197 |

**Assay 2 testing of hexosaminidase activity**

The hexosaminidase activity of the mature polypeptides with SEQ ID NO 20, SEQ ID NO 33, SEQ ID NO 34 SEQ ID NO 35 and SEQ ID NO 36 (not according to the present invention), was determined using 4-Methylumbeliferyl N-acetyl-β-D-glucosaminide (Sigma-Aldrich) as substrate. The enzymatic reaction was performed in triplicates in a 96 well flat bottom polystyrene microtiter plate (Thermo Scientific) with the following conditions: 20 mM 3-morpholinopropane-1-sulfonic acid pH 7 buffer, 5 mM 4-Methylumbeliferyl N-acetyl-β-D-glucosaminide and 20 nM purified enzyme sample in a total reaction volume of 200 μl. Blank samples without enzyme were run in parallel. The reactions were carried out at ambient temperature 20-25°C. The reaction kinetics was followed immediately after mixing of enzyme and substrate using a SpectraMax M2e plate reader. Excitation wavelength was set to 368 nm and fluorescence emission reading was done at 448 nm. The reaction was followed for 30 min with 60 second intervals. Increase in fluorescence signal was used to estimate the formation of 4-Methylumbeliferyl ion released because of enzymatic hydrolysis of the 4-Methylumbeliferyl N-acetyl-β-D-glucosaminide substrate. The results are summarized in the table below. The table shows the average initial rate of reaction measured as relative fluorescence units per minute (RFU/min) using excitation at 368 nm and fluorescence emission at 448 nm for each reaction performed in triplicates. It is seen that the reaction initial rate is higher for the reaction carried out with SEQ ID NO 20, SEQ ID NO 33, SEQ ID NO 34 SEQ ID NO 35 and SEQ ID NO 36 (not according to the present invention) compared to blank without enzyme which demonstrates that SEQ ID NO 20, SEQ ID NO 33, SEQ ID NO 34 SEQ ID NO 35 and SEQ ID NO 36 (not according to the present invention) exhibit hexosaminidase activity.

Table 2. Hexosaminidase activity of SEQ ID NO 20, SEQ ID NO 33, SEQ ID NO 34 SEQ ID NO 35 and SEQ ID NO 36 (not according to the present invention). Δ Reaction initial rate (RFU/min) = (Reaction initial $rate_{sample}$ -Reaction initial $rate_{blank}$)

| Enzyme | Enzyme concentrati on | Reaction initial rate (RFU/min) | Δ Reaction initial rate (RFU/min) |
|---|---|---|---|
| Blank | 0 nM | 1.1 | - |
| SEQ ID NO 20 | 25 nM | 10.3 | 9.2 |
| SEQ ID NO 33 | 25 nM | 7.7 | 6.6 |
| SEQ ID NO 35 | 25 nM | 20.7 | 19.6 |
| SEQ ID NO 34 | 25 nM | 10.4 | 9.3 |
| SEQ ID NO 36* | 25 nM | 6.4 | 5.3 |
| *(not according to the present invention) | | | |

## Examples

### Example 1

The DNA encoding the Glyco_hydro_20 hexosaminidase having the polypeptide comprised in with SEQ ID NO 12, 25, 26 and 27 were isolated from the bacterial strains *Curtobacterium oceanosedimentum; Curtobacterium flaccumfaciens; Curtobacterium luteum, Curtobacterium oceanosedimentum* respectively, isolated from environmental soil samples collected in USA and Germany (see table 2). Chromosomal DNA from the *Curtobacterium sp* strains was isolated by QIAamp DNA Blood Mini Kit (Qiagen, Hilden, Germany) and subjected to full genome sequencing using

Illumina technology. The genome sequence was analyzed for protein sequences that have glycosyl hydrolase domains. Four Glyco_hydro_20 genes and corresponding sequences (SEQ ID NO 16, 29, 30 and 31) were identified from the *Curtobacterium sp.* strains. The Glyco_hydro_20 gene and corresponding sequence (SEQ ID NO 32) encoding the Glyco_hydro_20 hexosaminidase having polypeptide sequence SEQ ID NO 28 was found in the public database from the donor Curtobacterium sp. Leaf154 (DSM102595 strain, origin Switzerland). The codon optimized synthetic DNA encoding the mature peptide sequence of the Glyco_hydro_20 hexosaminidases were ordered from the company Geneart (SEQ ID NO 20, 33, 34, 35 and 36)

Table 3:

| Enzyme | Donor | Country of origin |
|---|---|---|
| SEQ ID NO 20 | *Curtobacterium oceanosedimentum* | USA |
| SEQ ID NO 33 | *Curtobacterium flaccumfaciens* | USA |
| SEQ ID NO 34 | *Curtobacterium luteum* | Germany |
| SEQ ID NO 35 | *Curtobacterium oceanosedimentum* | USA |
| SEQ ID NO 36* | *Curtobacterium sp. Leaf154* | Switzerland |
| *(not according to the present invention) | | |

**Example 2: Cloning and expression of Glyco_hydro_20 hexosaminidases**

The codon optimized synthetic genes encoding the mature peptide sequences of the hexosaminidase with SEQ ID NO 20, 33, 35 and 36 were inserted into a *Bacillus* expression vector as described in WO12/025577. Briefly, the DNA encoding the mature peptide of the Glyco_hydro_20 hexosaminidase gene was cloned in frame to a *Bacillus clausii* secretion signal (BcSP; with the following amino acid sequence: MKKPLGKIVASTALLISVAFSSSIASA (SEQ ID NO 23). BcSP replaced the native secretion signal in the gene. Downstream of the BcSP sequence, an affinity tag sequence was introduced to ease the purification process (His-tag; with the following amino acid sequence: HHHHHHPR (SEQ ID NO 24) The gene that was expressed therefore comprised the BcSP sequence followed by the His-tag sequence followed by the mature wild type Glyco_hydro_20 sequence. The DNA encoding the mature peptide of the Glyco_hydro_20 beta-hexosaminidase gene SEQ ID 34 was amplified from the *Curtobacterium luteum* genomic DNA by standard PCR techniques using specific primers containing an overhang to the cloning vector. The gene was consecutively cloned in frame to a Bacillus clausii secretion signal as described above. The final expression plasmid (BcSP-His-tag- Glyco_hydro_20) was transformed into a *Bacillus subtilis* expression host. The Glyco_hydro_20 BcSP-fusion gene was integrated by homologous recombination into the *Bacillus subtilis* host cell genome upon transformation. The gene construct was expressed under the control of a triple promoter system (as described in WO99/43835). The gene coding for chloramphenicol acetyltransferase was used as maker (as described in (Diderichsen et al., 1993, Plasmid 30: 312-315)). Transformants were selected on LB media agar supplemented with 6 micrograms of chloramphenicol per ml. One recombinant *Bacillus subtilis* clone containing the Glyco_hydro_20 expression construct was selected and was cultivated on a rotary shaking table in 500 ml baffled Erlenmeyer flasks each containing 100 ml yeast extract-based media. After 3-5 days' cultivation time at 30 °C to 37°C, the enzyme containing supernatant was harvested by centrifugation and the enzymes was purified by His-tag purification.

**Example 3: His tag purification method**

The His-tagged Glyco_hydro_20 hexosaminidase enzyme was purified by immobilized metal chromatography (IMAC) using $Ni^{2+}$ as the metal ion on 5 mL HisTrap Excel columns (GE Healthcare Life Sciences). The purification took place at pH 7 and the bound protein was eluted with imidazole. The purity of the purified enzyme was checked by SDS-PAGE and the concentration of the enzyme determined by Absorbance 280 nm after a buffer exchange in 50mM HEPES, 100mM NaCl pH7.0

**Example 4: Biofilm assay**

Staphylococcus *aureus* was kindly provided by Iñigo Lasa (Valle et al., Mol Microbiol.2003 May; 48 (4):1075-87). The strain was grown on trypticase soy agar (TSA) at 37°C overnight. Next day, a single colony was transferred to 15 ml tripticase soy broth (TSB) and incubated 5 hours at 37°C under shaking. The culture was diluted 1:100 in TSB+1% glucose and 100 μL of the bacterial suspension was transferred to each well of a 96-well microtiter plates (Thermo

Scientific, Nunclon Delta Surface, cat # 167008) and incubated 24 hours at 37°C without shaking. Supernatant was aspirated and wells were washed with 100 μL of 0.9% sodium chloride and filled with 100 μL of either hard water or 3.3 gr/L Model detergent A containing 0 (control) or 20, 10, 5, 2.5, 1.25, 0.62, 0.31, 0.16, 0.08, 0.04, 0.02 and 0.01 μg/mL of enzyme (the mature polypeptide having SEQ ID NO 20, 33, 34, 35 and 36). After incubation at 37°C for 1 hour, wells were washed with water and stained for 15 min with 100 μL of 0.095% crystal violet solution (SIGMA V5265). Wells were then rinsed twice with 100 μL water, dried and the plates were scanned. The lowest concentration of each enzyme that could remove the visible formation of biofilm of S. *aureus* after 1 hour incubation, in the presence and absence of detergent was determined (see Table 4). All enzymes were assayed per duplicate with similar results.

Table 4. Minimal concentration of enzyme that can reduce the visible formation of S. *aureus* after 1 hour incubation in either hard water or Model detergent A.

Table 4

| Enzyme | Minimal concentration for biofilm reduction in Model A<br>μg/mL | Minimal concentration for biofilm reduction in Hard water<br>μg/mL |
|---|---|---|
| SEQ ID NO 20 | 5 | 1.25 |
| SEQ ID NO 33 | 0.31 | 0.04 |
| SEQ ID NO 34 | 0.16 | 0.02 |
| SEQ ID NO 35 | 1.25 | 0.08 |
| SEQ ID NO 36* | 5 | 2.5 |
| *(not according to the present invention) | | |

**Example 5 Deep-cleaning of polypeptides with hexosaminidase activity in liquid model detergent**

*Staphylococcus aureus* (kind gift from Iñigo Lasa (Valle et al., Mol Microbiol.2003 May; 48 (4):1075-87) was used as model microorganism in the present example. *S.aureus* was restreaked on Tryptone Soya Agar (TSA) (pH 7.3) (CM0131; Oxoid Ltd, Basingstoke, UK) and incubated for 1 day at 37°C. A single colony was inoculated into 10 mL of TSB and the culture was incubated for 16 hours at 37°C with shaking (200 rpm). After propagation, the *S.aureus* culture was diluted (1:100) in fresh TSB + 1% glucose (24563; Roquette Freres) and 2 mL aliquots were added to the wells of 12-well polystyrene flat-bottom microplates (3512; Costar, Corning Incorporated, Corning, NY, USA), in which round swatches (diameter 2 cm) of sterile polyester (prewashed WFK30A) had been placed. Sterile TSB + 1% glucose was added to control wells. After 48 h at 37°C (static incubation), the swatches were rinsed twice with $15^0$dH water. Five rinsed swatches (sterile or with *S.aureus)* were placed in 50 mL test tubes and 10 mL of wash liquor ($15^0$dH water with 0.2 g/L iron(III) oxide nanopowder (544884; Sigma-Aldrich) with 3.33g/L liquid model A detergent) and 0.2ppm enzyme (mature polypeptide with SEQ ID NO 20) was added to each tube. Washes without enzyme were included as controls. The test tubes were placed in a Stuart rotator and incubated for 1 hour at 37°C at 20 rpm. The wash liquor was then removed, and the swatches were rinsed twice with $15^0$dH water and dried on filter paper over night.

The color difference (L) values were measured using a Handheld Minolta CR-300, and are displayed in table 5. Delta values ($L_{\text{(swatch washed with enzyme)}}$ - $L_{\text{(swatch washed without enzyme)}}$) are also indicated.

The results show that the hexosaminidases display deep-cleaning properties in model detergent A.

Table 5. Deep-cleaning effects of hexosaminidase in model detergent A

| Enzyme | Enzyme concentration (ppm) | L values | ΔL (Lwith enzyme - Lwithout enzyme) |
|---|---|---|---|
| No enzyme | 0 | 102.9 | |
| SEQ ID NO 20 | 0.2 | 108.8 | 5.9 |

**Example 6 Deep-cleaning effects of hexosaminidases in liquid model detergent on biofilm swatches**

*Staphylococcus aureus* biofilms were grown on textile swatches (wfk30A) as previously described (example 5). Swatches incubated with sterile medium were included as controls. For examining the deep-cleaning properties of the hexosaminidases, five rinsed swatches (sterile or with *S.aureus)* were placed in 50 mL conical centrifuge tubes and 10 mL of wash liquor (15$^0$dH water with 0.2 g/L iron(III) oxide nanopowder (544884; Sigma-Aldrich) with 3.33 g/L liquid model A detergent) and 2ppm enzyme was added to each tube. Washes without enzyme were included as controls. The test tubes were placed in a Stuart rotator and incubated for 1 hour at 37°C at 20rpm. The wash liquor was then removed, and the swatches were rinsed twice with 15$^0$dH water and dried on filter paper over night. The color difference (L) values were measured using a Handheld Minolta CR-300, and are displayed in table 6. Delta values ($L_{(swatch\ washed\ with}$ enzyme) - $L_{(swatch\ washed}$ without enzyme)) are also indicated.

The results show that these hexosaminidases also display deep-cleaning properties in model A liquid detergent.

Table 6. Deep-cleaning effects of hexosaminidase in model A detergent

| Swatch | Enzyme | Enzyme concentration (ppm) | L values | $\Delta L$ ($L_{with\ enzyme}$-$L_{without\ enzyme}$) |
|---|---|---|---|---|
| Clean Textile | No enzyme | 0 | 91.9 | |
| *S.aureus* biofilm swatch | No enzyme | 0 | 83.3 | |
| *S.aureus* biofilm swatch | SEQ ID NO 33 | 2 | 88.1 | 4.8 |
| *S.aureus* biofilm swatch | SEQ ID NO 35 | 2 | 89.6 | 6.3 |
| *S.aureus* biofilm swatch | SEQ ID NO 34 | 2 | 89.0 | 5.7 |
| *S.aureus* biofilm swatch | SEQ ID NO 36* | 2 | 86.9 | 3.6 |
| *(not according to the present invention) | | | | |

Combined with the previous example, the results show that all the polypeptides of the invention have deep-cleaning properties i.e. disrupt and/or remove the biofilm components of the biofilm tested when compared to samples comprising no enzyme.

**Example 7 Deep-cleaning properties of the hexosaminidase on swatches soiled with EPS from *S.aureus***

A crude extract of biofilm extracellular polymeric substances (EPS) were prepared from *S.aureus* (kind gift from Iñigo Lasa (Valle, J., A. Toledo-Arana, C. Berasain, J. M. Ghigo, B. Amorena, J. R. Penades, and I. Lasa. 2003, Mol. Microbiol. 48:1075-1087) as follows: The strain was restreaked on Tryptone Soya Agar (TSA) (pH 7.3) (CM0131; Oxoid Ltd, Basingstoke, UK) and incubated for 1 day at 37°C. 500 mL of TSB + 2% glucose (24563; Roquette Freres) was then inoculated, aliquoted into 50ml conical centrifuge tubes (339652; Thermo Scientific Nunc) (33 ml in each), and incubated for 48 hours at 37°C with shaking (200 rpm). The cells were subsequently pelleted by centrifugation (10 min, 6000g, 25°C), pooled and resuspended in a total of 4 ml 3M NaCl. The suspension was vortexed vigorously and incubated for 15min at ambient temperature to extract the surface-associated EPS. The cells were then re-pelleted (10 min, 5000g, 25°C) and the EPS-containing supernatant was retrieved and diluted to 10ml with sterile MilliQ water. The supernatant was sterile filtered twice (0.45 $\mu$m followed by 0.2 $\mu$m), tested for sterility and stored at -20°C until further use. For wash performance testing, 50ul aliquots of the EPS extract were spotted on sterile textile swatches (WFK20A, cotton/polyester blend) and incubated for 15 min at ambient temperature. The swatches (sterile or with EPS) were placed in 50 mL test tubes and 10 mL of wash liquor (15$^0$dH water with 0.2 g/L iron(III) oxide nano-powder (544884; Sigma-Aldrich) with 3.33g/L liquid model A detergent) and 0.2 $\mu$g/ml or 2 $\mu$g/ml enzyme was added to each tube. Washes without enzyme were included as controls. The test tubes were placed in a Stuart rotator and incubated for 1 hour at 37°C at 20 rpm. The wash liquor was then removed, and the swatches were rinsed twice with 15$^0$dH water and dried on filter paper over night. The tristimulus light intensity (Y) values were measured using a DigiEYE colour measurement and imaging system (VeriVide) equipped with a Nikon D90 digital camera, and are displayed in table 7.

Delta values ($Y_{(swatches\ washed\ with}$ enzyme) - $Y_{(swatches\ washed}$ without enzyme)) are also indicated. The data

clearly shows that the hexosaminidases show direct deep-cleaning properties against the EPS of *S.aureus.*

Table 7. Deep-cleaning effects of hexosaminidase on swatches soiled with EPS from *S.aureus*

| Origin of EPS | Enzyme | Enzyme concentration (ppm) | Average Y values | ΔY |
|---|---|---|---|---|
| Clean textile, no EPS | | 0 | 85.1 | |
| *S. aureus* EPS | | 0 | 61.0 | |
| *S. aureus* EPS | SEQ ID NO 35 | 0.2 | 71.7 | 10.7 |
| *S. aureus* EPS | SEQ ID NO 35 | 2 | 81.1 | 20.1 |

**Example 8 Deep-cleaning effects of hexosaminidase in liquid model detergent on EPS from other microorganisms**

Crude extracts of biofilm extracellular polymeric substances (EPS) were prepared from *Pseudomonas fluorescens* (Isolate from Iceland) and *Acinetobacteriwoffi* (textile isolate from Denmark). *P.fluorescens* was restreaked on Tryptone Soya Agar (TSA) (pH 7.3) (CM0131; Oxoid Ltd, Basingstoke, UK) and incubated for 1 day at 20°C. The strain was inoculated into 10 mL of TSB and the culture was incubated statically for 16 hours at 20°C. After propagation, the culture was diluted (1:100) in 400ml M63 supplemented medium (15mM $(NH_4)_2SO_4$, 100mM $KH_2PO_4$, 1.8μM, $FeSO_4$, 1mM $MgSO_4.7H_2O$, 0.4% (w/v) glycerol, 0.2% (w/v) Casamino acids and 0.0001% (w/v) Thiamine), added to Corning® CellBIND® 225cm$^2$ Angled Neck Cell Culture Flasks with Vent Cap (Product #3293) and incubated statically for 3 days at 20°C. The biofilm culture was subsequently pelleted by centrifugation (10 min, 8000g, 25°C), and the cells resuspended in 4ml 3M NaCl and incubated for 30 min at 30°C to extract the surface-associated EPS. The EPS-containing supernatant obtained after centrifugation (10 min, 5000 g, 25°C) was then sterile filtered and stored at -20°C until further.

The crude EPS extract from *A.iwoffi* was prepared as follows: 10ml LB broth (L3152, Fluka) was inoculated and the culture was incubated for 2 day at 30°C. After propagation, the culture was diluted (1:100) in 300ml fresh LB in a Corning® CellBIND® 225cm$^2$ Angled Neck Cell Culture Flasks with Vent Cap (Product #3293) and incubated statically at 30°C. After 3 days, the culture was aliquoted into conical centrifuge tubes and the cells were pelleted by centrifugation (10 min, 6000g, 25°C). The pellets were then resuspended in a total of 1.5ml 3M NaCl and incubated for 15 min at room temperature. The EPS-containing supernatant obtained after centrifugation (10 min, 6000g, 25°C) was sterile filtered and stored at -20°C until use.

For wash performance testing, 50ul aliquots of the different crude EPS extracts were spotted on sterile textile swatches (WFK20A) and incubated for 15 min at ambient temperature. The swatches (sterile or with EPS) were placed in 50 mL test tubes and 10 mL of wash liquor (15$^0$dH water with 0.2 g/L iron(III) oxide nano-powder (544884; Sigma-Aldrich) with 3.33g/L liquid model A detergent) and 0.2 μg/ml or 2 μg/ml enzyme was added to each tube. Washes without enzyme were included as controls. The test tubes were placed in a Stuart rotator and incubated for 1 hour at 37°C at 20rpm. The wash liquor was then removed, and the swatches were rinsed twice with 15$^0$dH water and dried on filter paper over night.

The tristimulus light intensity (Y) values were measured using a DigiEYE colour measurement and imaging system (VeriVide) equipped with a Nikon D90 digital camera, and are displayed in table 8.

Delta values ($Y_{(\text{swatches washed with}}$ enzyme) - $Y_{(\text{swatches washed}}$ without enzyme)) are also indicated.

Table 8. Deep-cleaning effects of hexosaminidase in liquid model detergent on biofilm EPS from *P.fluorescens* and *A.iwoffi*

| Origin of EPS | Enzyme | Enzyme concentration (ppm) | Average Y values | ΔY |
|---|---|---|---|---|
| *P. fluorescens* | | 0 | 57.6 | |
| *P. fluorescens* | SEQ ID NO 35 | 0.2 | 72.6 | 14.9 |
| *P. fluorescens* | SEQ ID NO 35 | 2 | 78.7 | 21.1 |
| *A. iwoffii* | | 0 | 52.5 | |
| *A. iwoffii* | SEQ ID NO 35 | 0.2 | 60.0 | 7.5 |

(continued)

| Origin of EPS | Enzyme | Enzyme concentration (ppm) | Average Y values | ΔY |
|---|---|---|---|---|
| *A. iwoffii* | SEQ ID NO 35 | 2 | 58.0 | 5.5 |

The data clearly shows that the hexosaminidases also show deep-cleaning properties against EPS from gram-negative bacteria.

**Example 9 Deep-cleaning effects of hexosaminidase in different detergents**

A crude extract of biofilm extracellular polymeric substances (EPS) were prepared from *S.aureus* (kind gift from Iñigo Lasa (Valle, J., A. Toledo-Arana, C. Berasain, J. M. Ghigo, B. Amorena, J. R. Penades, and I. Lasa. 2003, Mol. Microbiol. 48:1075-1087) as previously described in example 8.

Two circular swatches of 1 cm in diameter (20A wfk) were placed in each well of a 24 wells plate and spotted with 50 μl EPS from S. *aureus* for 10 min at RT. Swatches without EPS were included as controls. To each well was added one mechanical stress object (metal rod with plastic cover connected by rubbering); 975 μl of detergent solution (detergent and final concentration: Liquid model A detergent 3.33 g/L, Liquid model N detergent 2.0 g/L); hexosaminidase in a final concentration of 5 mg/L; and 1000 μl soiled detergent with final concentration of 15 g/L rose clay facial mask purchased from www.saebevaerkstedet.dk. Swatches without PNAG and wells without enzyme was included as controls. The plate was shaken for 30 min at RT with repeating intervals of 30 sec at 1000 and 1200 rpm, respectively. All solutions were diluted in 15 °dH water hardness. After washing the swatches were rinsed twice in water and dried on filter paper for a minimum of 2 hours or overnight. Dried swatches were fixed on a sheet of white paper for scanning and the color intensity (L) values were measured using the software Color Analyzer.

The tables 9 and 10 shows wash data for hexosaminidases in 2 different detergents

Table 9 shows results in Model detergent A

| Enzyme | Enzyme concentration (ppm) | L values | ΔL (Lwith enzyme - Lwithout enzyme) |
|---|---|---|---|
| No Enzyme | 0 | 348 | 0 |
| SEQ ID NO 20 | 5 | 382 | 34 |
| SEQ ID NO 33 | 5 | 383 | 34 |
| SEQ ID NO 35 | 5 | 369 | 20 |
| SEQ ID NO 34 | 5 | 365 | 17 |
| SEQ ID NO 36* | 5 | 361 | 13 |

Table 10 Shows results in Model detergent N

| Enzyme | Enzyme concentration (ppm) | L values | ΔL (Lwith enzyme - Lwithout enzyme) |
|---|---|---|---|
| No Enzyme | 0 | 345 | 0 |
| SEQ ID NO 20 | 5 | 360 | 16 |
| SEQ ID NO 33 | 5 | 349 | 4 |
| SEQ ID NO 35 | 5 | 364 | 20 |
| SEQ ID NO 34 | 5 | 355 | 10 |
| SEQ ID NO 36* | 5 | 356 | 11 |
| *(not according to the present invention) | | | |

**Example 10: Construction of clades and phylogenetic trees**

The Glyco_hydro_20 domain includes the polypeptides of the invention having PNAG activity and comprises

the DSP domain as well as the clusters such as the clades.

A phylogenetic tree was constructed, of polypeptide sequences containing a Glyco_hydro_20 domain, as defined in PFAM (PF00728, Pfam version 31.0 Finn (2016). Nucleic Acids Research, Database Issue 44:D279-D285). The phylogenetic tree was constructed from a multiple alignment of mature polypeptide sequences containing at least one Glyco_hydro_20 domain. The sequences were aligned using the MUSCLE algorithm version 3.8.31 (Edgar, 2004. Nucleic Acids Research 32(5): 1792-1797), and the trees were constructed using FastTree version 2.1.8 (Price et al., 2010, PloS one 5(3)) and visualized using iTOL (Letunic & Bork, 2007. Bioinformatics 23(1): 127-128).

The polypeptides containing a Glyco_hydro_20 domain comprises several motifs one example is GXDE (SEQ ID NO 41), situated in positions 277 to 278 in *Curtobacterium luteum* (SEQ ID NO 34). Residues D and E are the key catalytic residues of Glyco_hydro_20 enzymes (position 277 to 278 in SEQ ID NO 34).

As already described the polypeptides of the invention having PNAG activity may comprise the structural domains of Glyco_hydro_20.

The polypeptides may be separated into multiple distinct sub-clusters, or clades, where we denoted the clades listed below. The distinct motifs for each clade are described in details below.

Generation of IES domain

A domain, preferably shared by the polypeptides of the invention, was identified. This domain has not been described previously. The domain is termed IES and polypeptides of this domain comprises Glyco_hydro_20 domain polypeptides of bacterial origin and are in addition to having PNAG activity, characterized by comprising certain motifs. The polypeptides of the domain comprise the motif example [EQ][NRSHA][YVFL][AGSTC][IVLF][EAQYN][SN] (SEQ ID NO 46), corresponding to QNVGIES at positions 156 to 162 of SEQ ID NO 34.

Generation of ARAY clade

The ARAY clade comprises Glyco_hydro_20 domain polypeptides of bacterial origin, having PNAG activity. The polypeptides of the clade comprise the motif example ARAYYPV (SEQ ID NO 42), corresponding to pos 124 to 130 of SEQ ID NO 9, where R at position 125 in SEQ ID NO 34 is part of the active site.

Another motif which may be comprised by the polypeptides of the ARAY clade is AWNDGID (SEQ ID NO 43), 306 to 312 in SEQ ID NO 34, where R (position 307 in SEQ ID NO 34) is part of the active site. A further motif which may be comprised by the polypeptides of the ARAY clade is DDQNVGI (SEQ ID NO 44), 154 to 160 in SEQ ID NO 34. An additional motif which may be comprised by the polypeptides of the ARAY clade is DPRIH (SEQ ID NO 45), 320 to 324 in SEQ ID NO 34.

An alignment of the polypeptides of the invention comprised in the clade is shown in Figure 1.

A phylogenetic tree of the ARAY clade is shown in Figure 2.

**Example 11 Hmm model**

**:** The GH20 Catalytic Domain HMM

HMMER3/f [3.1b2 | February 2015]
NAME GH20
LENG 349
ALPH amino
RF no
MM no
CONS yes
CS no
MAP yes
DATE Tue Apr 19 15:50:13 2016
NSEQ 7
EFFN 0.721191
CKSUM 975545072
STATS LOCAL MSV -11.4545 0.70012
STATS LOCAL VITERBI -11.9986 0.70012
STATS LOCAL FORWARD -5.4957 0.70012

HMM A C D E F G H I K L M N P

(continued)

Q R S T V W Y
m->m m->i m->d i->m i->i d->m d->d

COMPO 2.60174 4.67169 2.79327 2.61588 3.29203 2.91852 3.66283 2.75216 2.66516 2.45487 3.74224 2.89789 3.53715 3.04900 3.06221 2.65049 2.86470 2.72758 4.38456 3.25755

2.68614 4.42170 2.77513 2.73135 3.46347 2.40530 3.72523 3.29326 2.67755 2.69351 4.24655 2.90337 2.73753 3.18095 2.89809 2.37893 2.77513 2.98527 4.58506 3.61494
0.45793 1.55220 1.86030 1.93584 0.15584 0.00000 *

1 2.62542 4.83427 2.82121 2.16711 4.08650 3.36627 3.59200 3.50267 2.32909 3.09104 3.28010 2.61805 3.79033 2.27737 2.74111 2.64445 2.85824 3.17885 5.31504 3.98423 18e---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

2 2.59520 4.80934 2.56843 2.35720 4.04573 3.35360 3.12363 3.48055 2.19222 2.71416 3.89420 2.88005 3.77532 2.74381 2.81030 2.39844 2.83053 3.15335 5.29560 3.95248 19 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

3 2.44337 4.24133 3.44826 2.94557 3.60875 3.32170 3.90746 2.74241 2.86666 2.33269 3.55471 3.28416 3.84623 3.20748 3.20284 2.11243 2.42884 2.23327 5.07294 3.82985 20 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.40460 1.10034

4 2.32383 4.74064 2.88292 2.47855 4.20972 3.29447 3.71653 3.63312 2.49182 3.22640 4.04628 2.96533 3.15174 1.88659 2.91020 2.35619 2.87019 3.25803 5.44415 4.11846 21 q ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

5 2.60305 4.92460 2.34604 2.33546 4.20764 3.36563 3.59784 3.20833 2.23451 3.20814 3.99952 2.60210 3.26130 2.72857 2.85746 2.60147 2.59997 3.28261 5.41136 4.04410 22 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

6 2.96397 5.16780 3.08807 2.61611 4.55299 3.55911 3.63937 3.95530 1.33438 3.42043 4.28125 3.07821 3.97783 2.08646 2.29309 2.94591 3.16238 3.61961 5.49982 4.25880 23 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

7 2.87810 5.26730 2.45384 1.39315 4.56042 3.33537 3.71463 4.03551 2.48787 3.54742 4.39801 2.82640 3.87420 2.18876 2.91430 2.81748 3.13497 3.66128 5.70116 4.30753 24 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

8 2.31131 4.64341 3.00787 2.53623 4.11949 2.84873 3.72249 3.52885 2.04434 3.14224 3.96200 3.00481 3.78934 2.88956 2.92616 2.36328 2.26473 3.16299 5.37868 4.06985 25 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

9 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 26 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

10 3.27661 4.60563 4.81638 4.28979 3.17506 4.53343 4.88108 1.60763 4.11630 1.03186 2.99695 4.54933 4.75580 4.30643 4.26407 3.90698 3.51388 2.04894 5.23833 4.09917 27 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

11 2.23703 4.27706 3.59749 3.09133 3.59810 3.42501 4.01297 2.70077 3.00197 2.52770 2.56062 3.41118 3.94133 3.33484 3.32008 2.76392 1.85004 2.48383 5.10447 3.88249 28 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

12 3.27661 4.60563 4.81638 4.28979 3.17506 4.53343 4.88108 1.60763 4.11630 1.03186 2.99695 4.54933 4.75580 4.30643 4.26407 3.90698 3.51388 2.04894 5.23833 4.09917 29 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

13 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 30 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

14 2.89299 4.37089 4.29165 3.84509 3.55549 3.98375 4.62445 1.14199 3.69578 2.18331 3.42439 4.07889 4.43466 4.03173 3.92165 3.38134 2.66753 1.83054 5.36349 4.14337 31 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

15 1.21141 4.16247 3.48581 3.20999 4.27083 2.95735 4.25932 3.61453 3.25267 3.36905 4.22177 3.34067 3.71129 3.54606 3.54832 1.72877 2.72467 3.10484 5.63933 4.42397 32 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

16 3.18427 5.00812 3.69114 3.17333 4.40169 3.63992 3.94487 3.95597 2.31269 3.44427 4.44251 3.52033 4.13693 3.15941 0.76856 3.26233 3.44101 3.67383 5.42399 4.29809 33 r---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

17 2.91802 5.11009 3.26657 2.65260 4.45768 3.59624 2.28209 3.87264 1.65036 3.34797 4.18853 3.10273 3.96804 2.73579 1.89425 2.91169 3.10260 3.54063 5.42158 4.18113 34 k -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

18 3.60906 4.85790 4.61301 4.29200 1.07612 4.37531 3.49533 3.27851 4.13915 2.61105 3.87947 4.07109 4.68672 4.11993 4.17923 3.74459 3.83280 3.21007 3.64011 1.42609 35 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

19 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 36 y -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

20 2.32198 4.30679 3.25189 2.89710 4.22510 3.05357 4.03256 3.63023 2.93492 3.29831 4.12849 3.19386 2.11014 3.26010 3.30004 1.65444 2.37000 3.15702 5.54568 4.29162 37 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

21 2.66597 4.22511 3.91716 3.37375 3.43772 3.72193 4.15633 1.85319 3.28493 2.33346 3.36759 3.67002 3.26998

3.57237 3.55940 3.03288 2.59141 1.64956 5.01744 3.80727 38 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

22 2.82044 5.34850 2.13403 1.52422 4.64944 3.28466 3.68463 4.14292 2.24269 3.63308 4.43539 2.42022 3.82375 2.82399 3.08185 2.73861 3.07900 3.72182 5.78514 4.33259 39 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

23 2.56043 4.26125 3.87113 3.43769 3.72631 3.45624 4.31955 2.39581 3.33689 2.55074 3.63922 3.64954 4.05083 3.66947 3.61016 2.86041 1.73397 1.58618 5.33176 4.10720 40 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

24 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 41 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

25 3.11697 5.08145 3.30276 2.89877 4.50288 3.58722 3.85206 3.94235 0.84697 3.47537 4.43095 3.31562 4.07873 3.03659 2.47177 3.15035 3.36006 3.64238 5.49660 4.33243 42 k -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

26 2.13272 4.90400 2.43559 2.34272 4.27641 3.30690 3.67133 3.71686 2.49641 3.28917 4.09511 2.87194 3.79353 2.33192 2.97421 2.15688 2.89582 3.34042 5.49710 4.12889 43 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

27 3.16649 4.52728 4.63396 4.13742 1.55523 4.29617 4.32301 1.73842 4.00286 1.76408 3.11947 4.27581 4.58882 4.13968 4.13035 3.65327 3.40793 2.23962 4.62924 3.16052 44 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

28 3.11618 4.44875 4.77519 4.29382 3.52475 4.41912 5.00622 1.05857 4.16245 2.01027 3.34073 4.51329 4.74777 4.43442 4.35709 3.81802 3.38913 1.49499 5.51169 4.30526 45 i -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

29 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 46 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

30 2.67413 4.78076 2.75214 1.93151 4.20765 3.30969 3.79991 3.51271 2.62236 3.21069 4.09287 2.98239 3.85595 2.98671 3.03254 2.72726 1.69281 3.18792 5.49375 4.17123 47 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

31 3.15114 4.53079 4.54737 4.17094 3.48096 4.22724 4.87288 0.90672 4.00200 2.03850 3.39535 4.39520 4.64605 4.33611 4.18143 3.73942 3.44853 1.86187 5.40162 4.15144 48 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

32 2.27518 4.59840 2.98273 2.59620 4.01461 3.27359 2.35884 3.51023 2.60535 3.13774 3.98239 3.04442 3.81201 2.98424 3.00023 1.88039 2.86191 3.15155 5.33302 3.99996 49 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

33 2.20797 4.82901 2.95598 2.43130 4.08188 3.41762 3.17477 3.49920 2.19484 2.78090 3.90630 2.64872 3.81741 2.54863 2.77108 2.65181 2.85410 3.17381 5.31346 3.98688 50 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

34 1.72399 4.44652 3.01228 2.70067 4.25777 3.10875 3.93504 3.68015 2.82445 3.32584 4.14997 2.16806 3.75134 3.13888 3.22977 1.96545 2.80720 3.22507 5.55414 4.26228 51 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

35 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 52 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

36 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 53 G -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

37 2.70697 5.04010 1.97763 2.27126 4.43079 3.26888 3.71680 3.88291 2.60077 3.45129 4.26710 2.22267 3.80974 2.87048 3.10900 2.68588 2.10398 3.48789 5.64569 4.24126 54 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

38 3.60906 4.85790 4.61301 4.29200 1.07612 4.37531 3.49533 3.27851 4.13915 2.61105 3.87947 4.07109 4.68672 4.11993 4.17923 3.74459 3.83280 3.21007 3.64011 1.42609 55 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

39 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 56 l -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

40 2.87113 5.02265 2.89430 2.54183 3.92175 3.47916 1.83985 3.76451 2.31828 3.27943 4.16746 3.02970 3.94298 2.13713 2.63327 2.87433 3.10615 3.44925 5.22235 3.79305 57 h - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

41 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 58 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

42 3.17489 4.96114 3.28685 3.06447 3.40116 3.58476 0.85747 3.90143 2.90380 3.37516 4.43190 3.46668 4.14687 3.43336 3.15915 3.24489 3.48443 3.63377 4.86369 3.34821 59 h ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

43 3.45825 4.81603 4.44812 4.19374 0.79153 4.10787 3.95340 2.96558 4.09750 2.30148 3.67455 4.19402 4.56467 4.22394 4.17942 3.71886 3.76029 2.96711 4.16127 2.51081 60 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

44 2.39611 4.31724 3.34558 3.17089 4.23153 3.04461 4.27530 3.77599 3.25503 3.48527 4.40012 3.36943 3.79653 3.59279 3.53464 0.88216 2.88812 3.27739 5.59224 4.31654 61 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

45 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 62 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

46 2.82433 4.98743 2.72626 2.47424 3.84772 3.39017 1.79546 3.80387 2.51314 3.34581 4.22739 2.14774 3.91198 2.95173 2.88393 2.82843 3.09659 3.46249 5.21617 3.73245 63 h - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

47 3.09089 5.22592 2.56202 0.82634 4.59241 3.37774 3.98099 4.07700 2.88130 3.67069 4.64056 3.02951 3.99035 3.19510 3.30408 3.05927 3.39713 3.74605 5.73522 4.46059 64 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

48 2.66333 4.85194 2.82911 2.47306 4.29809 3.32358 3.71334 3.75949 2.41795 3.32573 4.15172 1.76097 3.83522 2.87346 2.41981 2.34515 2.95006 3.37838 5.48902 4.15662 65 n ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

49 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 66 y -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

50 0.81435 4.27445 3.62315 3.42433 4.20975 3.09435 4.42756 3.35852 3.44466 3.23284 4.24993 3.53357 3.84826

3.77227 3.68299 2.62446 2.91253 2.98636 5.62159 4.43962 67 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

51 3.25587 4.57778 4.82700 4.30157 3.21221 4.53220 4.89700 1.44546 4.13392 1.15442 3.03350 4.55420 4.75994 4.32908 4.28372 3.90604 3.49491 1.97962 5.26452 4.11932 68 l -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

52 2.03769 4.92952 2.57987 1.46106 4.37380 3.28153 3.79326 3.70661 2.65006 3.37321 4.24554 2.90322 3.84882 2.96755 3.10005 2.73885 3.03061 3.35463 5.62496 4.26393 69 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

53 2.39611 4.31724 3.34558 3.17089 4.23153 3.04461 4.27530 3.77599 3.25503 3.48527 4.40012 3.36943 3.79653 3.59279 3.53464 0.88216 2.88812 3.27739 5.59224 4.31654 70 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

54 2.65102 4.90823 2.76780 1.98616 4.19563 3.36273 2.75116 3.63175 2.42514 3.20954 4.02330 2.89383 3.81175 2.79138 2.87317 2.43615 2.37119 3.28393 5.41942 4.06591 71 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

55 2.80253 4.22179 4.31017 3.74307 2.20561 3.92303 4.06161 2.16322 3.61262 1.81545 3.11713 3.90030 4.25514 3.78488 3.75700 3.22501 3.03186 2.06234 4.53432 2.46480 72 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

56 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 73 l -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

57 2.82752 5.31584 2.08949 1.97162 4.63397 2.31340 3.71862 4.12629 2.64864 3.63806 4.45421 1.91931 3.82675 2.86930 3.19982 2.75097 3.10082 3.71075 5.80956 4.35188 74 n ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

58 3.01009 4.99534 3.01191 2.77152 4.18365 3.47191 3.93031 3.84023 2.58229 3.32714 4.35311 3.23169 4.02910 0.98524 2.86243 3.05251 3.30807 3.56112 5.42967 4.13451 75 q - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

59 2.76322 4.88423 3.14125 2.60350 4.14363 3.49723 3.64782 3.58659 2.20103 2.76990 4.01628 2.32855 3.90954 2.80978 1.76364 2.79935 2.98927 3.27553 5.32057 4.02555 76 r - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

60 1.88301 4.70754 2.77889 2.04292 4.23171 2.70975 3.76605 3.65306 2.62652 3.26332 4.08493 2.94632 3.78772 2.93801 3.08209 2.29057 2.87475 3.26489 5.49618 4.16190 77 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

61 2.12998 4.95682 2.55707 1.40093 4.39593 3.28197 3.79449 3.73028 2.65304 3.39386 4.26878 2.89522 3.85234 2.96839 3.10479 2.74883 3.04574 3.37772 5.64282 4.27718 78 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

62 2.87132 5.23258 2.02114 2.22822 4.58008 3.23965 3.81527 4.14555 2.79632 3.70120 4.56321 1.32198 3.86081 2.99894 3.32852 2.81489 3.18464 3.73193 5.81429 4.36009 79 n ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

63 1.26091 4.25315 3.38399 3.08468 4.20504 3.04239 4.17235 3.52109 3.12261 3.26340 4.14811 3.30699 2.21225 3.44135 3.44300 2.49769 2.78295 3.07867 5.56731 4.34523 80 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.19054 4.02509 1.86030 0.61958 0.77255 0.48576 0.95510

64 2.63710 4.26471 3.83287 3.32297 3.52352 3.62954 4.20457 1.98169 3.21852 2.29057 3.41590 3.62719 4.10629 3.54798 3.51624 2.97480 2.00441 1.82363 5.16958 3.94507 81 v---

2.68621 4.42228 2.77522 2.73126 3.46357 2.40516 3.72497 3.29357 2.67744 2.69358 4.24693 2.90350 2.73742 3.18125 2.89766 2.37890 2.77522 2.98521 4.58480 3.61506

0.30705 1.36957 4.58850 0.25181 1.50235 0.63100 0.75941

65 2.64016 4.96447 2.26629 2.06144 4.25840 2.81506 3.61637 3.69941 2.46807 3.26998 4.08149 2.48330 3.75866 2.76465 2.96551 2.62345 2.89483 2.89292 5.48085 4.09568 83 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

66 2.32512 4.76066 2.99914 2.60494 4.22816 3.34939 3.69714 3.52706 1.48205 3.17750 4.07526 3.04563 3.85645 2.86989 2.53074 2.75769 2.98044 3.20786 5.39552 4.13466 84 k -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

67 2.69811 4.98517 1.85769 2.24050 4.02912 3.27664 3.63332 3.69170 2.54094 3.26778 4.11119 2.47116 3.78689 2.81989 3.03591 2.67839 2.95708 3.34690 5.35319 3.03969 85 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

68 2.65708 4.43313 3.32956 3.22817 4.37057 0.73893 4.34882 3.96866 3.40858 3.62358 4.59928 3.49387 3.82406 3.73324 3.64531 2.82881 3.13782 3.49820 5.46738 4.47226 86 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

69 2.71555 4.26084 4.01045 3.49314 3.50176 3.78219 4.32824 1.82828 3.38934 2.22742 3.37809 3.78266 4.22210 3.70242 3.66847 3.12550 2.31069 1.54261 5.19718 3.98072 87 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

70 3.23798 4.72953 3.82891 3.55712 2.29361 3.82278 3.61560 3.24807 3.41482 2.70363 3.92376 3.71568 4.29677 3.72237 3.59982 3.37530 3.52463 3.11833 3.93713 0.97636 88 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

71 2.87230 4.31165 4.19157 3.65625 2.39581 3.95642 3.98324 1.69315 3.53508 1.94049 3.15076 3.85549 4.28439 3.73693 3.72476 3.26422 3.10466 2.25034 4.42927 2.55852 89 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

72 2.71820 4.71455 2.70482 2.58966 4.07708 3.17974 3.91591 3.78610 2.81907 3.43368 4.38897 1.16929 3.83129 3.18866 3.17352 2.78563 3.09985 3.40895 5.38372 4.02587 90 n ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

73 2.74311 4.50151 3.34959 3.18771 4.18344 3.18938 4.26569 3.72722 3.24447 3.35475 4.40432 3.49505 0.83946 3.63344 3.49072 2.91040 3.18417 3.37332 5.37173 4.30402 91 p - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

74 2.57998 4.49024 3.18601 2.64336 3.65510 3.45032 3.68065 3.00658 2.10954 2.70983 3.60157 3.09739 3.85237 2.91430 2.85071 2.70990 2.56455 2.49907 5.03209 3.15736 92 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

75 2.40932 4.25438 3.42249 3.17420 3.96330 3.09387 4.19201 3.08966 3.11705 2.95338 4.00431 3.38459 3.79712 3.51219 3.37604 2.61052 1.18267 2.78532 5.40166 4.17993 93 t -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.40460 1.10034

76 2.70229 4.94580 2.62102 2.36264 4.26419 2.44373 2.95596 3.81010 2.54731 3.37338 4.20279 1.83844 3.83055 2.89237 2.99078 2.70546 2.98733 3.43091 5.51220 4.12097 94 n ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

77 2.73064 4.86893 2.99005 2.58925 4.35918 3.38261 3.72140 3.77606 1.45572 3.34226 4.18943 3.04768 3.88536 2.88118 2.59856 2.08548 3.01111 3.40813 5.48642 4.20070 95 k -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

78 1.98628 4.26405 3.34273 3.00228 4.17542 3.05695 4.09779 3.47602 3.01866 3.21897 4.08702 3.25826 1.66206 3.34795 3.35647 2.48647 2.28667 3.04559 5.53327 4.29925 96 p - --

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

79 3.60906 4.85790 4.61301 4.29200 1.07612 4.37531 3.49533 3.27851 4.13915 2.61105 3.87947 4.07109 4.68672

4.11993 4.17923 3.74459 3.83280 3.21007 3.64011 1.42609 97 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

80 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 98 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

81 2.28585 4.22285 3.40606 3.08808 4.19721 3.01742 4.15603 3.54303 3.09540 3.28360 4.14309 3.29427 3.73505 3.42322 3.41193 1.62744 1.55290 3.07900 5.55939 4.32472 99 t ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

82 2.80548 4.54103 3.51754 2.99292 2.48793 3.69083 3.65985 3.04567 2.18102 2.65617 3.63318 3.36147 4.06968 3.21180 3.16262 2.96440 3.03488 2.83807 4.39569 1.87581 100 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

83 2.46104 5.05388 3.00611 2.26593 4.40780 3.48091 3.60489 3.80877 1.58818 3.32066 4.13656 2.98553 3.88379 2.73457 2.24172 2.77136 2.99409 3.45542 5.45676 4.16445 101 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

84 2.86785 5.21525 2.54398 1.83107 4.51410 3.36448 3.70136 3.97862 2.41801 3.49251 4.34395 2.86427 3.88267 1.65279 2.81169 2.82002 3.11732 3.61462 5.64287 4.27431 102 q - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

85 3.30073 4.65702 4.74385 4.22920 3.12452 4.49680 4.82353 1.90464 4.02827 0.88495 2.96754 4.50096 4.73304 4.24343 4.18267 3.87892 3.54126 2.17263 5.19002 4.02959 103 l---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

86 2.65877 4.99581 2.47013 2.14619 4.26940 3.35960 3.61847 3.71008 2.20644 2.53460 4.06683 2.40169 3.79985 2.75424 2.87825 2.64487 2.89639 3.34825 5.46578 4.09240 104 e---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

87 2.83807 5.33618 1.69859 1.78951 4.65491 3.25658 3.72510 4.14887 2.66491 3.66171 4.48056 2.73333 3.82881 2.87694 3.22207 2.07820 3.11390 3.73051 5.83102 4.36614 105 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

88 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 106 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

89 2.67869 4.42348 3.47794 2.94624 3.62577 3.60203 3.89183 1.84663 2.22721 2.56504 3.54443 3.34835 4.01262 3.17151 3.02552 2.89929 2.56473 2.44088 5.08673 3.85005 107 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

90 1.82839 4.62244 2.58343 2.54313 3.77433 3.38531 3.72959 3.25100 2.63974 2.92527 3.79709 3.02891 3.85128 2.95691 3.07254 2.69815 2.87475 2.96579 5.15933 2.82327 108 a---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

91 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 109 y---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

92 1.21141 4.16247 3.48581 3.20999 4.27083 2.95735 4.25932 3.61453 3.25267 3.36905 4.22177 3.34067 3.71129 3.54606 3.54832 1.72877 2.72467 3.10484 5.63933 4.42397 110 a---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

93 2.86665 5.10493 2.84216 2.49547 4.48992 3.44045 3.67909 3.93633 1.41713 3.44879 4.29421 2.18473 3.91870 2.82764 2.52526 2.85172 3.10667 3.57413 5.54267 4.24105 111 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

94 2.43678 5.18726 1.97399 2.03436 4.50995 3.28744 3.67198 3.98616 2.54413 3.50748 4.30465 2.43356 3.80476 2.81062 3.07138 2.19673 3.00070 3.57938 5.68251 4.25373 112 d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

95 3.08499 5.19289 3.51099 2.82787 4.65937 3.69242 3.62259 4.00128 1.31465 3.43365 4.30648 3.22313 4.05699 2.75656 1.52521 3.07826 3.24642 3.68364 5.46575 4.30730 113k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

96 2.80542 5.19687 2.02773 2.20857 4.61825 2.67418 3.77417 4.12310 2.73202 3.65759 4.48827 1.55432 3.83044 2.93961 3.27520 2.75334 3.11040 3.69515 5.82269 4.37736 114 n---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

97 3.11613 4.44968 4.77262 4.29194 3.52300 4.41684 5.00429 1.05559 4.15968 2.00856 3.33974 4.51143 4.74646 4.43229 4.35428 3.81617 3.38941 1.50231 5.51006 4.30319 115 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

98 2.72273 4.76136 2.94932 1.67024 3.90234 3.46579 3.76456 3.18245 2.56433 2.81297 2.60535 3.06133 3.91515 2.95853 2.96031 2.79620 2.97154 2.95873 5.29546 3.98620 116 e---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

99 3.16163 4.49254 4.76914 4.22636 3.29555 4.45874 4.86010 1.76060 4.10085 1.23526 3.10116 4.47633 4.70773 4.29372 4.27087 3.81174 3.40261 1.51980 5.29741 4.16378 117 1---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

100 3.11130 4.42474 4.82205 4.32945 3.57088 4.45888 5.04233 1.20522 4.21559 2.06591 3.37517 4.54602 4.77150 4.47909 4.41151 3.85036 3.37865 1.25185 5.54730 4.34363 118 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

101 2.96585 4.69526 3.58992 3.45271 4.43091 3.37425 4.51572 4.01796 3.52354 3.62729 4.68520 3.74295 0.58713

3.90765 3.74983 3.13863 3.41988 3.64553 5.57332 4.55440 119 P---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

102 2.96406 5.52572 1.67512 1.28150 4.81416 3.24219 3.78335 4.33080 2.79447 3.83502 4.68921 2.70807 3.86182 2.94974 3.38467 2.84856 3.25200 3.90619 5.98418 4.48605 120 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

103 3.13982 4.44529 4.83263 4.29726 3.40582 4.49343 4.93951 1.48570 4.18488 1.54415 3.20710 4.52934 4.74725 4.39010 4.35704 3.85324 3.38440 1.35670 5.38804 4.23484 121 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

104 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 122 d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

105 2.47570 4.32120 3.41534 2.95632 3.73107 3.30775 3.95200 2.98811 2.88215 2.10158 3.69589 3.29288 3.86540 3.23896 3.21708 1.75645 2.49035 2.71766 5.17536 3.91851 123 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

106 2.96585 4.69526 3.58992 3.45271 4.43091 3.37425 4.51572 4.01796 3.52354 3.62729 4.68520 3.74295 0.58713 3.90765 3.74983 3.13863 3.41988 3.64553 5.57332 4.55440 124 P---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

107 1.85721 4.55854 2.85901 2.64278 4.28840 3.14642 3.94666 3.69016 2.83077 3.38284 4.24500 1.57103 3.79755 3.15922 3.22584 2.60808 2.90624 3.26550 5.59505 4.27125 125 n---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

108 3.17489 4.96114 3.28685 3.06447 3.40116 3.58476 0.85747 3.90143 2.90380 3.37516 4.43190 3.46668 4.14687 3.43336 3.15915 3.24489 3.48443 3.63377 4.86369 3.34821 126 h---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

109 2.54708 4.30603 3.56761 3.11842 3.54148 3.40565 4.01831 2.82517 2.99881 2.51286 1.82933 3.42092 3.94676 3.36376 3.29058 2.09180 2.88069 2.61684 5.06929 3.80793 127 m - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

110 2.71423 4.78247 3.16282 2.63537 4.14187 3.46292 3.68040 3.50746 2.03512 3.11264 3.96760 3.08828 3.89674 2.84833 2.27234 2.77509 1.81528 3.19654 5.32878 4.06891 128 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

111 1.37678 4.16990 3.47143 3.15263 4.25712 1.99759 4.20963 3.60472 3.19472 3.33701 4.17373 3.31367 3.70623 3.48136 3.51256 2.41500 2.27599 3.09969 5.61177 4.40326 129 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

112 2.91787 4.35079 4.31360 3.77834 2.88033 3.98633 4.10805 1.47498 3.56553 2.00482 3.21189 3.95307 4.33627 3.82542 3.72767 3.32584 3.15861 2.29746 2.60881 3.14763 130 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

113 3.32075 4.67106 4.65248 4.18787 1.42808 4.36915 4.17707 2.44930 4.03693 1.35663 3.11212 4.29060 4.64318 4.14491 4.14864 3.74047 3.55563 2.53074 4.41270 2.86475 131 l---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

114 2.66073 5.07937 2.26031 2.28403 4.39463 3.34789 3.60201 3.85527 2.18826 3.37229 4.15086 2.30124 3.78910 2.72353 2.59107 2.62894 2.61999 3.45846 5.53515 4.14389 132 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

115 3.27661 4.60563 4.81638 4.28979 3.17506 4.53343 4.88108 1.60763 4.11630 1.03186 2.99695 4.54933 4.75580 4.30643 4.26407 3.90698 3.51388 2.04894 5.23833 4.09917 133 l ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

116 3.18836 4.56014 4.67858 4.11504 3.12109 4.40005 4.72037 2.14883 3.97244 1.10732 2.12990 4.38679 4.63146 4.13770 4.13124 3.73504 3.41689 1.97706 5.13923 4.05041 134 l ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

117 2.73946 5.10568 2.75550 1.85563 4.43286 3.39812 3.61445 3.86814 1.90313 3.37982 4.17993 2.88391 3.83738 2.46628 2.67483 2.70967 2.65657 3.49090 5.52943 4.17806 135 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

118 2.26057 4.88016 2.89797 2.03946 4.15763 3.41225 3.63010 3.54479 2.01125 2.81671 3.97038 2.94046 3.83099 2.77760 2.75755 2.67920 2.89372 3.21779 5.37090 4.04307 136 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

119 2.73194 5.17244 2.36766 2.07445 4.48588 3.35243 3.05785 3.95237 1.72160 3.45174 4.23972 2.82417 3.81517 2.74655 2.81056 2.68500 2.96930 3.55265 5.59666 4.20411 137 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

120 2.70692 5.07134 2.20922 2.33678 4.33648 3.38047 2.76322 3.83446 2.32985 3.35441 4.15259 2.87232 3.82189 2.74764 2.08040 2.68121 2.94146 3.45631 5.48854 4.10941 138 r---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

121 2.28383 4.25259 3.23331 3.03121 4.40948 1.54264 4.21451 3.88421 3.19676 3.54852 4.37163 3.25256 3.71581 3.47652 3.52832 1.53475 2.77442 3.29961 5.72416 4.48793 139s---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

122 2.62670 4.71872 3.04803 2.57995 4.12542 2.45687 3.71580 3.48454 1.72319 3.12639 3.97514 3.04494 3.84872 2.88662 2.76011 2.69224 2.90242 2.73030 5.36328 4.07138 140 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

123 2.63671 4.94741 2.34491 2.17813 4.21572 3.36577 3.61482 3.64916 2.23177 2.53478 4.01943 2.86706 3.18257 2.75230 2.87923 2.63249 2.87359 3.29527 5.42737 4.06118 141 e---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

124 3.59200 4.88189 4.49524 4.16753 1.72141 4.33085 3.49196 3.32239 4.02300 2.67191 3.93511 4.01871 4.65923 4.05817 4.10592 3.70493 3.82201 3.24078 3.65279 0.91520 142 y---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

125 2.71495 4.30385 3.95717 3.43967 3.52461 3.73632 4.27384 2.20561 3.32711 2.07872 3.42685 3.73220 4.19239 3.64722 3.60799 3.07686 2.00598 1.60679 5.17470 3.95543 143 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

126 2.74811 5.23021 2.03597 2.22845 4.54525 3.32170 3.63837 4.02450 2.05939 3.51749 4.30407 2.49091 3.81017 2.21990 2.91368 2.68889 2.99363 3.61117 5.66339 4.24548 144 d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.19054 4.02509 1.86030 0.61958 0.77255 0.48576 0.95510

127 2.71622 4.93032 2.91837 2.44757 4.27024 2.56128 3.58138 3.67599 1.97586 3.21555 4.04364 2.94106 3.83300 2.39687 2.25860 2.72275 2.94038 3.33632 5.37783 4.08673 145 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.40460 1.10034

128 3.25623 4.57823 4.82697 4.30148 3.21152 4.53230 4.89679 1.44812 4.13378 1.15209 3.03278 4.55421 4.75989 4.32875 4.28350 3.90611 3.49523 1.98079 5.26405 4.11904 146 l ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

129 2.76965 4.81252 3.27318 2.67726 4.10048 3.54325 3.66039 3.41986 1.63425 3.04914 3.92375 3.12545 3.93359 2.82771 2.22017 2.83233 2.98598 2.37446 5.28619 4.03751 147 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

130 2.27947 4.22349 3.39397 3.08701 4.23309 3.00493 4.16580 3.59064 3.10392 3.32390 4.17920 3.29014 3.72924

3.42991 3.42118 1.32616 1.91464 3.10928 5.58890 4.35270 148 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

131 2.67579 5.08486 2.23936 2.31120 4.41131 3.36717 3.59815 3.86701 1.87336 3.37634 4.15705 2.85162 3.23771 2.71846 2.50489 2.64496 2.91032 3.47145 5.52830 4.15033 149 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

132 2.74928 4.33772 3.77022 3.21659 2.76784 3.72972 3.73047 2.81034 3.08105 2.44844 2.93599 3.50889 4.09464 2.86730 3.35124 3.00321 2.97780 2.62097 3.26940 1.87300 150 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

133 2.50678 4.38589 3.29084 2.76381 3.69713 3.38207 3.80528 2.69232 2.72769 2.74801 3.62662 2.82365 3.85080 3.06040 3.11393 2.01338 2.48850 2.46781 5.09617 3.83707 151 s---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

134 2.49533 5.39227 1.51489 1.70195 4.70218 3.24989 3.74391 4.18282 2.71006 3.70710 4.53879 2.72149 3.83890 2.90065 3.28002 2.78565 3.15621 3.76799 5.88297 4.40554 152 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

135 2.97307 5.54957 1.50577 1.39709 4.83553 3.23796 3.78637 4.35667 2.80824 3.85643 4.71221 2.70112 3.86230 2.95346 3.40678 2.85301 3.26249 3.92831 6.00550 4.49928 153 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

136 2.72718 4.93439 2.61804 1.49335 4.33894 3.30646 3.76751 3.67401 2.59271 3.33255 4.20236 2.90985 3.85192 2.93759 3.02961 2.74013 2.20277 3.33154 5.58398 4.22854 154 e---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

137 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 155 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

138 2.95523 5.42503 1.11559 2.15164 4.74517 3.22157 3.82919 4.32308 2.88202 3.85279 4.72738 2.31205 3.86871 3.01344 3.47406 2.85854 3.27048 3.89231 5.96726 4.46584 156 d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

139 2.92771 4.38160 4.18777 3.68403 2.87871 3.98849 4.07589 1.46781 3.52094 2.10957 3.30013 3.89757 4.34430 3.78857 3.71880 3.32039 3.17069 2.23072 4.56709 2.22863 157 i---

2.68620 4.42227 2.77522 2.73125 3.46356 2.40515 3.72402 3.29356 2.67743 2.69357 4.24692 2.90349 2.73742 3.18148 2.89803 2.37889 2.77522 2.98520 4.58479 3.61505
0.19054 1.80294 4.74744 0.29772 1.35678 0.48576 0.95510

140 2.51237 4.54662 2.93829 2.66324 4.19713 3.18523 3.90862 3.58273 2.75896 3.27756 4.14122 1.93962 3.80600 3.11971 3.14115 2.61616 1.72805 3.18967 5.50879 4.19718 159 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

141 2.85659 4.85399 2.80347 2.70562 4.24217 3.29164 4.05411 3.98803 2.97206 3.61820 4.57439 0.91375 3.95207 3.32914 3.33018 2.91922 3.24472 3.59467 5.53072 4.18110 160 n---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

142 1.74377 4.34722 3.35148 2.91046 3.85123 3.25637 3.95818 3.10284 2.86728 2.50196 3.78844 3.25438 2.27364 3.21773 3.21967 2.62493 2.81345 2.80777 5.27163 4.01558 161 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

143 2.71443 5.02081 2.10019 1.81887 4.29437 3.33197 3.68423 3.65345 2.54608 3.29538 4.12984 2.83915 3.82448 2.83713 3.04401 2.69984 2.96863 2.45579 5.53823 4.15145 162 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

144 1.46953 4.17388 3.45867 3.17721 4.28612 2.95868 4.24142 3.64480 3.22453 3.38528 4.23217 3.32417 3.70914 3.51944 3.52939 1.41567 2.72535 3.12671 5.64613 4.42637 163 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

145 3.17986 4.48584 4.85345 4.31787 3.34930 4.52242 4.95098 1.30809 4.19457 1.45288 3.14773 4.55657 4.76297 4.38907 4.35894 3.88514 3.42256 1.63986 5.36621 4.22596 164 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

146 2.19206 4.59706 3.02321 2.20462 3.80326 3.42131 3.70135 3.18863 2.56375 2.86094 3.72247 3.03836 3.84729 2.91152 2.98299 2.69095 2.57574 2.91188 5.15688 2.78594 165 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

147 2.69121 4.23437 4.01198 3.47838 2.18721 3.73202 4.14203 2.34175 3.38228 2.17261 3.27746 3.72889 4.15431 3.63869 3.62204 3.05574 2.24998 1.86799 4.86438 3.59027 166 v - --

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

148 2.16327 4.28973 4.14987 3.60035 3.34509 3.91632 4.31761 1.67864 3.48868 2.05829 2.44722 3.87788 4.28802 3.75666 3.72777 3.23203 3.05623 2.01968 5.05166 3.86753 167 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

149 2.84215 5.15423 2.83333 2.44047 4.51411 3.45204 3.63120 3.94343 1.57939 3.43137 4.25384 2.23716 3.89609 2.44386 2.50563 2.81167 3.06268 3.57620 5.54106 4.23133 168 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

150 2.54657 4.64700 2.96230 2.55541 4.15005 3.27663 3.75875 3.56240 2.54084 3.18018 4.01361 3.01635 3.80318 2.23570 2.93984 1.92622 2.29936 3.19360 5.41403 4.10554 169 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

151 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 170 l---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

152 3.21898 4.53838 4.84094 4.28138 3.18699 4.51286 4.85609 1.40100 4.14479 1.30535 2.53239 4.52874 4.72118 4.29228 4.28259 3.85938 3.44731 2.01558 5.22354 4.13017 171 l---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

153 2.05284 4.78631 2.04263 2.39189 4.38722 2.40812 3.78019 3.83554 2.67243 3.41777 4.22614 2.89540 3.77689 2.94298 3.16245 2.30432 2.90471 3.40274 5.62409 4.25782 172d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

154 3.09089 5.22592 2.56202 0.82634 4.59241 3.37774 3.98099 4.07700 2.88130 3.67069 4.64056 3.02951 3.99035 3.19510 3.30408 3.05927 3.39713 3.74605 5.73522 4.46059 173 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

155 2.92986 4.43291 4.33452 4.00469 3.66577 3.87938 4.78135 2.05459 3.87481 2.32779 3.60998 4.16937 4.43309 4.22468 4.07226 3.39436 3.28877 0.85226 5.47522 4.21531 174 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

156 2.56402 4.38424 4.45298 3.92171 3.37648 4.18157 4.61136 1.40775 3.78913 1.59308 3.22495 4.17963 4.51375 4.05199 3.99938 3.52396 3.23303 1.86041 5.22598 4.04809 175 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

157 2.76966 5.13236 1.68545 2.02405 4.28443 3.31730 3.68465 3.83835 2.58028 3.39775 4.22770 2.80315 3.83157 2.84843 3.09274 2.72962 3.02446 3.47856 5.54601 2.82533 176 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

158 2.56402 4.38424 4.45298 3.92171 3.37648 4.18157 4.61136 1.40775 3.78913 1.59308 3.22495 4.17963 4.51375 4.05199 3.99938 3.52396 3.23303 1.86041 5.22598 4.04809 177 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

159 3.45825 4.81603 4.44812 4.19374 0.79153 4.10787 3.95340 2.96558 4.09750 2.30148 3.67455 4.19402 4.56467

4.22394 4.17942 3.71886 3.76029 2.96711 4.16127 2.51081 178f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

160 2.69267 4.54144 3.24044 2.91365 3.32451 1.59390 3.86112 3.33411 2.93967 2.97617 3.92164 3.29072 3.95793 3.28437 3.27656 2.83383 3.02119 3.05035 4.83161 2.16869 179 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

161 2.76715 5.21558 1.86580 2.22803 4.45801 3.31594 2.69552 3.98934 2.48954 3.49902 4.30256 2.78913 3.82030 2.18010 2.98255 2.71057 3.01778 3.59171 5.63324 4.20144 180 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

162 2.05529 4.28649 3.31109 2.90912 4.18794 3.05621 4.01997 3.58661 2.93119 3.25570 4.08039 3.20093 2.34739 3.24611 3.30463 1.71515 2.33928 3.12139 5.51242 4.26388 181 s---

2.68593 4.42234 2.77529 2.73132 3.46290 2.40496 3.72503 3.29363 2.67716 2.69364 4.24699 2.90356 2.73749 3.18155 2.89810 2.37896 2.77529 2.98527 4.58486 3.61512
0.19054 1.80294 4.74744 1.14246 0.38424 0.48576 0.95510

163 2.64436 4.74751 2.95172 2.59249 4.28010 3.30937 3.77237 3.70607 1.89804 3.30537 4.15267 3.04417 3.85072 2.94725 2.77513 1.59951 2.95732 3.32804 5.48107 4.18238 186 s - --

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

164 2.86169 5.12724 3.02635 2.29350 4.49470 3.51639 3.61130 3.89662 2.08523 3.37892 4.20738 3.01438 3.92179 1.95758 1.81265 2.84235 3.06492 3.54592 5.48524 4.21303 187 r---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

165 2.92039 4.98831 3.24387 2.72304 4.00298 3.57684 1.80210 3.74629 2.14586 3.25720 4.14395 3.15911 3.99296 2.85717 1.86055 2.94659 3.13341 3.44200 5.21564 3.86174 188 h - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

166 3.16649 4.52728 4.63396 4.13742 1.55523 4.29617 4.32301 1.73842 4.00286 1.76408 3.11947 4.27581 4.58882 4.13968 4.13035 3.65327 3.40793 2.23962 4.62924 3.16052 189f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

167 2.71616 4.54379 3.33734 2.89839 3.35881 3.52119 1.88099 3.03052 2.73076 2.72740 3.72287 3.29273 3.99214 3.17166 3.04330 2.87211 2.99426 2.11386 4.86847 3.42750 190 h---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

168 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 191 i---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

169 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 192 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

170 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 193 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

171 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 194 d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

172 3.09089 5.22592 2.56202 0.82634 4.59241 3.37774 3.98099 4.07700 2.88130 3.67069 4.64056 3.02951 3.99035 3.19510 3.30408 3.05927 3.39713 3.74605 5.73522 4.46059 195 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

173 3.03706 4.46123 4.46014 3.98106 1.57167 4.10443 4.24942 2.28280 3.85931 1.91458 3.22087 4.12421 4.47266 4.03641 4.01751 3.47073 3.30403 1.73003 4.62332 3.14202 196 f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

174 2.32225 4.32069 3.13729 2.87421 4.35136 1.65657 4.08217 3.79969 3.01777 3.44336 4.26128 3.17370 2.25259 3.31182 3.38967 2.14062 2.77627 3.26797 5.65409 4.39735 197 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

175 2.77356 4.55240 3.36005 3.02883 3.03128 2.19986 3.81466 3.28169 3.02848 2.90846 3.88407 3.37503 4.02902 3.36419 3.34043 2.92590 3.08810 3.02523 4.58745 1.50614 198 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

176 1.95337 4.19969 3.34800 3.07948 4.36705 2.35937 4.20484 3.79149 3.18170 3.46901 4.28439 3.26868 3.69495 3.46172 3.51355 1.24981 2.72315 3.22225 5.69329 4.47112 199 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

177 2.39774 4.20251 3.60629 3.18490 3.80064 2.23296 4.12488 2.85980 3.14287 2.80777 3.74384 3.41498 3.85700 3.45238 3.44611 2.62720 2.36371 1.66673 5.26621 4.04642 200 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

178 2.38790 5.01217 2.16371 1.84521 4.31095 3.32870 3.65438 3.71322 2.49136 3.30979 4.12031 2.83572 3.80369 2.79616 2.98853 2.65926 2.92717 2.85244 5.52656 4.13944 201 e- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

179 2.61109 4.80868 2.83140 2.42367 4.14867 3.33387 2.63708 3.60488 2.46727 3.19337 4.01193 2.36100 3.80714 2.83564 2.90423 2.19422 2.59690 3.24945 5.39695 4.04967 202 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

180 2.81732 5.09001 2.58061 2.36617 4.38635 3.33443 3.73003 3.93915 2.47400 3.47372 4.32793 1.64051 3.87553 2.12574 2.86476 2.79602 3.09304 3.56473 5.58156 4.19469 203 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

181 2.65819 4.65095 3.05975 2.68725 3.79892 3.36853 1.84754 3.44029 2.57169 3.07100 3.97226 3.12206 3.89114 3.03336 2.90858 2.75178 2.16790 3.13088 5.17761 3.79287 204 h - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

182 2.96406 5.52572 1.67512 1.28150 4.81416 3.24219 3.78335 4.33080 2.79447 3.83502 4.68921 2.70807 3.86182 2.94974 3.38467 2.84856 3.25200 3.90619 5.98418 4.48605 205 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

183 3.45825 4.81603 4.44812 4.19374 0.79153 4.10787 3.95340 2.96558 4.09750 2.30148 3.67455 4.19402 4.56467 4.22394 4.17942 3.71886 3.76029 2.96711 4.16127 2.51081 206 f -- -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

184 3.15114 4.53079 4.54737 4.17094 3.48096 4.22724 4.87288 0.90672 4.00200 2.03850 3.39535 4.39520 4.64605 4.33611 4.18143 3.73942 3.44853 1.86187 5.40162 4.15144 207 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

185 2.29359 4.57411 3.23618 2.72238 4.07343 3.33651 3.77690 3.40764 2.45363 3.07074 3.93300 3.13396 3.84538 2.96550 1.96761 2.66852 2.04261 3.07564 5.33763 4.07790 208 r - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

186 3.63652 4.88388 4.59704 4.28042 1.43903 4.38085 3.47632 3.33528 4.12987 2.66923 3.93490 4.06005 4.69349 4.11295 4.17606 3.75032 3.85988 3.25834 3.62050 1.04097 209 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

187 2.17639 4.26574 4.04726 3.51142 3.41588 3.78284 4.27057 2.20297 3.40765 2.18515 2.39451 3.78341 4.20778 3.68936 3.66373 3.11174 3.00081 1.61474 5.07651 3.88099 210 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

188 2.85659 4.85399 2.80347 2.70562 4.24217 3.29164 4.05411 3.98803 2.97206 3.61820 4.57439 0.91375 3.95207

3.32914 3.33018 2.91922 3.24472 3.59467 5.53072 4.18110 211 n---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

189 2.66941 5.00923 2.47300 2.32302 4.31106 3.35985 3.62270 3.75120 2.19826 3.29945 4.09839 2.86151 3.80561 2.23981 2.83829 2.65411 2.21029 3.38199 5.48709 4.11773 212 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

190 3.27661 4.60563 4.81638 4.28979 3.17506 4.53343 4.88108 1.60763 4.11630 1.03186 2.99695 4.54933 4.75580 4.30643 4.26407 3.90698 3.51388 2.04894 5.23833 4.09917 213 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

191 1.63294 4.41666 3.04918 2.73549 4.24684 3.09761 3.95513 3.66617 2.85127 3.31811 4.14383 2.29638 3.74809 3.16486 3.24963 1.95333 2.79798 3.20877 5.55041 4.26583 214 a---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

192 2.42661 5.01947 2.53569 1.98436 4.31175 3.35699 3.59114 3.76608 2.17969 3.29982 4.08000 2.84213 3.78084 2.51070 2.84308 2.60825 2.86464 3.38286 5.47827 3.40544 215 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

193 2.73220 4.34615 3.90677 3.46424 1.63890 3.64664 4.03956 2.62607 3.37613 2.27422 3.42454 3.68477 4.14040 3.64678 3.61607 3.02242 2.16275 2.48775 4.66374 3.20637 216f---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

194 2.16825 4.34510 4.19396 3.65621 3.35689 3.97994 4.39486 1.79160 3.53820 1.55186 3.23332 3.94174 4.34864 3.81543 3.78020 3.30539 3.12015 2.00109 5.11793 3.93073 217 l ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

195 2.79107 5.18477 2.71691 2.14080 4.52818 3.40467 3.62058 3.96898 1.73192 3.45474 4.25667 2.34029 3.85501 2.42487 2.63243 2.74760 3.01738 3.58213 5.57965 4.23023 218 k - - -

2.68618 4.42225 2.77519 2.73123 3.46354 2.40513 3.72494 3.29354 2.67741 2.69355 4.24690 2.90347 2.73739

3.18146 2.89801 2.37887 2.77519 2.98518 4.58477 3.61503
0.02689 4.02509 4.74744 0.61958 0.77255 0.48576 0.95510

196 2.17351 4.91848 2.82525 2.38430 4.24494 3.37072 3.63096 3.67443 2.18425 3.23659 4.04258 2.63554 3.81110 2.28399 2.79466 2.43337 2.89157 3.31531 5.43364 4.08866 219 a---

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

197 2.73064 4.86893 2.99005 2.58925 4.35918 3.38261 3.72140 3.77606 1.45572 3.34226 4.18943 3.04768 3.88536 2.88118 2.59856 2.08548 3.01111 3.40813 5.48642 4.20070 220 k - - -

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

198 2.56290 4.62791 2.74963 2.60888 4.43158 1.46710 3.99297 3.95890 2.92107 3.57347 4.42130 2.00642 3.80821 3.21235 3.32962 2.64710 2.97621 3.47103 5.69418 4.37504 221 g - - -

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

199 2.73189 4.38668 3.61904 3.07898 3.11938 3.68134 3.81351 2.76901 2.55667 1.72266 3.41540 3.43335 4.05792 3.26008 3.12675 2.97024 2.96137 2.59805 4.66904 2.43252 222 l ---

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

200 2.65108 4.74259 3.04709 2.29681 3.98555 3.45784 3.65624 2.67551 1.89518 2.98080 3.83352 3.01835 3.85857 2.82707 2.76275 2.71055 2.60859 3.02589 5.25791 3.96166 223 k- - -

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

201 2.38113 4.31424 3.32791 2.99399 4.12869 3.10910 4.08577 3.43979 2.98866 3.17399 4.06943 3.27264 2.27484 3.34184 3.31817 2.54702 1.45834 3.04225 5.49054 4.25327 224 t - - -

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

202 2.98916 5.10962 3.32022 2.74098 4.48074 3.61552 3.64222 3.89779 1.98743 3.35988 4.23864 3.16576 4.01180 2.07330 1.39674 2.99326 3.17981 3.58157 5.43508 4.22036 225 r - - -

2.68618 4.42225 2.77519 2.73123 3.46354 2.40513 3.72494 3.29354 2.67741 2.69355 4.24690 2.90347 2.73739
3.18146 2.89801 2.37887 2.77519 2.98518 4.58477 3.61503
0.02689 4.02509 4.74744 0.61958 0.77255 0.48576 0.95510

203 3.09044 4.44185 4.68618 4.13199 3.26701 4.35354 4.73982 1.51154 4.00631 1.73823 1.99577 4.37110 4.62020 4.19557 4.17179 3.69350 3.32913 1.76431 5.21204 4.08646 226 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

204 3.60096 4.91925 4.33214 4.07762 2.83833 3.90754 4.10001 3.63349 3.79776 3.00100 4.25985 4.19643 4.43850 4.17747 3.89848 3.80310 3.90961 3.52799 0.62664 2.83283 227 W---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

205 2.85659 4.85399 2.80347 2.70562 4.24217 3.29164 4.05411 3.98803 2.97206 3.61820 4.57439 0.91375 3.95207 3.32914 3.33018 2.91922 3.24472 3.59467 5.53072 4.18110 228 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

206 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 229 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

207 2.28767 4.25336 3.22565 3.03895 4.41562 1.37205 4.22746 3.89341 3.21589 3.56086 4.38699 3.25727 3.71863 3.49420 3.54255 1.71487 2.78132 3.30636 5.73165 4.49652 230 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

208 3.25623 4.57823 4.82697 4.30148 3.21152 4.53230 4.89679 1.44812 4.13378 1.15209 3.03278 4.55421 4.75989 4.32875 4.28350 3.90611 3.49523 1.98079 5.26405 4.11904 231 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

209 2.80010 4.33166 4.12940 3.66116 3.55045 3.84603 4.46252 1.34018 3.52807 2.25174 3.43779 3.91340 4.31270 3.85909 3.77462 3.22431 2.22474 1.92066 5.27920 4.06016 232 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

210 2.79829 4.93956 3.01406 2.59905 4.38362 3.43304 3.72082 3.79421 1.40715 3.34475 4.20267 3.06716 2.45331

2.87825 2.57747 2.82418 3.05809 3.44407 5.48224 4.21630 233 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

211 2.14795 5.03770 2.52238 1.85139 4.41195 3.28995 3.71268 3.84816 2.56224 3.42164 4.24450 2.05658 3.82237 2.86803 3.04128 2.70563 3.00132 3.46734 5.62053 4.23176 234 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

212 2.44376 4.49494 2.95928 2.65452 4.24938 2.20366 3.90473 3.66924 2.78324 3.30955 4.13709 2.29943 3.76449 3.10457 3.19217 2.54902 1.89843 3.23177 5.53976 4.24332 235 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

213 3.11698 4.44078 4.79517 4.23551 2.67649 4.40366 4.73857 1.47037 4.12062 1.44738 3.03832 4.44024 4.64572 4.25671 4.24830 3.74254 3.34840 1.77847 5.13226 3.97767 236 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

214 2.68834 5.15778 2.35823 2.07116 4.46480 3.32522 3.03255 3.94105 2.41427 3.44528 4.22121 2.54463 3.78861 2.21954 2.91428 2.41944 2.93063 3.53111 5.60196 4.18680 237 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

215 2.78137 5.18786 2.63785 2.12327 4.51511 3.37631 3.63283 3.96515 2.12021 3.46170 4.26394 2.22167 3.84352 2.04027 2.72768 2.73503 3.01511 3.57673 5.60021 4.23159 238 q - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

216 3.29986 4.65272 4.75365 4.23684 3.12824 4.50328 4.83093 1.87890 4.03958 0.89447 2.96781 4.50797 4.73645 4.25041 4.19306 3.88386 3.53958 2.16159 5.19499 4.03863 239 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

217 2.89919 5.34935 1.71617 2.17213 4.67656 3.23525 3.79474 4.23262 2.79260 3.76123 4.61425 1.50751 3.85502 2.96887 3.35665 2.81751 3.20239 3.80806 5.88804 4.40854 240 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

218 2.80413 4.99658 3.13747 2.58273 4.37486 3.50907 3.62429 3.76811 1.81527 3.28795 4.12050 3.05219 2.40061 2.76252 1.94630 2.81408 3.01946 3.42826 5.42318 4.16153 241 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

219 2.78727 5.25825 2.11249 1.98930 4.57502 3.27529 3.69467 4.06071 2.59247 3.57583 4.38220 2.01326 3.81702 2.83915 3.12919 2.10157 3.05347 3.64961 5.74702 4.30348 242 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

220 3.06362 4.40738 4.70321 4.15807 2.25304 4.31291 4.61746 1.40759 4.03918 1.78965 3.10819 4.34583 4.59211 4.19971 4.17936 3.65397 3.30298 1.78011 5.03341 3.78118 243 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

221 2.74310 5.10413 2.57119 1.79870 4.42891 3.33094 3.66692 3.88451 2.44904 3.41973 4.23166 2.83556 3.82732 2.22809 2.90068 2.13381 2.99672 3.50373 5.59369 4.20923 244 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

222 3.11590 4.45267 4.76412 4.28578 3.51743 4.40926 4.99791 1.04667 4.15057 2.00332 3.33678 4.50531 4.74212 4.42536 4.34502 3.81003 3.39025 1.52516 5.50474 4.29626 245 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

223 2.66285 4.37534 3.71357 3.25967 3.54783 3.54839 4.15639 2.55844 3.10913 1.83168 3.50335 3.56378 4.07135 3.49067 3.39233 2.92788 1.65978 2.40085 5.17034 3.92022 246 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

224 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 247 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

225 3.60096 4.91925 4.33214 4.07762 2.83833 3.90754 4.10001 3.63349 3.79776 3.00100 4.25985 4.19643 4.43850 4.17747 3.89848 3.80310 3.90961 3.52799 0.62664 2.83283 248 W---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.19054 4.02509 1.86030 0.61958 0.77255 0.48576 0.95510

226 2.31190 4.23221 3.20093 3.01641 4.06999 2.96221 4.12644 3.59174 3.08975 3.31246 4.23908 3.24220 3.70049 3.43956 3.37598 1.10062 2.79156 3.12823 5.44298 4.15348 249 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

227 3.23798 4.72953 3.82891 3.55712 2.29361 3.82278 3.61560 3.24807 3.41482 2.70363 3.92376 3.71568 4.29677 3.72237 3.59982 3.37530 3.52463 3.11833 3.93713 0.97636 250 y ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

228 2.98282 5.11011 0.92716 2.33101 4.48628 3.20589 3.92549 4.07302 3.00841 3.68247 4.66044 2.88705 3.85823 3.16463 3.52377 2.94589 3.32126 3.71287 5.65612 4.36023 251 d- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

229 2.65708 4.43313 3.32956 3.22817 4.37057 0.73893 4.34882 3.96866 3.40858 3.62358 4.59928 3.49387 3.82406 3.73324 3.64531 2.82881 3.13782 3.49820 5.46738 4.47226 252 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

230 2.83473 5.25837 1.37516 2.10126 4.57628 3.15435 3.73554 4.13385 2.75360 3.68191 4.54871 2.21985 3.78459 2.91774 3.31984 2.75590 3.14441 3.71856 5.80743 4.32822 253 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

231 1.87653 4.19185 3.40469 2.94377 3.81375 3.13129 3.95270 3.00747 2.90476 2.83368 3.73463 3.23401 2.87014 3.22707 3.24995 2.50645 2.33265 2.33069 5.22956 3.99695 254 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

232 2.87575 4.86127 2.89877 2.65342 4.02899 3.36186 3.81166 3.65879 2.47464 3.16177 4.18981 3.11309 3.91524 1.24532 2.75894 2.92378 3.17273 3.38886 5.30213 3.99546 255 q - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

233 2.41899 5.21973 1.80670 1.92196 4.52691 3.20434 3.65972 4.00235 2.59039 3.53493 4.35784 2.34466 3.76804 2.81406 3.13577 2.69270 3.03520 3.60188 5.71875 4.27077 256 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.40460 1.10034

234 2.81580 5.10307 2.79216 2.16513 4.47096 3.42522 3.64987 3.88379 1.46686 3.40247 4.22936 2.93968 3.88555 2.78962 2.57994 2.49116 3.04799 3.52196 5.54016 4.22559 257 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

235 2.83358 5.20058 2.53761 2.09198 4.51162 3.35379 3.68148 3.97571 2.40863 3.49193 4.32040 2.48310 3.86434 1.64646 2.81525 2.78610 3.08153 3.59969 5.63746 4.26133 258 q- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

236 2.43482 5.16048 2.04732 1.84642 4.49572 3.28349 3.68297 3.96379 2.56062 3.49693 4.29959 2.78033 3.80735 2.82537 3.08519 2.11200 3.00356 3.56137 5.67864 4.25482 259 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

237 2.34706 4.45402 3.19665 2.42774 3.69805 3.43232 3.75883 2.49411 2.66416 2.74040 3.61797 3.13325 3.86377 3.00067 3.06327 2.46650 2.29246 2.74007 5.08842 3.82155 260 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

238 2.24371 4.63974 3.17377 2.62187 3.85290 3.48107 3.68434 3.22312 2.41011 2.30770 3.73727 3.08964 3.88042 2.39579 2.48289 2.73901 2.87752 2.95645 5.16854 3.89489 261 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

239 2.63695 4.87264 2.88253 1.96285 4.12306 3.39891 3.61882 3.56319 2.36872 3.14390 3.95830 2.92286 3.81479

2.76660 2.52556 2.20831 2.87080 3.22744 5.35052 3.37891 262 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

240 2.85358 4.99403 3.08886 2.63719 4.37776 3.48290 3.68537 3.85142 2.16243 3.37009 4.22735 2.21122 3.94249 2.84227 1.48771 2.87143 3.09524 3.50308 5.44726 4.17392 263 r - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

241 2.75465 4.78117 3.22906 2.78590 4.35488 2.17295 3.81911 3.80189 2.36225 3.36434 4.22896 3.19546 3.92329 2.99973 1.40706 2.82785 3.05972 3.42919 5.46478 4.24602 264 r - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

242 2.20101 5.05804 2.47317 1.98804 4.36317 3.35363 3.60088 3.81801 2.20618 3.34357 4.12538 2.83753 3.78990 2.72407 2.57485 2.62833 2.89281 3.42934 5.51398 4.12789 265 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

243 2.75348 4.39430 3.70171 3.14573 3.36241 3.75526 4.01294 1.80133 2.98311 2.13442 2.82860 3.52809 4.11819 2.47948 3.28188 3.03460 2.98709 2.39123 4.98230 3.76796 266 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

244 2.61364 4.69998 3.12146 2.65979 4.27617 2.72110 3.75212 3.69618 2.36249 3.27491 4.10869 3.08671 3.84610 2.91961 1.61994 2.31938 2.92260 3.31091 5.44651 4.17600 267 r - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

245 2.45733 4.23093 4.00985 3.44521 3.32408 3.81725 4.16947 2.23469 3.34821 1.79842 2.76896 3.73818 4.18660 3.61374 3.60131 3.11208 2.62154 1.80191 4.94904 3.75739 268 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

246 2.76092 5.14264 1.70565 2.22245 4.56863 3.23368 3.75263 4.06575 2.68811 3.60327 4.42311 2.44221 3.81705 2.91194 3.22493 1.89100 3.06175 3.63884 5.77454 4.33594 269 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

247 3.11349 4.48621 4.63246 4.07039 3.17950 4.33632 4.67836 2.08695 3.93732 1.29228 2.22825 4.32892 4.59200 4.11854 4.10432 3.66807 3.34622 1.67574 5.14291 4.03188 270 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

248 2.71971 4.81987 2.85700 2.56299 4.22841 3.33437 3.80271 3.71370 2.53336 3.28043 4.16984 3.03870 1.75864 2.11513 2.88890 2.76818 3.02510 3.36563 5.47091 4.15744 271 p---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

249 2.97289 5.54922 1.50860 1.39490 4.83522 3.23803 3.78628 4.35630 2.80796 3.85609 4.71182 2.70120 3.86227 2.95335 3.40637 2.85290 3.26228 3.92797 6.00516 4.49906 272 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

250 3.32508 4.67165 4.72729 4.22925 1.99281 4.44057 4.42007 2.34080 4.08403 0.99220 2.97759 4.39966 4.67805 4.18892 4.19822 3.80743 3.55414 2.46146 4.67349 3.24070 273 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

251 2.75073 4.43475 3.55181 2.34149 3.46655 3.72841 3.99695 2.05397 2.94925 1.80947 3.37599 3.45472 4.10290 3.30123 3.28386 3.01000 2.98637 2.33408 5.06057 3.82578 274 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

252 1.88494 4.88238 2.61903 1.57478 4.33632 3.28033 3.79591 3.66483 2.65101 3.33863 4.20937 2.91965 3.84486 2.97237 3.09640 2.72567 3.00924 3.31495 5.59647 4.24410 275 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

253 2.82293 5.14889 2.73180 2.39082 4.48956 3.41412 3.64604 3.93246 1.88521 3.43481 4.25714 1.88069 3.87925 2.42818 2.61224 2.79021 3.05405 3.56270 5.56070 4.22736 276 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

254 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 277 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

255 3.32075 4.67106 4.65248 4.18787 1.42808 4.36915 4.17707 2.44930 4.03693 1.35663 3.11212 4.29060 4.64318 4.14491 4.14864 3.74047 3.55563 2.53074 4.41270 2.86475 278 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

256 2.24759 4.60593 3.04042 2.58380 4.12442 3.27735 3.75496 3.51675 2.11361 3.14698 3.97472 3.03517 3.79537 2.92994 2.93036 1.90830 2.50489 3.14958 5.38891 4.09154 279 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

257 2.92986 4.43291 4.33452 4.00469 3.66577 3.87938 4.78135 2.05459 3.87481 2.32779 3.60998 4.16937 4.43309 4.22468 4.07226 3.39436 3.28877 0.85226 5.47522 4.21531 280 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

258 3.07555 4.54456 4.17165 3.68951 2.63692 4.05019 3.94163 2.61906 3.49418 1.25994 3.29193 3.89312 4.39198 3.76636 3.68862 3.39142 3.31141 2.59153 4.34766 2.02939 281 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

259 2.85659 4.85399 2.80347 2.70562 4.24217 3.29164 4.05411 3.98803 2.97206 3.61820 4.57439 0.91375 3.95207 3.32914 3.33018 2.91922 3.24472 3.59467 5.53072 4.18110 282 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

260 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 283 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

261 2.85659 4.85399 2.80347 2.70562 4.24217 3.29164 4.05411 3.98803 2.97206 3.61820 4.57439 0.91375 3.95207 3.32914 3.33018 2.91922 3.24472 3.59467 5.53072 4.18110 284 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

262 1.43858 4.17262 3.46197 3.17982 4.28413 2.95862 4.24230 3.64182 3.22630 3.38304 4.23010 3.32547 3.70912 3.52089 3.53067 1.44573 2.72482 3.12450 5.64474 4.42553 285 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

263 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 286 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

264 2.74307 4.51705 3.42036 3.04746 2.87779 3.52330 3.74578 3.20288 3.00035 2.82798 3.81047 3.37158 4.03361 3.33906 3.30723 2.08907 3.05136 2.95530 4.45959 1.58668 287 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

265 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 288 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

266 3.47415 4.90106 4.06661 3.82674 2.31411 4.01031 3.69985 3.46280 3.67999 2.87675 4.12240 3.91595 4.48161 3.94901 3.83146 3.59477 3.76213 3.34085 3.96297 0.74537 289 y - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

267 3.06590 4.44659 4.57685 4.04403 1.61299 4.22908 4.39647 1.95419 3.92037 1.77072 3.09615 4.22291 4.52802 4.07839 4.06953 3.56593 3.30701 1.93196 4.78360 3.40738 290 f - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

268 2.60870 4.27440 3.61511 3.08337 3.46566 3.58777 3.96543 2.51818 2.99375 2.01440 3.42185 2.95296 4.01275

3.32065 3.30807 2.88412 2.53476 1.85585 4.97454 3.74232 291 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

269 2.96585 4.69526 3.58992 3.45271 4.43091 3.37425 4.51572 4.01796 3.52354 3.62729 4.68520 3.74295 0.58713 3.90765 3.74983 3.13863 3.41988 3.64553 5.57332 4.55440 292 P - --

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.19054 4.02509 1.86030 0.61958 0.77255 0.48576 0.95510

270 2.89331 5.05333 2.98954 2.55821 4.40661 3.47941 3.59759 3.81675 1.40054 3.30995 4.18773 3.01921 3.91542 2.33069 2.27380 2.88761 3.10173 3.49586 5.41357 4.16617 293 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

271 2.33815 4.83772 2.63892 2.08725 4.18880 2.83748 3.63933 3.61624 2.45851 3.20259 4.02467 2.83958 3.76436 2.48516 2.91281 2.36814 2.87531 3.26022 5.42481 4.07350 294 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

272 2.72453 5.16074 2.05455 1.93695 4.48138 2.74002 3.64595 3.95845 2.55403 3.48728 4.30034 2.39737 3.76070 2.79550 3.08997 2.34261 2.99410 3.55704 5.67109 4.23618 295 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

273 2.03246 4.29147 3.29315 2.78127 3.57563 3.31601 3.77695 2.98354 2.72297 2.67806 3.55939 3.16812 3.11459 3.06317 3.08341 2.37611 2.74277 2.70919 3.55740 3.72364 296 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

274 2.39983 4.44256 2.86269 2.57916 4.16160 2.56227 3.83532 3.57218 2.70942 3.22526 4.06283 2.22335 3.70868 3.04080 3.10904 2.50457 2.04754 3.15068 5.45869 4.16143 297 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

275 2.71118 4.24977 3.93892 3.40057 2.74462 3.77463 4.14039 1.86116 3.30717 2.06302 3.21694 3.70036 4.16399 3.58284 3.57194 3.08586 2.26934 2.07541 4.90669 3.65253 298 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

276 2.47273 4.49077 2.96554 2.58865 3.92862 3.20661 3.02877 3.40567 2.57809 3.04785 3.90219 3.01874 3.75850 2.96736 2.95498 1.83384 2.46325 3.05340 5.25968 3.94115 299 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

277 2.66096 4.78608 2.90902 2.45376 3.67156 3.41435 2.66487 3.43737 2.37202 3.02249 3.87755 2.64078 3.83076 2.50558 2.76268 2.69511 2.89080 3.13519 5.03827 3.04247 300 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.40460 1.10034

278 2.35280 5.08499 1.66136 2.24355 4.52064 3.24305 3.73444 3.99270 2.64603 3.53844 4.35045 2.44678 3.80721 2.88826 3.17351 2.32812 3.01748 3.57307 5.72351 4.30230 301 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

279 1.97030 4.21121 3.41504 3.03831 4.22733 2.50950 4.11541 3.63140 3.06682 3.30897 4.12984 3.25922 3.70518 3.36238 3.41696 1.65407 1.93366 3.12889 5.56294 4.33605 302 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

280 2.43503 5.04431 2.55485 2.33252 4.36675 3.37932 3.58876 3.81549 2.02126 3.33246 4.11340 2.57572 3.79834 2.70850 2.25513 2.63424 2.88877 3.42730 5.49043 4.12191 303 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

281 3.42223 4.78022 4.33714 3.94373 1.08833 4.22279 2.73056 3.32322 3.80837 2.74126 3.90317 3.89995 4.55033 3.90484 3.94359 3.55599 3.64354 3.18795 3.67790 1.74048 304 f - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

282 1.25768 4.17189 3.58631 3.26102 4.14671 3.02964 4.25932 3.27851 3.24025 3.15892 4.07464 3.39470 3.75976 3.55430 3.52728 2.47938 1.90838 2.88161 5.56334 4.35799 305 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

283 2.08170 4.57084 3.05405 2.62529 4.05159 3.29052 3.78750 3.40497 2.58532 3.06929 3.92758 3.06775 3.81579 2.25806 2.97215 2.62917 2.05152 3.06777 5.35578 4.06730 306 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

284 2.83175 5.14730 2.89501 1.71566 4.50964 3.47051 3.61557 3.92131 1.97022 3.40835 4.22614 2.96037 3.89450 2.74439 2.02273 2.80291 3.04473 3.55675 5.52226 4.22157 307 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

285 3.15998 5.30397 0.72157 2.44238 4.70300 3.33206 4.07440 4.32728 3.18878 3.90937 4.89317 3.00552 3.99587 3.31546 3.72392 3.10675 3.50263 3.95099 5.84132 4.55374 308 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

286 3.10465 4.41921 4.81466 4.32149 3.58133 4.45215 5.03748 1.30485 4.20927 2.08345 3.38653 4.53823 4.76727 4.47513 4.40756 3.84267 3.37231 1.15212 5.55075 4.34415 309 v - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

287 2.86282 4.34353 4.18270 3.63707 3.34634 3.98519 4.37187 1.77850 3.50843 1.57442 3.22490 3.92839 4.34363 3.79253 3.75031 3.30414 2.29534 2.01067 5.09916 3.91247 3101 ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

288 2.81017 5.21863 2.59352 1.87222 4.55344 3.36881 3.64927 4.00429 1.78795 3.49979 4.30902 2.27355 3.85315 2.78354 2.74442 2.75861 3.04813 3.61433 5.63373 4.26239 311 k---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

289 2.78345 5.07319 2.82941 2.42917 4.40388 3.42570 3.63635 3.85576 2.24103 3.36935 4.19039 1.95019 3.87310 2.16956 2.32851 2.76665 3.01448 3.49249 5.49894 4.17188 312 n---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

290 2.96493 4.42175 4.24644 3.72871 2.75501 3.97278 3.99940 1.90908 3.49686 2.07632 3.28935 3.90362 4.33545 3.77977 3.67133 3.32195 3.20919 2.44765 1.84148 2.93628 313 w - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

291 2.77068 5.21507 1.63809 2.20979 4.53543 3.28893 3.68499 4.00817 2.30622 3.53236 4.33841 2.45292 3.81734 2.82784 3.06700 2.71389 2.65575 3.60510 5.70473 4.27646 314 d ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

292 3.07555 4.54456 4.17165 3.68951 2.63692 4.05019 3.94163 2.61906 3.49418 1.25994 3.29193 3.89312 4.39198 3.76636 3.68862 3.39142 3.31141 2.59153 4.34766 2.02939 315 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

293 2.46723 4.30593 3.41146 2.94189 3.71832 2.03027 3.93625 2.99694 2.88166 2.73796 2.49579 3.28313 3.85679 3.22461 3.22167 2.65944 2.42962 2.72427 5.15318 3.91282 316g---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

294 2.70431 4.42044 3.55159 2.98113 3.55074 3.68133 3.92481 2.27836 2.08770 2.24339 3.45925 3.39572 4.05388 3.20959 3.13430 2.94496 2.93779 1.99785 5.06664 3.82890 317 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

295 3.60096 4.91925 4.33214 4.07762 2.83833 3.90754 4.10001 3.63349 3.79776 3.00100 4.25985 4.19643 4.43850 4.17747 3.89848 3.80310 3.90961 3.52799 0.62664 2.83283 318 W---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

296 1.98570 4.97518 1.41558 2.32162 4.50250 3.21472 3.84670 3.88113 2.81882 3.54191 4.41029 2.85524 3.83484 3.02864 3.33839 2.73358 3.07083 3.48636 5.76413 4.36173 319d---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

297 2.69267 4.54144 3.24044 2.91365 3.32451 1.59390 3.86112 3.33411 2.93967 2.97617 3.92164 3.29072 3.95793 3.28437 3.27656 2.83383 3.02119 3.05035 4.83161 2.16869 320 g - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

298 2.21146 5.00527 2.91460 2.22523 4.32023 3.42770 3.59528 3.74451 2.08955 3.27456 4.07496 2.92605 3.83286 2.27647 2.39487 2.68562 2.91731 3.38359 5.43862 4.10770 321 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

299 2.81830 4.78567 3.00119 2.68472 3.15135 3.49788 3.68581 3.46076 2.73029 3.04468 3.99274 1.69978 3.98400 3.09700 3.10366 2.87953 3.08823 3.18893 4.68571 2.21104 322 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

300 2.64885 4.94211 2.79848 2.36681 4.26955 3.36475 3.62791 3.70427 2.20331 3.25986 4.06190 2.32020 3.80721 2.50811 2.80560 2.21848 2.60129 3.33904 5.45181 4.09937 323 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

301 2.77130 5.11841 2.81513 2.20210 4.46166 3.42626 3.61280 3.89013 1.72485 3.39017 4.19532 2.91415 3.85823 2.21692 2.58496 2.47751 2.99421 3.51624 5.52419 4.19306 324 k - - -

2.68634 4.42241 2.77499 2.73139 3.46224 2.40529 3.72510 3.29308 2.67757 2.69371 4.24706 2.90363 2.73755 3.18162 2.89817 2.37878 2.77535 2.98534 4.58493 3.61349
0.19054 1.80294 4.74744 1.59375 0.22710 0.48576 0.95510

302 2.81830 4.78567 3.00119 2.68472 3.15135 3.49788 3.68581 3.46076 2.73029 3.04468 3.99274 1.69978 3.98400 3.09700 3.10366 2.87953 3.08823 3.18893 4.68571 2.21104 332 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

303 2.45078 4.83585 3.10014 2.52017 4.10118 3.47518 3.13718 3.07078 1.79435 3.08529 3.91462 3.01122 3.85880 2.76648 2.38592 2.71453 2.89794 3.18724 5.29651 4.00273 333 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

304 2.72071 4.38562 3.63683 3.10081 3.47607 3.70767 4.01238 2.20337 2.96292 2.31855 3.40113 3.48887 4.09819 2.46818 3.27800 2.99681 2.96723 1.69539 5.05050 3.81401 334 v- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

305 2.67950 4.88566 3.02495 2.29925 4.16327 3.45629 3.60778 3.56642 2.09891 3.13823 3.95856 2.97731 3.84695 2.32151 2.38190 2.69992 2.89944 2.52691 5.34041 4.03408 335 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

306 2.91402 5.39319 1.52296 2.15501 4.71409 3.23200 3.79389 4.27088 2.80268 3.79105 4.64407 1.67576 3.85525 2.96681 3.37850 2.82350 3.21615 3.84170 5.91979 4.42986 336 d - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

307 2.56890 4.50622 3.31100 2.87118 4.03458 3.30972 3.88971 3.35763 2.58003 3.05022 3.96233 3.23240 3.87462 3.11323 2.40892 2.70623 1.44002 3.03785 5.34723 4.09854 337 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

308 2.63362 5.03424 2.51857 2.29662 4.33547 3.35630 2.76825 3.79303 2.20584 3.31957 4.09719 2.84091 3.17158 2.50318 2.82936 2.38046 2.86908 3.40417 5.49087 4.10493 338 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

309 2.82659 5.20800 2.57242 2.08730 4.53914 3.36521 3.66607 3.98701 1.92933 3.49486 4.31462 1.83994 3.86208 2.80711 2.76495 2.77788 3.06869 3.60584 5.63596 4.26808 339 n- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

310 3.11414 4.43068 4.81561 4.32476 3.55929 4.45387 5.03709 1.14792 4.20760 2.04996 3.36501 4.54217 4.76839 4.47155 4.40283 3.84667 3.38230 1.32751 5.53992 4.33715 340 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

311 2.40396 4.35929 3.48101 2.91497 3.49471 3.59476 3.84852 2.28339 2.26694 2.45846 2.65064 3.32377 3.97669 3.15196 3.11428 2.85415 2.86442 2.48102 4.96886 3.73712 341 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

312 2.86527 4.62317 3.57011 3.49011 4.62505 0.52816 4.59887 4.26391 3.69199 3.89823 4.87687 3.73474 4.01908 4.00427 3.90917 3.04194 3.35840 3.76278 5.67765 4.73288 342 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

313 1.69752 4.18345 3.43150 3.16346 4.30119 2.95892 4.24125 3.66483 3.21931 3.40398 4.25258 3.31758 3.71103 3.51749 3.52451 1.23544 2.73257 3.14227 5.65899 4.43456 343 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

314 1.48834 4.17464 3.45665 3.17575 4.28735 2.95870 4.24102 3.64656 3.22362 3.38669 4.23351 3.32345 3.70917 3.51872 3.52871 1.39816 2.72573 3.12803 5.64702 4.42693 344 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

315 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 345 I - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

316 2.06584 4.19822 3.57712 3.15818 3.90798 3.14557 4.12723 3.04807 3.12205 2.93589 3.84949 3.37347 3.80465 3.43083 3.43658 1.60722 2.76076 1.98844 5.34623 4.12095 346 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

317 3.13957 4.48635 4.69093 4.17921 2.14551 4.33740 4.52133 1.31504 4.05073 1.76020 3.11148 4.35084 4.62044 4.20491 4.18599 3.69575 3.38191 2.05584 4.87047 3.49872 347 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

318 3.60096 4.91925 4.33214 4.07762 2.83833 3.90754 4.10001 3.63349 3.79776 3.00100 4.25985 4.19643 4.43850 4.17747 3.89848 3.80310 3.90961 3.52799 0.62664 2.83283 348 W---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

319 2.86527 4.62317 3.57011 3.49011 4.62505 0.52816 4.59887 4.26391 3.69199 3.89823 4.87687 3.73474 4.01908

4.00427 3.90917 3.04194 3.35840 3.76278 5.67765 4.73288 349 G - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

320 3.09089 5.22592 2.56202 0.82634 4.59241 3.37774 3.98099 4.07700 2.88130 3.67069 4.64056 3.02951 3.99035 3.19510 3.30408 3.05927 3.39713 3.74605 5.73522 4.46059 350 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

321 2.67457 5.07040 2.51288 2.32096 4.37867 3.37742 2.46681 3.83562 2.12444 3.34985 4.13318 2.59039 3.80325 2.71544 2.51562 2.64684 2.90571 3.44794 5.50375 4.13004 351 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

322 1.14955 4.15975 3.49222 3.22294 4.26745 2.95714 4.26847 3.60294 3.26518 3.36558 4.22220 3.34742 3.71321 3.55904 3.55623 1.83278 2.72661 3.09693 5.63978 4.42521 352 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

323 2.64228 4.88139 2.68741 2.38349 4.35033 2.21629 3.71811 3.79531 2.25822 3.36108 4.17488 2.21099 3.80719 2.87328 2.95866 2.38528 2.93469 3.40017 5.55484 4.19996 353 n - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

324 2.06373 4.76793 2.03727 2.40851 4.36490 3.20037 3.79444 3.79559 2.69130 3.40145 4.21915 2.90775 3.78264 2.96287 3.17508 1.88061 2.91020 3.37323 5.61541 4.25254 354 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

325 3.31291 4.68989 4.70501 4.16903 3.06956 4.47757 4.76869 2.21596 3.95514 0.85086 2.45756 4.45720 4.69951 4.16625 4.10977 3.84449 3.54474 2.34334 5.14329 4.01962 355 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

326 2.68556 4.91318 2.92743 2.46064 4.27560 3.40303 3.64009 3.69681 2.10413 3.25042 4.06639 2.64979 3.84250 2.78308 2.37643 1.87701 2.92907 3.34132 5.42809 4.11261 356 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

327 2.73384 5.16697 2.26038 2.05842 4.47205 3.30905 3.65623 3.94222 2.49309 3.46512 4.26233 2.79002 3.80787 2.19583 2.99662 2.01165 2.98632 3.54556 5.63826 4.22328 357 s - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

328 2.47304 5.20505 2.25958 1.72883 4.51131 3.31587 3.63621 3.98735 2.23621 3.49082 4.27593 2.78353 3.80166 2.49647 2.95232 2.67314 2.97505 3.57793 5.64854 4.22756 358 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

329 2.31309 4.23782 3.42672 2.99267 4.01638 2.16856 4.03209 3.37031 2.98113 3.08706 3.93683 3.25944 3.74798 3.29195 3.33356 2.14881 1.87650 2.54167 5.38693 4.15279 359 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

330 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 360 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

331 2.73070 4.83158 2.95882 2.31091 4.00995 3.46767 3.68062 3.40112 2.37768 2.31986 3.89168 3.01979 3.89268 1.95665 2.73773 2.77602 2.96076 3.13183 5.29737 3.98938 361 q - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

332 2.91182 5.20068 2.89705 2.24903 4.57907 3.49904 3.63025 3.97818 1.36116 3.45021 4.28561 2.98825 3.93283 2.45099 2.40200 2.87724 3.11802 3.62284 5.54679 4.26966 362 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

333 2.41393 4.72714 2.94864 2.46805 3.93736 3.40528 3.65291 3.37964 2.46157 3.00100 3.83688 2.68148 3.82558 2.31785 2.89170 2.46055 2.85036 3.07334 5.23863 2.87824 363 q- - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503

0.02689 4.02509 4.74744 0.61958 0.77255 0.48576 0.95510

334 2.61198 4.75416 2.76739 1.96203 4.19843 3.28646 3.75307 3.58268 2.58481 3.22154 4.06081 2.95032 3.81628 2.92853 3.01852 2.38680 1.89746 3.22765 5.46722 4.13649 364 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

335 2.19974 4.95289 2.98222 2.52036 4.32341 3.44754 3.65065 3.71385 1.59024 3.26670 4.10407 3.00641 3.88570 2.50656 2.54360 2.77309 2.99388 3.37404 5.43905 4.15062 365 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

336 2.63930 4.90473 2.64813 2.15091 4.31824 2.82213 3.70014 3.76303 2.54055 3.33295 4.14327 2.55980 2.31973 2.85291 3.01742 2.37161 2.92138 3.37746 5.54014 4.17172 366 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

337 3.33002 4.74841 4.43394 4.07687 3.19997 4.17458 4.68490 2.41486 3.84312 0.70779 3.17954 4.33748 4.58523 4.17477 3.99325 3.76683 3.61819 2.49136 5.13215 3.88991 367 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

338 3.27661 4.60563 4.81638 4.28979 3.17506 4.53343 4.88108 1.60763 4.11630 1.03186 2.99695 4.54933 4.75580 4.30643 4.26407 3.90698 3.51388 2.04894 5.23833 4.09917 368 l - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

339 2.72589 5.08508 2.78918 2.01263 4.41068 3.40328 3.60798 3.84476 1.96810 3.35893 4.15722 2.89084 3.83403 2.20531 2.66343 2.70000 2.60210 3.46923 5.51047 4.16207 369 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

340 1.55374 4.43261 3.01620 2.72155 4.24969 3.10364 3.95467 3.66488 2.85125 3.32280 4.15289 2.15851 3.75481 3.16599 3.24839 2.20449 2.81204 3.21299 5.55493 4.26670 370 a - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

341 3.01710 4.36412 4.67085 4.12132 2.29109 4.27203 4.60455 1.63727 4.00480 1.85684 3.14379 4.30821 4.56211 4.17766 4.15036 3.60918 3.25731 1.48378 5.05634 3.82126 371 v---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

342 3.22367 4.54297 4.81042 4.29477 3.28052 4.51488 4.91059 1.22931 4.12691 1.39564 3.11095 4.54183 4.76195 4.35034 4.28848 3.89419 3.46866 1.87895 5.31062 4.13865 372 i - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

343 2.63538 4.83548 2.97849 2.44469 4.05169 2.65172 3.12938 3.50730 2.33651 3.09262 3.91198 2.95563 3.82485 2.36990 2.51828 2.66186 2.86262 3.18284 5.29063 3.27945 373 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.02689 4.02509 4.74744 0.61958 0.77255 0.48576 0.95510

344 2.87128 4.82370 3.35071 2.80664 3.94897 3.61266 3.74750 3.24928 1.45400 2.14061 3.87283 3.23468 4.01891 2.94093 2.52181 2.95948 3.09277 3.04076 5.25603 3.98953 374 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.19054 4.02509 1.86030 0.61958 0.77255 0.48576 0.95510

345 2.23775 4.15855 3.30025 3.00592 4.06012 2.96551 4.07132 3.35269 2.99940 3.13830 4.02753 3.22203 3.67752 3.34873 3.30253 2.02036 1.52718 2.93111 5.44319 4.20039 375 t - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

346 2.74399 4.99909 2.69002 2.35595 4.23084 3.35595 2.95780 3.74658 2.02829 3.28474 4.12100 2.05927 3.82464 2.76246 2.62644 2.73093 2.98086 3.40001 5.40330 4.04864 376 k - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

347 2.29867 4.61639 2.31944 2.44568 3.95273 2.83167 3.68936 2.78374 2.56224 2.98071 3.83580 2.94017 3.77569 2.87735 3.01242 2.60957 2.81529 2.96635 5.27868 3.96005 377 a ---

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.03160 3.86615 4.58850 0.61958 0.77255 0.63100 0.75941

348 2.64330 4.95659 2.26242 2.04359 4.27999 3.25698 3.63141 3.71459 2.48703 3.29015 4.10632 2.78289 2.72426

2.78273 2.98069 2.62871 2.52159 3.34724 5.50264 4.11839 378 e - - -

2.68618 4.42225 2.77519 2.73123 3.46354 2.40513 3.72494 3.29354 2.67741 2.69355 4.24690 2.90347 2.73739 3.18146 2.89801 2.37887 2.77519 2.98518 4.58477 3.61503
0.11129 3.86615 2.47241 0.61958 0.77255 0.63100 0.75941

349 2.71240 4.96152 2.57638 2.05512 4.31062 2.68638 3.61078 3.72641 1.89653 3.28651 4.12646 2.82370 3.78758 2.76814 2.68529 2.70076 2.96495 3.37534 5.46373 4.13251 379 k - - -

2.68621 4.42229 2.77523 2.73127 3.46357 2.40516 3.72422 3.29357 2.67744 2.69358 4.24693 2.90350 2.73743 3.18150 2.89804 2.37871 2.77523 2.98522 4.58480 3.61507
0.19020 1.75326 * 0.66005 0.72738 0.00000

## Claims

**1.** A cleaning composition comprising one or more polypeptides having hexosaminidase activity,

wherein the polypeptide comprises one or more Glyco_hydro_20 catalytic domains,
wherein the polypeptide comprises

the conserved motif I [IV]P[ED][LVI]DXP[AN]H (SEQ ID NO 21),
the conserved motif II NYN[AS]Y[SY]LY (SEQ ID NO 22) and/or
the conserved motif III GXDE (SEQ ID NO 41); and

wherein said composition

(a) is a solid, preferably granular, laundry detergent composition and
further comprises

(a1) at least one zeolite builder, preferably in an amount of 10 to 50 wt.-%, more preferably 20-30 wt.-%;
(a2) at least one phosphonate builder, preferably in an amount of 0.1 to 5 wt.-%, more preferably 0.4 to 1.5 wt.-%;
(a3) at least one further enzyme, preferably a cellulase, preferably in an amount of active enzyme of 100 to 5000 ppb, more preferably 1000 to 2000 ppb; and
(a4) at least one polymer, preferably a polyvinylpyrrolidon polymer, preferably in an amount of 0.01 to 1 wt.-%, more preferably 0.1 to 0.3 wt.-%; or

(b) is a solid laundry detergent composition and
further comprises

(b1) at least one silicate builder, preferably in an amount of 2 to 20 wt.-%, more preferably 5-10 wt.-%;
(b2) optionally carboxymethylcellulose, preferably in an amount of 0.1 to 10 wt.-%, more preferably 0.1 to 4 wt.-%;
(b3) at least one further enzyme, preferably a cellulase, preferably in an amount of active enzyme of 0.1 to 100 ppm, more preferably 0.1 to 10 ppm;
(b4) optionally at least one soil release polymer in an amount of 0.1 to 3 wt.-%, more preferably 0.1 to 1.0 wt.-%; and
(b5) at least one bleaching system, comprising a bleaching agent, a bleach activator and a bleach catalyst, preferably in an amount of 0.1 to 50 wt.-%, more preferably 0.1 to 30 wt.-%; or

(c) is liquid laundry detergent composition and
further comprises

(c1) at least one surfactant, preferably nonionic surfactant, preferably in an amount of 1 to 20 wt.-%, preferably 3 to 15 wt.-%;
(c2) optionally at least one phosphonate builder, preferably in an amount of 0.1 to 3 wt.-%, more

preferably 0.25 to 1.5 wt.-%

(c3) optionally at least at least one further enzyme, preferably a cellulase, preferably in an amount of enzyme composition of 0.001 to 1 wt.-%, more preferably 0.001 to 0.6 wt.-%; and

(c4) optionally at least one organic solvent, preferably glycerol, preferably in an amount of 0.1 to 10 wt.-%, more preferably 0.1 to 5 wt.-%; or

(d) is a liquid laundry detergent in unit dose form, preferably a pouch comprising a watersoluble film, and further comprises

(d1) water in an amount of up to 20 wt.-%, preferably 5 to 15 wt.-%;

(d2) optionally at least one bittering agent, preferably Benzyldiethyl(2,6-xylylcarbamoyl)-methylammoniumbenzoate, preferably in an amount of 0.00001 to 0.04 wt.-%;

(d3) optionally at least one optical brightener, preferably in an amount of 0.01 to 2 wt.-%, more preferably 0.01 to 1 wt.-%; and

(d4) optionally at least one polymer, preferably in an amount of 0.01 to 7 wt.-%, more preferably 0.1 to 5 wt.-%; or

(e) is a fabric finisher and further comprises

(e1) at least one softening silicone, preferably an amino-functionalized silicone, preferably in an amount of 0.1 to 10 wt.-%, more preferably 0.1 to 2 wt.-%;

(e2) at least one perfume, preferably at least partially encapsulated in microcapsules, more preferably at least partially encapsulated in melamine-formaldehyde microcapsules, preferably in an amount of 0.01 to 3 wt.-%, more preferably 0.1 to 1 wt.-%;

(e3) optionally polyquaternium 10 in an amount of 0.1 to 20 wt.-%, preferably 0.1 to 13 wt.-%;

(e4) optionally polyquaternium 37 in an amount of 0.1 to 20 wt.-%, preferably 0.1 to 13 wt.-%;

(e5) optionally a plant-based esterquat, preferably a canola- or palm-based esterquat, in an amount of 0.1 to 20 wt.-%, preferably 0.1 to 13 wt.-%; and

(e6) optionally adipic acid, in an amount of 0.1 to 20 wt.-%, preferably 0.1 to 13 wt.-%; or

(f) is an acidic cleaning agent, preferably having a pH less than 6, and further comprises

(f1) plant-based or bio-based surfactants, preferably each in an amount of 0.1 to 5, more preferably each in an amount of 0.1 to 2 wt.-%;

(f2) at least one acidic biocide, preferably selected from acids, more preferably HCl and formic acid; and

(f3) at least one soil release, water repellant or water spreading polymer, preferably in an amount of 0.01 to 3 wt.-%, more preferably 0.01 to 0.5 wt.-%; or

(g) is a neutral cleaning agent, preferably having a pH between 6.0 and 7.5, and further comprises

(g1) plant-based or bio-based surfactants, preferably each in an amount of 0.1 to 5, more preferably each in an amount of 0.1 to 2 wt.-%;

(g2) at least one biocide, preferably selected from quaternary ammonium compounds and alcohols; and

(g3) at least one soil release, water repellant or water spreading polymer, preferably in an amount of 0.01 to 3 wt.-%, more preferably 0.01 to 0.5 wt.-%; or

(h) is an alkaline cleaning agent, preferably having a pH of more than 7.5, and further comprises

(h1) plant-based or bio-based surfactants, preferably each in an amount of 0.1 to 5, more preferably each in an amount of 0.1 to 2 wt.-%; or

(i) is a hand dishwashing agent, preferably liquid hand dishwashing agent, and further comprises

(i1) at least one anionic surfactant, preferably in an amount of 0.1 to 40 wt.-%, more preferably 5 to 30 wt.-%;

(i2) at least one amphoteric surfactant, preferably betain, preferably in an amount of 0.1 to 25 wt.-%, more preferably 1 to 15 wt.-%;

(i3) at least one nonionic surfactant, preferably in an amount of 0.1 to 25 wt.-%, more preferably 2 to

10 wt.-%;
(i4) at least one further enzyme, preferably selected from proteases, amylases and combinations thereof, preferably in an amount of enzyme composition of up to 1 wt.-%, more preferably up to 0.6 wt.-%; or

(j) is an automatic dishwashing composition and further comprises

(j1) at least one builder selected from citrate, aminocarboxylates and combinations thereof, preferably in an amount of 5 to 30 wt.-%, more preferably 10 to 20 wt.-%;
(j2) at least one phosphonate builder, preferably in an amount of 0.1 to 5 wt.-%, more preferably 0.4 to 1.5 wt.-%;
(j3) at least one nonionic surfactant, preferably in an amount of 0.1 to 10 wt.-%, more preferably 1 to 5 wt.-%;
(j4) at least one bleaching system, comprising a bleaching agent, a bleach activator and a bleach catalyst, preferably in an amount of 0.1 to 50 wt.-%, more preferably 0.1 to 30 wt.-%;
(j5) at least one polymer selected from sulfopolymers, cationic polymers and polyacrylates, preferably in an amount of 0.01 to 15 wt.-%, more preferably 2 to 10 wt.-%; or

(k) further comprises
(k1) at least one sulfopolymer, preferably in an amount of 1 to 15, more preferably 2 to 10 wt.-% and is preferably a dishwashing, more preferably an automatic dishwashing composition; or
(l) further comprises
(l1) at least one adjunct ingredient selected from probiotics, preferably microbes, spores or combinations thereof; or
(m) is in unit dose form, preferably a pouch, and comprises at least 2, preferably 2, 3, 4 or 5 separate compartments;

wherein the polypeptide has at least 93%, at least 94%, at least 95%, at least 96%, at least 97%, at least 98%, at least 99% or 100% sequence identity to the mature polypeptide of SEQ ID NO 20, SEQ ID NO 33, SEQ ID NO 34, or SEQ ID NO 35.

**2.** The composition according to claim 1, wherein the polypeptide has N-acetylglucosaminidase activity and/or β-N-acetylglucosamininidase activity.

**3.** The composition according to any of claims 1 or 2, wherein the polypeptide comprises one or more of the motifs

ARAYYPV (SEQ ID NO 42),
AWNDGID (SEQ ID NO 43),
DDQNVGI (SEQ ID NO 44) or
DPRIH (SEQ ID NO 45).

**4.** The composition according to any of claims 1 to 3, wherein the composition is selected from compositions (a), (b), (d), (k), (l), and (m) and further comprises from up to 50 wt %, from about 5 wt % to about 40 wt %, from about 5 wt % to about 30 wt %, from about 5 wt % to about 20 wt % or from about 5 wt % to about 10 wt % of at least one surfactant, wherein the surfactant is preferably anionic and/or nonionic.

**5.** Use of a composition of any of claims 1 to 4 for deep-cleaning of an item, wherein the item is a textile.

**6.** A method for cleaning, preferably laundering, an item comprising the steps of:

a. exposing an item to a wash liquor comprising a cleaning composition according to any of claims 1 to 4;
b. optionally completing at least one wash cycle; and
c. optionally rinsing the item,

wherein the item is a textile or a hard surface.

**7.** Use of a composition of any of claims 1 to 4,

(i) for cleaning or conditioning of an item;

(ii) for preventing, reducing or removing stickiness of the item;
(iii) for pretreating stains on the item;
(iv) for preventing, reducing or removing redeposition of soil during a wash cycle;
(v) for preventing, reducing or removing adherence of soil to the item;
(vi) for maintaining or improving whiteness of the item; or
(vii) for preventing, reducing or removing malodor from the item, wherein the item is a textile.

## Patentansprüche

**1.** Reinigungszusammensetzung, umfassend ein oder mehrere Polypeptide, die Hexosaminidaseaktivität aufweisen, wobei das Polypeptid ein oder mehrere Glyco_hydro_20 katalytische Domänen umfasst,

wobei das Polypeptid umfasst
das konservierte Motiv I [IV]P[ED][LVI]DXP[AN]H (SEQ ID NO 21),
das konservierte Motiv II NYN[AS]Y[SY]LY (SEQ ID NO 22) und/oder
das konservierte Motiv III GXDE (SEQ ID NO 41); und
wobei die Zusammensetzung
(a) eine feste, vorzugsweise granulatförmige Wäschewaschmittelzusammensetzung ist und ferner umfasst

(a1) mindestens eine Zeolithbuildersubstanz, vorzugsweise in einer Menge von 10 bis 50 Gew.-%, stärker bevorzugt 20-30 Gew.-%;
(a2) mindestens eine Phosphonatbuildersubstanz, vorzugsweise in einer Menge von 0,1 bis 5 Gew.-%, stärker bevorzugt 0,4 bis 1,5 Gew.-%;
(a3) mindestens ein weiteres Enzym, vorzugsweise eine Cellulase, vorzugsweise in einer Menge von aktivem Enzym von 100 bis 5000 ppb, stärker bevorzugt 1000 bis 2000 ppb; und
(a4) mindestens ein Polymer, vorzugsweise ein Polyvinylpyrrolidonpolymer, vorzugsweise in einer Menge von 0,01 bis 1 Gew.-%, stärker bevorzugt 0,1 bis 0,3 Gew.-%, oder

(b) eine feste Waschmittelzusammensetzung ist und ferner umfasst

(b1) mindestens eine Silikatbuildersubstanz, vorzugsweise in einer Menge von 2 bis 20 Gew.-%, stärker bevorzugt 5-10 Gew.-%;
(b2) optional Carboxymethylcellulose, vorzugsweise in einer Menge von 0,1 bis 10 Gew.-%, stärker bevorzugt 0,1 bis 4 Gew.-%;
(b3) mindestens ein weiteres Enzym, vorzugsweise eine Cellulase, vorzugsweise in einer Menge von aktivem Enzym von 0,1 bis 100 ppb, stärker bevorzugt 0,1 bis 10 ppb;
(b4) optional mindestens ein Schmutzfreisetzungspolymer in einer Menge von 0,1 bis 3 Gew.-%, stärker bevorzugt 0,1 bis 1,0 Gew.-%; und
(b5) mindestens ein Bleichsystem, umfassend ein Bleichmittel, einen Bleichaktivator und einen Bleichkatalysator, vorzugsweise in einer Menge von 0,1 bis 50 Gew.-%, stärker bevorzugt 0,1 bis 30 Gew.-%; oder

(c) eine flüssige Wäschewaschmittelzusammensetzung ist und ferner umfasst

(c1) mindestens ein Tensid, vorzugsweise ein nichtionisches Tensid, vorzugsweise in einer Menge von 1 bis 20 Gew.-%, stärker bevorzugt 3 bis 15 Gew.-%,
(c2) optional mindestens eine Phosphonatbuildersubstanz, vorzugsweise in einer Menge von 0,1 bis 3 Gew.-%, stärker bevorzugt 0,25 bis 1,5 Gew.-%;
(c3) optional mindestens ein weiteres Enzym, vorzugsweise eine Cellulase, vorzugsweise in einer Menge von Enzymzusammensetzung von 0,001 bis 1 Gew.-%, stärker bevorzugt 0,001 bis 0,6 Gew.-%; und
(c4) optional mindestens ein organisches Lösungsmittel, vorzugsweise ein Glycerin, vorzugsweise in einer Menge von 0,1 bis 10 Gew.-%, stärker bevorzugt 0,1 bis 5 Gew.-%, oder

(d) ein flüssiges Wäschewaschmittel in Einheitsdosisform ist, vorzugsweise ein Beutel, umfassend einen wasserlöslichen Film und ferner

(d1) Wasser in einer Menge von bis zu 20 Gew.-%, vorzugsweise 5 bis 15 Gew.-%,
(d2) optional mindestens ein Bitterstoff, vorzugsweise Benzyldiethyl(2,6-xylylcarbamoyl)-methylammoniumbenzoat, vorzugsweise in einer Menge von 0,00001 bis 0,04 Gew.-%,
(d3) optional mindestens einen optischen Aufheller, vorzugsweise in einer Menge von 0,01 bis 2 Gew.-%, stärker bevorzugt 0,01 bis 1 Gew.-%; und
(d4) optional mindestens ein Polymer, vorzugsweise in einer Menge von 0,01 bis 7 Gew.-%, stärker bevorzugt 0,1 bis 5 Gew.-%; oder

(e) ein Gewebeveredler ist und ferner umfasst

(e1) mindestens ein Weichmachersilikon, vorzugsweise ein aminofunktionalisiertes Silikon, vorzugsweise in einer Menge von 0,1 bis 10 Gew.-%, stärker bevorzugt 0,1 bis 2 Gew.-%;
(e2) mindestens einen Duftstoff, vorzugsweise mindestens teilweise in Mikrokapseln eingekapselt, stärker bevorzugt mindestens teilweise in MelaminFormaldehyd-Mikrokapseln eingekapselt, vorzugsweise in einer Menge von 0,01 bis 3 Gew.-%, stärker bevorzugt 0,1 bis 1 Gew.-%;
(e3) optional Polyquaternium 10 in einer Menge von 0,1 bis 20 Gew.-%, vorzugsweise 0,1 bis 13 Gew.-%;
(e4) optional Polyquaternium 37 in einer Menge von 0,1 bis 20 Gew.-%, vorzugsweise 0,1 bis 13 Gew.-%;
(e5) optional ein Esterquat auf pflanzlicher Basis, vorzugsweise ein Esterquat auf Raps- oder Palmenbasis, in einer Menge von 0,1 bis 20 Gew.-%, vorzugsweise 0,1 bis 13 Gew.-%; und
(e6) optional Adipinsäure in einer Menge von 0,1 bis 20 Gew.-%, vorzugsweise 0,1 bis 13 Gew.-%; oder

(f) ein Säurereinigungsmittel ist, das vorzugsweise einen pH-Wert unter 6 aufweist und ferner umfasst

(f1) Tenside auf pflanzlicher Basis oder auf Bio-Basis, vorzugsweise jeweils in einer Menge von 0,1 bis 5, stärker bevorzugt in einer Menge von 0,1 bis 2 Gew.-%;
(f2) mindestens ein saures Biozid, vorzugsweise ausgewählt aus Säuren, stärker bevorzugt HCl und Ameisensäure; und
(f3) mindestens ein Schmutzfreisetzungs-, Wasserabweisungs- oder Wasserausbreitungspolymer, vorzugsweise in einer Menge von 0,01 bis 3 Gew.-%, stärker bevorzugt 0,01 bis 0,5 Gew.-%; oder

(g) ein Neutralreinigungsmittel ist, das vorzugsweise einen pH-Wert zwischen 6,0 und 7,5 aufweist und ferner umfasst

(g1) Tenside auf pflanzlicher Basis oder auf Bio-Basis, vorzugsweise jeweils in einer Menge von 0,1 bis 5, stärker bevorzugt in einer Menge von 0,1 bis 2 Gew.-%;
(g2) mindestens ein Biozid, das vorzugsweise aus quaternären Ammoniumverbindungen und Alkoholen ausgewählt ist; und
(g3) mindestens ein Schmutzfreisetzungs-, Wasserabweisungs- oder Wasserausbreitungspolymer, vorzugsweise in einer Menge von 0,01 bis 3 Gew.-%, stärker bevorzugt 0,01 bis 0,5 Gew.-%; oder

(h) ein alkalisches Reinigungsmittel ist, das vorzugsweise einen pH-Wert über 7,5 aufweist, und ferner umfasst
(h1) Tenside auf pflanzlicher Basis oder auf Bio-Basis, vorzugsweise jeweils in einer Menge von 0,1 bis 5, stärker bevorzugt in einer Menge von 0,1 bis 2 Gew.-%, oder
(i) ein Handgeschirrspülmittel ist, vorzugsweise ein flüssiges Handgeschirrspülmittel, und ferner umfasst

(i1) mindestens ein anionisches Tensid, vorzugsweise in einer Menge von 0,1 bis 40 Gew.-%, stärker bevorzugt 5 bis 30 Gew.-%;
(i2) mindestens ein amphoteres Tensid, vorzugsweise Betain, vorzugsweise in einer Menge von 0,1 bis 25 Gew.-%, stärker bevorzugt 1 bis 15 Gew.-%;
(i3) mindestens ein nichtionisches Tensid, vorzugsweise in einer Menge von 0,1 bis 25 Gew.-%, stärker bevorzugt 2 bis 10 Gew.-%;
(i4) mindestens ein weiteres Enzym, das vorzugsweise aus Proteasen, Amylasen und Kombinationen davon ausgewählt ist, vorzugsweise in einer Menge von Enzymzusammensetzung von bis zu 1 Gew.-%, stärker bevorzugt bis zu 0,6 Gew.-%, oder

(j) eine Geschirrspülzusammensetzung ist und ferner umfasst

(j1) mindestens eine Buildersubstanz, die aus Citrat, Aminocarboxylaten und Kombinationen davon ausgewählt ist, vorzugsweise in einer Menge von 5 bis 30 Gew.-%, stärker bevorzugt 10 bis 20 Gew.-%;
(j2) mindestens eine Phosphonatbuildersubstanz, vorzugsweise in einer Menge von 0,1 bis 5 Gew.-%,

stärker bevorzugt 0,4 bis 1,5 Gew.-%;
(j3) mindestens ein nichtionisches Tensid, vorzugsweise in einer Menge von 0,1 bis 10 Gew.-%, stärker bevorzugt 1 bis 5 Gew.-%;
(j4) mindestens ein Bleichsystem, umfassend ein Bleichmittel, einen Bleichaktivator und einen Bleichkatalysator, vorzugsweise in einer Menge von 0,1 bis 50 Gew.-%, stärker bevorzugt 0,1 bis 30 Gew.-%;
(j5) mindestens ein Polymer, ausgewählt aus Sulfopolymeren, kationischen Polymeren und Polyacrylaten, vorzugsweise in einer Menge von 0,01 bis 15 Gew.-%, stärker bevorzugt 2 bis 10 Gew.-%; oder

(k) ferner umfasst
(k1) mindestens ein Sulfopolymer, vorzugsweise in einer Menge von 1 bis 15, stärker bevorzugt 2 bis 10 Gew.-%, und vorzugsweise eine Geschirrspülzusammensetzung, stärker bevorzugt eine automatische Geschirrspülzusammensetzung, ist; oder
(l) ferner umfasst
(l1) mindestens einen zusätzlichen Inhaltsstoff, der aus Probiotika, vorzugsweise Mikroben, Sporen oder Kombinationen davon ausgewählt ist; oder
(m) in Einheitsdosisform, vorzugsweise in einem Beutel, vorliegt, und mindestens 2, vorzugsweise 2, 3, 4 oder 5 separate Fächer umfasst;

wobei das Polypeptid mindestens 93 %, mindestens 94 %, mindestens 95 %, mindestens 96 %, mindestens 97 %, mindestens 98 %, mindestens 99 % oder 100 % Sequenzidentität zu dem reifen Polypeptid von SEQ ID NO 20, SEQ ID NO 33, SEQ ID NO 34 oder SEQ ID NO 35 aufweist.

**2.** Zusammensetzung nach Anspruch 1, wobei das Polypeptid N-Acetylglucosaminidaseaktivität und/oder β-N-Acetylglucosaminidaseaktivität aufweist.

**3.** Zusammensetzung nach einem der Ansprüche 1 oder 2, wobei das Polypeptid eines oder mehrere der Motive umfasst

ARAYYPV (SEQ ID NO 42),
AWNDGID (SEQ ID NO 43),
DDQNVGI (SEQ ID NO 44) oder
DPRIH (SEQ ID NO 45).

**4.** Zusammensetzung nach einem der Ansprüche 1 bis 3, wobei die Zusammensetzung aus Zusammensetzungen (a), (b), (d), (k), (l) und (m) ausgewählt ist und ferner von bis zu 50 Gew.-%, von etwa 5 Gew.-% bis etwa 40 Gew.-%, von etwa 5 Gew.-% bis etwa 30 Gew.-%, von etwa 5 Gew.-% bis etwa 20 Gew.-% oder von etwa 5 Gew.-% bis etwa 10 Gew.-% mindestens eines Tensids umfasst, wobei das Tensid vorzugsweise anionisch und/oder nichtionisch ist.

**5.** Verwendung einer Zusammensetzung nach einem der Ansprüche 1 bis 4 zum Tiefenreinigen eines Objekts, wobei das Objekt eine Textilie ist.

**6.** Verfahren zum Reinigen, vorzugsweise zum Waschen, eines Objekts, umfassend die Schritte:

a. Aussetzen eines Objekts einer Waschlauge, umfassend eine Reinigungszusammensetzung nach einem der Ansprüche 1 bis 4;
b. optional Abschließen mindestens eines Waschzyklus; und
c. optional Spülen des Objekts,

wobei das Objekt eine Textilie oder eine harte Oberfläche ist.

**7.** Verwendung einer Zusammensetzung nach einem der Ansprüche 1 bis 4,

(i) zum Reinigen oder Aufbereiten eines Objekts;
(ii) zum Verhindern, Reduzieren oder Entfernen von Klebrigkeit des Objekts;
(iii) zum Vorbehandeln von Verunreinigungen auf dem Objekt;
(iv) zum Verhindern, Reduzieren oder Entfernen von Wiederablagerung von Verunreinigungen während eines Waschzyklus;
(v) zum Verhindern, Reduzieren oder Entfernen einer Haftung von Verunreinigungen am Objekt;

(vi) zum Erhalten oder Verbessern einer Weiße des Objekts, oder
(vii) zum Verhindern, Reduzieren oder Entfernen von schlechten Gerüchen des Objekts,

wobei das Objekt eine Textilie ist.

**Revendications**

1. Composition de nettoyage comprenant un ou plusieurs polypeptides ayant une activité d'hexosaminidase, dans laquelle le polypeptide comprend un ou plusieurs domaines catalytiques Glyco_hydro_20,

dans laquelle le polypeptide comprend
le motif conservé I [IV]P[ED][LVI]DXP[AN]H (SEQ ID NO 21),
le motif conservé II NYN[AS]Y[SY]LY (SEQ ID NO 22) et/ou
le motif conservé III GXDE (SEQ ID NO 41) ; et
dans laquelle ladite composition
(a) est une composition solide, de préférence granulaire, de détergent à lessive et comprend en outre

(a1) au moins un adjuvant zéolite, de préférence en une quantité de 10 à 50 % en poids, plus préférablement 20 à 30 % en poids ;
(a2) au moins un adjuvant phosphonate, de préférence en une quantité de 0,1 à 5 % en poids, plus préférablement 0,4 à 1,5 % en poids ;
(a3) au moins une enzyme supplémentaire, de préférence une cellulase, de préférence en une quantité d'enzyme active de 100 à 5000 ppb, plus préférablement 1000 à 2000 ppb ; et
(a4) au moins un polymère, de préférence un polymère polyvinylpyrrolidone, de préférence en une quantité de 0,01 à 1 % en poids, plus préférablement 0,1 à 0,3 % en poids ; ou

(b) est une composition solide de détergent à lessive et comprend en outre

(b1) au moins un adjuvant silicate, de préférence en une quantité de 2 à 20 % en poids, plus préférablement 5 à 10 % en poids ;
(b2) facultativement de la carboxyméthylcellulose, de préférence en une quantité de 0,1 à 10 % en poids, plus préférablement 0,1 à 4 % en poids ;
(b3) au moins une enzyme supplémentaire, de préférence une cellulase, de préférence en une quantité d'enzyme active de 0,1 à 100 ppm, plus préférablement 0,1 à 10 ppm ;
(b4) facultativement au moins un polymère antisalissure en une quantité de 0,1 à 3 % en poids, plus préférablement 0,1 à 1,0 % en poids ; et
(b5) au moins un système de blanchiment, comprenant un agent de blanchiment, un activateur de blanchiment et un catalyseur de blanchiment, de préférence en une quantité de 0,1 à 50 % en poids, plus préférablement 0,1 à 30 % en poids ; ou

(c) est une composition liquide de détergent à lessive et comprend en outre

(c1) au moins un agent tensioactif, de préférence un agent tensioactif non ionique, de préférence en une quantité de 1 à 20 % en poids, de préférence 3 à 15 % en poids ;
(c2) facultativement au moins un adjuvant phosphonate, de préférence en une quantité de 0,1 à 3 % en poids, plus préférablement 0,25 à 1,5 % en poids
(c3) facultativement au moins une enzyme supplémentaire, de préférence une cellulase, de préférence en une quantité de composition enzymatique de 0,001 à 1 % en poids, plus préférablement 0,001 à 0,6 % en poids ; et
(c4) facultativement au moins un solvant organique, de préférence du glycérol, de préférence en une quantité de 0,1 à 10 % en poids, plus préférablement 0,1 à 5 % en poids ; ou

(d) est un détergent liquide à lessive sous forme de dose unitaire, de préférence un sachet comprenant un film hydrosoluble, et comprend en outre

(d1) de l'eau en une quantité allant jusqu'à 20 % en poids, de préférence 5 à 15 % en poids ;
(d2) facultativement au moins un agent amérisant, de préférence benzoate de benzyldiéthyl(2,6-xylylcarbamoyl)-méthylammonium, de préférence en une quantité de 0,00001 à 0,04 % en poids ;
(d3) facultativement au moins un azurant optique, de préférence en une quantité de 0,01 à 2 % en poids, plus préférablement 0,01 à 1 % en poids ; et
(d4) facultativement au moins un polymère, de préférence en une quantité de 0,01 à 7 % en poids, plus préférablement 0,1 à 5 % en poids ; ou

(e) est un finisseur de tissu et comprend en outre

(e1) au moins une silicone adoucissante, de préférence une silicone à fonctionnalisation amino, de préférence en une quantité de 0,1 à 10 % en poids, plus préférablement 0,1 à 2 % en poids ;
(e2) au moins un parfum, de préférence au moins partiellement encapsulé dans des microcapsules, plus préférablement au moins partiellement encapsulé dans des microcapsules de mélamine-formaldéhyde, de préférence en une quantité de 0,01 à 3 % en poids, plus préférablement 0,1 à 1 % en poids ;
(e3) facultativement du polyquaternium 10 en une quantité de 0,1 à 20 % en poids, de préférence 0,1 à 13 % en poids ;
(e4) facultativement du polyquaternium 37 en une quantité de 0,1 à 20 % en poids, de préférence 0,1 à 13 % en poids ;
(e5) facultativement un esterquat à base de plantes, de préférence un esterquat à base de canola ou de palme, en une quantité de 0,1 à 20 % en poids, de préférence 0,1 à 13 % en poids ; et
(e6) facultativement de l'acide adipique, en une quantité de 0,1 à 20 % en poids, de préférence 0,1 à 13 % en poids ; ou

(f) est un agent de nettoyage acide, ayant de préférence un pH inférieur à 6, et comprend en outre

(f1) des agents tensioactifs à base de plantes ou biosourcés, de préférence chacun en une quantité de 0,1 à 5, plus préférablement chacun en une quantité de 0,1 à 2 % en poids ;
(f2) au moins un biocide acide, choisi de préférence parmi des acides, plus préférablement HCl et acide formique ; et
(f3) au moins un polymère antisalissure, hydrofuge ou d'étalement d'eau, de préférence en une quantité de 0,01 à 3 % en poids, plus préférablement 0,01 à 0,5 % en poids ; ou

(g) est un agent de nettoyage neutre, ayant de préférence un pH entre 6,0 et 7,5, et comprend en outre

(g1) des agents tensioactifs à base de plantes ou biosourcés, de préférence chacun en une quantité de 0,1 à 5, plus préférablement chacun en une quantité de 0,1 à 2 % en poids ;
(g2) au moins un biocide, choisi de préférence parmi des composés d'ammonium quaternaire et des alcools ; et
(g3) au moins un polymère antisalissure, hydrofuge ou d'étalement d'eau, de préférence en une quantité de 0,01 à 3 % en poids, plus préférablement 0,01 à 0,5 % en poids ; ou

(h) est un agent de nettoyage alcalin, ayant de préférence un pH de plus de 7,5, et comprend en outre
(h1) des agents tensioactifs à base de plantes ou biosourcés, de préférence chacun en une quantité de 0,1 à 5, plus préférablement chacun en une quantité de 0,1 à 2 % en poids ; ou
(i) est un agent de lavage de vaisselle à la main, de préférence un agent liquide de lavage de vaisselle à la main, et comprend en outre

(i1) au moins un agent tensioactif anionique, de préférence en une quantité de 0,1 à 40 % en poids, plus préférablement 5 à 30 % en poids ;
(i2) au moins un agent tensioactif amphotère, de préférence de la bétaïne, de préférence en une quantité de 0,1 à 25 % en poids, plus préférablement 1 à 15 % en poids ;
(i3) au moins un agent tensioactif non ionique, de préférence en une quantité de 0,1 à 25 % en poids, plus préférablement 2 à 10 % en poids ;
(i4) au moins une enzyme supplémentaire, choisie de préférence parmi protéases, amylases et combinaisons de celles-ci, de préférence en une quantité de composition enzymatique allant jusqu'à 1 % en poids, plus préférablement jusqu'à 0,6 % en poids ; ou

(j) est une composition de lavage automatique de la vaisselle et comprend en outre

(j1) au moins un adjuvant choisi parmi citrate, aminocarboxylates et combinaisons de ceux-ci, de préférence en une quantité de 5 à 30 % en poids, plus préférablement 10 à 20 % en poids ;
(j2) au moins un adjuvant phosphonate, de préférence en une quantité de 0,1 à 5 % en poids, plus préférablement 0,4 à 1,5 % en poids ;
(j3) au moins un agent tensioactif non ionique, de préférence en une quantité de 0,1 à 10 % en poids, plus préférablement 1 à 5 % en poids ;
(j4) au moins un système de blanchiment, comprenant un agent de blanchiment, un activateur de blanchiment et un catalyseur de blanchiment, de préférence en une quantité de 0,1 à 50 % en poids, plus préférablement 0,1 à 30 % en poids ;
(j5) au moins un polymère choisi parmi sulfopolymères, polymères cationiques et polyacrylates, de préférence en une quantité de 0,01 à 15 % en poids, plus préférablement 2 à 10 % en poids ; ou

(k) comprend en outre
(k1) au moins un sulfopolymère, de préférence en une quantité de 1 à 15, plus préférablement 2 à 10 % en poids et est de préférence une composition de lavage de la vaisselle, plus préférablement de lavage automatique de la vaisselle ; ou
(l) comprend en outre
(l1) au moins un ingrédient additif choisi parmi des probiotiques, de préférence microbes, spores ou combinaisons de ceux-ci ; ou
(m) est sous forme de dose unitaire, de préférence un sachet, et comprend au moins 2, de préférence 2, 3, 4 ou 5 compartiments séparés ;

dans laquelle le polypeptide a au moins 93 %, au moins 94 %, au moins 95 %, au moins 96 %, au moins 97 %, au moins 98 %, au moins 99 % ou 100 % d'identité de séquence par rapport au polypeptide mature de SEQ ID NO 20, SEQ ID NO 33, SEQ ID NO 34 ou SEQ ID NO 35.

**2.** Composition selon la revendication 1, dans laquelle le polypeptide a une activité de N-acétylglucosaminidase et/ou une activité de βN-acétylglucosaminidase.

**3.** Composition selon l'une quelconque des revendications 1 ou 2, dans laquelle le polypeptide comprend un ou plusieurs des motifs

ARAYYPV (SEQ ID NO 42),
AWNDGID (SEQ ID NO 43),
DDQNVGI (SEQ ID NO 44) ou
DPRIH (SEQ ID NO 45).

**4.** Composition selon l'une quelconque des revendications 1 à 3, dans laquelle la composition est choisie parmi les compositions (a), (b), (d), (k), (l) et (m) et comprend en outre jusqu'à 50 % en poids, d'environ 5 % en poids à environ 40 % en poids, d'environ 5 % en poids à environ 30 % en poids, d'environ 5 % en poids à environ 20 % en poids ou d'environ 5 % en poids à environ 10 % en poids d'au moins un agent tensioactif, dans laquelle l'agent tensioactif est de préférence anionique et/ou non ionique.

**5.** Utilisation d'une composition selon l'une quelconque des revendications 1 à 4 pour le nettoyage en profondeur d'un article, dans laquelle l'article est un textile.

**6.** Procédé pour le nettoyage, de préférence le lessivage, d'un article comprenant les étapes consistant à :

a. exposer un article à une liqueur de lavage comprenant une composition de nettoyage selon l'une quelconque des revendications 1 à 4 ;
b. accomplir facultativement au moins un cycle de lavage ; et
c. rincer facultativement l'article,

dans lequel l'article est un textile ou une surface dure.

**7.** Utilisation d'une composition selon l'une quelconque des revendications 1 à 4,

(i) pour le nettoyage ou le conditionnement d'un article ;

(ii) pour la prévention, la réduction ou l'élimination du caractère collant de l'article ;
(iii) pour le prétraitement de taches sur l'article ;
(iv) pour la prévention, la réduction ou l'élimination de redéposition de salissures pendant un cycle de lavage ;
(v) pour la prévention, la réduction ou l'élimination d'adhérence de salissures sur l'article ;
(vi) pour le maintien ou l'amélioration de blancheur de l'article ; ou
(vii) pour la prévention, la réduction ou l'élimination de mauvaises odeurs de l'article,

dans laquelle l'article est un textile.

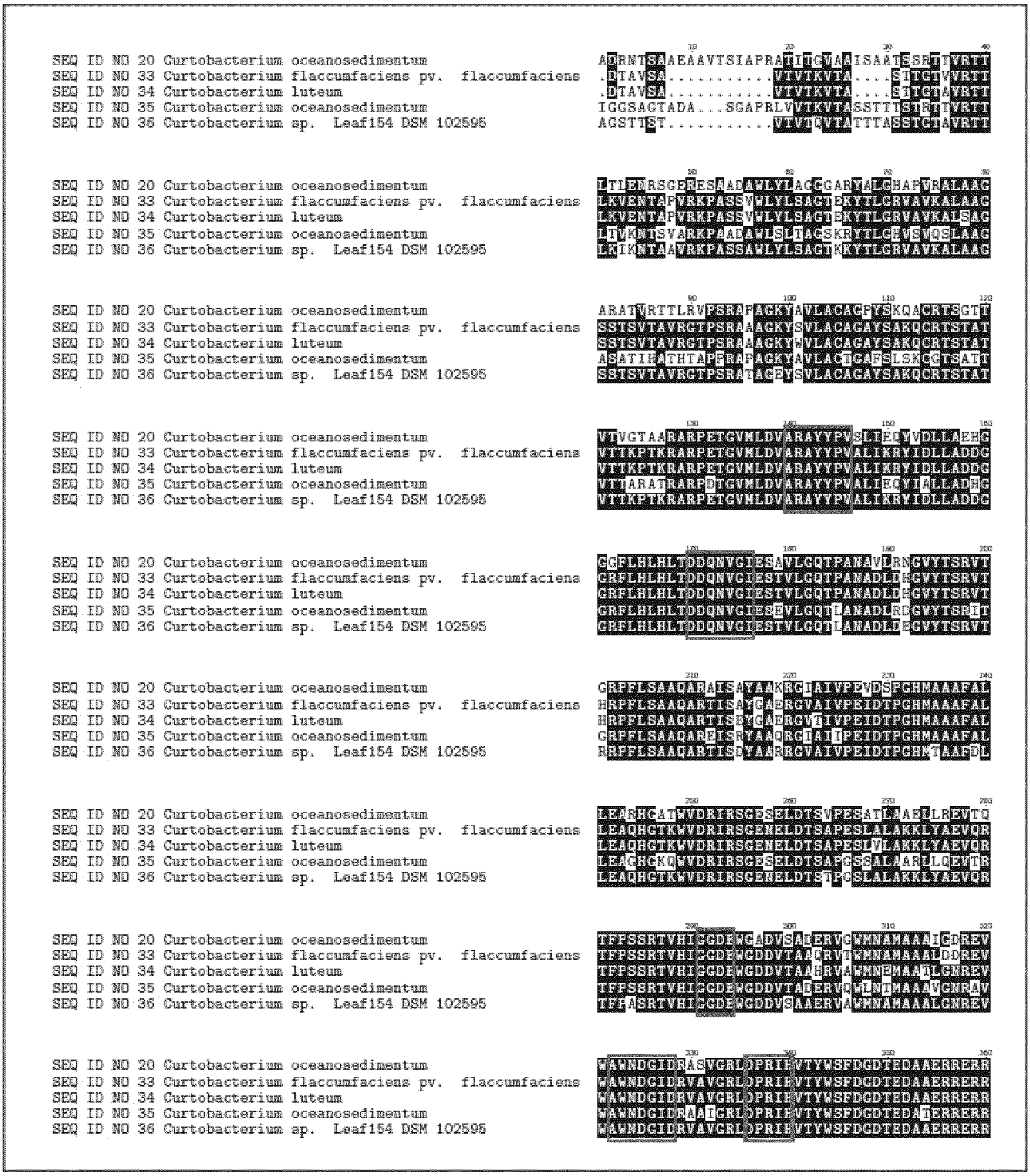
10 20 30 40
SEQ ID NO 20 Curtobacterium oceanosedimentum ADRNTSAAEAAVTSIAPRATITGVAAISAATESRTTVRTT
SEQ ID NO 33 Curtobacterium flaccumfaciens pv. flaccumfaciens .DTAVSA...........VTVTKVTA....STTGTVVRTT
SEQ ID NO 34 Curtobacterium luteum .DTAVSA...........VTVTKVTA....STTGTAVRTT
SEQ ID NO 35 Curtobacterium oceanosedimentum IGGSAGTADA...SGAPRLVVTKVTASSTTTSTRTTVRTT
SEQ ID NO 36 Curtobacterium sp. Leaf154 DSM 102595 AGSTTST...........VTVTQVTATTTASSTGTAVRTT
50 60 70 80
SEQ ID NO 20 Curtobacterium oceanosedimentum LTIEMRSGERESAADAWLYLACGGARYALGHAFVRALAAG
SEQ ID NO 33 Curtobacterium flaccumfaciens pv. flaccumfaciens LKVENTAPVRKPASSVWLYLSAGTEKYTLGRVAVKALAAG
SEQ ID NO 34 Curtobacterium luteum LKVENTAPVRKPASSVWLYLSAGTEKYTLGRVAVKALSAG
SEQ ID NO 35 Curtobacterium oceanosedimentum LTVKNTSVARKPAADAWLSLTAGSKRYTLGHVSVQSLAAG
SEQ ID NO 36 Curtobacterium sp. Leaf154 DSM 102595 LKIKNTAAVRKPASSAWLYLSAGTKKYTLGRVAVKALAAG
90 100 110 120
SEQ ID NO 20 Curtobacterium oceanosedimentum ARATVRTTLRVPSRAPAGKYAVLACAGPYSKQACRTSGTT
SEQ ID NO 33 Curtobacterium flaccumfaciens pv. flaccumfaciens SSTSVTAVRGTPSRAAAGKYSVLACAGAYSAKQCRTSTAT
SEQ ID NO 34 Curtobacterium luteum SSTSVTAVRGTPSRAAAGKYWVLACAGAYSAKQCRTSTAT
SEQ ID NO 35 Curtobacterium oceanosedimentum ASATIHATHTAPFRAPAGKYAVLACTGAFSLSKCGTSATT
SEQ ID NO 36 Curtobacterium sp. Leaf154 DSM 102595 SSTSVTAVRGTPSRATAGEYSVLACAGAYSAKQCRTSTAT
130 140 150 160
SEQ ID NO 20 Curtobacterium oceanosedimentum VTVGTAARARPETGVMLDVARAYYPVSLIEQYVDLLAEHG
SEQ ID NO 33 Curtobacterium flaccumfaciens pv. flaccumfaciens VTTKPTKRARPETGVMLDVARAYYPVALIKRYIDLLADDG
SEQ ID NO 34 Curtobacterium luteum VTTKPTKRARPETGVMLDVARAYYPVALIKRYIDLLADDG
SEQ ID NO 35 Curtobacterium oceanosedimentum VTTARATRARPDTGVMLDVARAYYPVALIEQYIALLADHG
SEQ ID NO 36 Curtobacterium sp. Leaf154 DSM 102595 VTTKPTKRARPETGVMLDVARAYYPVALIKRYIDLLADDG
170 180 190 200
SEQ ID NO 20 Curtobacterium oceanosedimentum GGFLHLHLTDDQNVGIESAVLGQTPANAVLRNGVYTSRVT
SEQ ID NO 33 Curtobacterium flaccumfaciens pv. flaccumfaciens GRFLHLHLTDDQNVGIESTVLGQTPANADLDHGVYTSRVT
SEQ ID NO 34 Curtobacterium luteum GRFLHLHLTDDQNVGIESTVLGQTPANADLDHGVYTSRVT
SEQ ID NO 35 Curtobacterium oceanosedimentum GRFLHLHLTDDQNVGIESEVLGQTLANADLRDGVYTSRIT
SEQ ID NO 36 Curtobacterium sp. Leaf154 DSM 102595 GRFLHLHLTDDQNVGIESTVLGQTLANADLDEGVYTSRVT
210 220 230 240
SEQ ID NO 20 Curtobacterium oceanosedimentum GRPFLSAAQARAISAYAAKRGIAIVPEVDSPGHMAAAFAL
SEQ ID NO 33 Curtobacterium flaccumfaciens pv. flaccumfaciens HRPFLSAAQARTISAYGAERGVAIVPEIDTPGHMAAAFAL
SEQ ID NO 34 Curtobacterium luteum HRPFLSAAQARTISEYGAERGVTIVPEIDTPGHMAAAFAL
SEQ ID NO 35 Curtobacterium oceanosedimentum GRPFLSAAQAREISRYAAQRGIAIIPEIDTPGHMAAAFAL
SEQ ID NO 36 Curtobacterium sp. Leaf154 DSM 102595 RRPFLSAAQARTISDYAARRGVAIVPEIDTPGHMTAAFDL
250 260 270 280
SEQ ID NO 20 Curtobacterium oceanosedimentum LEARHGATWVDRIRSGESELDTSVPESATLAAELLREVTQ
SEQ ID NO 33 Curtobacterium flaccumfaciens pv. flaccumfaciens LEAQHGTKWVDRIRSGENELDTSAPESLALAKKLYAEVQR
SEQ ID NO 34 Curtobacterium luteum LEAQHGTKWVDRIRSGENELDTSAPESLVLAKKLYAEVQR
SEQ ID NO 35 Curtobacterium oceanosedimentum LEAGHGKQWVDRIRSGESELDTSAPGSSALAARLLQEVTR
SEQ ID NO 36 Curtobacterium sp. Leaf154 DSM 102595 LEAQHGTKWVDRIRSGENELDTSTPGSLALAKKLYAEVQR
290 300 310 320
SEQ ID NO 20 Curtobacterium oceanosedimentum TFPSSRTVHIGGDEWGADVSADERVGWMNAMAAAIGDREV
SEQ ID NO 33 Curtobacterium flaccumfaciens pv. flaccumfaciens TFPSSRTVHIGGDEWGDDVTAAQRVTWMNAMAAALDDREV
SEQ ID NO 34 Curtobacterium luteum TFPSSRTVHIGGDEWGDDVTAAHRVAWMNEMAATLGNREV
SEQ ID NO 35 Curtobacterium oceanosedimentum TFPSSRTVHIGGDEWGDDVTADERVQWLNTMAAAVGNRAV
SEQ ID NO 36 Curtobacterium sp. Leaf154 DSM 102595 TFPASRTVHIGGDEWGDDVSAAERVAWMNAMAAALGNREV
330 340 350 360
SEQ ID NO 20 Curtobacterium oceanosedimentum WAWNDGIDRASVGRLDPRIPVTYWSFDGDTEDAAERRERR
SEQ ID NO 33 Curtobacterium flaccumfaciens pv. flaccumfaciens WAWNDGIDRVAVGRLDPRIPVTYWSFDGDTEDAAERRERR
SEQ ID NO 34 Curtobacterium luteum WAWNDGIDRVAVGRLDPRIPVTYWSFDGDTEDAAERRERR
SEQ ID NO 35 Curtobacterium oceanosedimentum WAWNDGIDRAAIGRLDPRIPVTYWSFDGDTEDATERRERR
SEQ ID NO 36 Curtobacterium sp. Leaf154 DSM 102595 WAWNDGIDRVAVGRLDPRIPVTYWSFDGDTEDAAERRERR

Figure 1

```
                                                                             370       380       390       400
SEQ ID NO 20 Curtobacterium oceanosedimentum                    ARRASATDLQRAGIDLLNYNSYYLYEVPTDLDPADSEYTV
SEQ ID NO 33 Curtobacterium flaccumfaciens pv.  flaccumfaciens  ARRASAVDLQQAGIDQLNYNSYYLYEVPTDLDPADSDYTV
SEQ ID NO 34 Curtobacterium luteum                              ARRASAVDLQQAGIDQLNYNSYYLYEVPTDLDPADSDYTV
SEQ ID NO 35 Curtobacterium oceanosedimentum                    ERRAGANDLYAAGIDLLNYNSYYLYEVPTDLDAADSEYTV
SEQ ID NO 36 Curtobacterium sp.  Leaf154 DSM 102595             ARRASAVDLQQAGIDMLNYNSYYLYEVPTDLDPADSEYTV

                                                                             410       420       430       440
SEQ ID NO 20 Curtobacterium oceanosedimentum                    ADLREHWSLRAWDGDSGARLAAPMSGAAVAIWGEDLDGAP
SEQ ID NO 33 Curtobacterium flaccumfaciens pv.  flaccumfaciens  ADLRENWSLRAWDGDSGSLLAAPMSGAAVAIWGEDLEDPP
SEQ ID NO 34 Curtobacterium luteum                              ADLRENWSLRAWDGDSGSLLAAPMSGAAVAIWGEDLEDPP
SEQ ID NO 35 Curtobacterium oceanosedimentum                    ADLRENWSLRTWDGDSGARLAGPTSGAAVAIWGEDLEAPP
SEQ ID NO 36 Curtobacterium sp.  Leaf154 DSM 102595             ADLRENWSLRTWDGDSGSLLAAPMSGAAVAIWGEDLEDPP

                                                                             450       460
SEQ ID NO 20 Curtobacterium oceanosedimentum                    SEALLRWSAPHVTANIETAAS
SEQ ID NO 33 Curtobacterium flaccumfaciens pv.  flaccumfaciens  SDALLRWSAPHVTANIETAAS
SEQ ID NO 34 Curtobacterium luteum                              SDALLRWSAPHVTANIETAAS
SEQ ID NO 35 Curtobacterium oceanosedimentum                    SDALLRWSAPHVLANIETAGS
SEQ ID NO 36 Curtobacterium sp.  Leaf154 DSM 102595             SDALLRWSAPHVTANIETAAS
```

Figure 1 continued

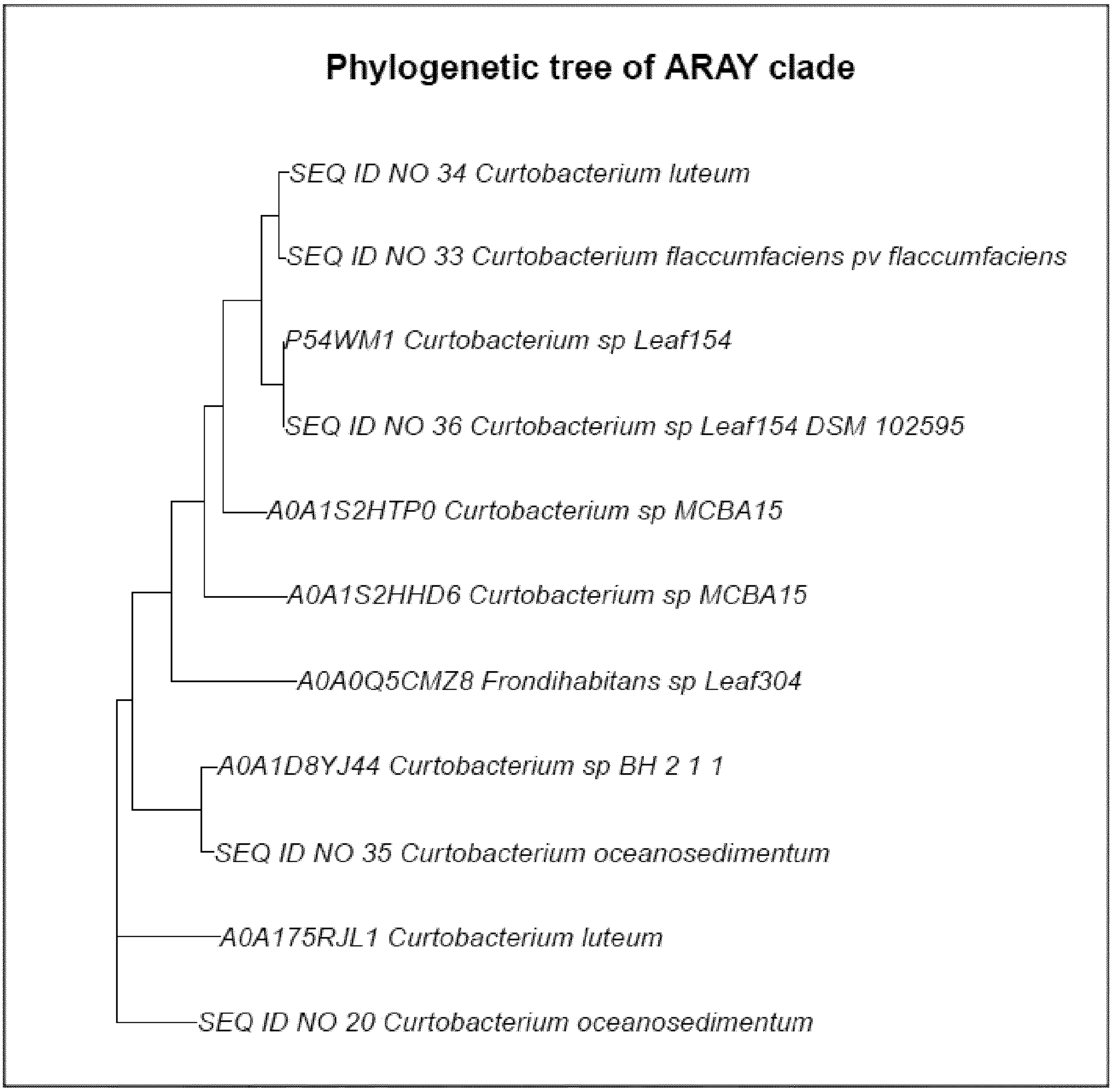
Phylogenetic tree of ARAY clade
SEQ ID NO 34 Curtobacterium luteum
SEQ ID NO 33 Curtobacterium flaccumfaciens pv flaccumfaciens
P54WM1 Curtobacterium sp Leaf154
SEQ ID NO 36 Curtobacterium sp Leaf154 DSM 102595
A0A1S2HTP0 Curtobacterium sp MCBA15
A0A1S2HHD6 Curtobacterium sp MCBA15
A0A0Q5CMZ8 Frondihabitans sp Leaf304
A0A1D8YJ44 Curtobacterium sp BH 2 1 1
SEQ ID NO 35 Curtobacterium oceanosedimentum
A0A175RJL1 Curtobacterium luteum
SEQ ID NO 20 Curtobacterium oceanosedimentum

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04061117 A2
- WO 2014087011 A
- WO 9909143 A
- WO 2012112718 A
- WO 200406117 A
- EP 0164514 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 02099091 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- WO 2016001449 A
- WO 2004067737 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 0787508 A

- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- WO 9324618 A
- WO 9510602 A
- WO 9815257 A
- WO 2006130575 A
- WO 200503274 A
- WO 200503275 A
- WO 200503276 A
- EP 1876226 A
- WO 2007087257 A
- WO 2007087243 A
- WO 2009087523 A
- WO 2007138054 A
- WO 2006108856 A
- WO 2006113314 A
- EP 1867808 A
- WO 2003040279 A
- US 20090011970 A1
- WO 9517413 A
- WO 9522625 A
- US 5223409 A
- WO 9206204 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- WO 9015861 A
- WO 2010096673 A
- WO 12025577 A


**Non-patent literature cited in the description**


- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **FINN.** *Nucleic Acids Research,* 2016, vol. 44, D279-D285
- **DURBIN et al.** Biological sequence analysis: probabilistic models of proteins and nucleic acids. Cambridge University Press, 1998
- **KROGH et al.** *J. Mol. Biol.,* 1994, vol. 235, 1501-1531
- **SIEZEN et al.** *Protein Engng.,* 1991, vol. 4, 719-737
- **SIEZEN et al.** *Protein Science,* 1997, vol. 6, 501-523
- Powdered Detergents, Surfactant science series. Marcel Dekker, Inc, vol. 71
- Powdered Detergents, Surfactant science series. Marcel Dekker, Inc, vol. 71
- **SAMBROOK et al.** Molecular Cloning, A Laboratory Manual. Cold Spring Harbor, 1989
- **INNIS et al.** PCR: A Guide to Methods and Application. Academic Press, 1990
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576

- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev,* 1981, vol. 45, 409-436
- **HAWKSWORTH et al.** Ainsworth and Bisby's Dictionary of The Fungi. CAB International, University Press, 1995
- **SKINNER.** Soc. App. Bacteriol. Symposium Series. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **DIDERICHSEN.** *Plasmid,* 1993, vol. 30, 312-315
- **VALLE et al.** *Mol Microbiol,* May 2003, vol. 48 (4), 1075-87
- **VALLE et al.** *Mol Microbiol.,* May 2003, vol. 48 (4), 1075-87
- **VALLE, J. ; A. TOLEDO-ARANA ; C. BERASAIN ; J. M. GHIGO ; B. AMORENA ; J. R. PENADES ; I. LASA.** *Mol. Microbiol.,* 2003, vol. 48, 1075-1087
- **VALLE, J. ; A. TOLEDO-ARANA ; C. BERASAIN ; J. M. GHIGO ; B. AMORENA ; J. R. PENADES ; I. LASA.** *Mol. Microbiol.,* 2003, vol. 48, 1075-1087
- **EDGAR.** *Nucleic Acids Research,* 2004, vol. 32 (5), 1792-1797
- **PRICE et al.** *PloS one,* 2010, vol. 5 (3
- **LETUNIC ; BORK.** *Bioinformatics,* 2007, vol. 23 (1), 127-128